# EUROPEAN PATENT SPECIFICATION

(11) **EP 4 426 858 B1**
(45) Date of publication and mention of the grant of the patent: **08.04.2026**
(21) Application number: 22814588.4
(22) Date of filing: 02.11.2022
(51) Int. Cl.: C12Q 1/6855

(54) **QUALITY CONTROL METHOD**
QUALITÄTSKONTROLLVERFAHREN
PROCÉDÉ DE CONTRÔLE DE QUALITÉ

(30) Priority: 02.11.2021 US 202163274868 P
(43) Date of publication of application: 11.09.2024
(73) Proprietor: Guardant Health, Inc., Palo Alto, CA 94304 (US)
(72) Inventor: KENNEDY, Andrew, Palo Alto, California 94304 (US)
(74) Representative: Carpmaels & Ransford LLP
(86) International application number: PCT/US2022/079173
(87) International publication number: WO 2023/081722

(56) References cited:
- WO-A1-2015/104302
- WO-A1-2016/016639
- WO-A1-2020/243609
- WO-A1-2021/005537
- WO-A2-2021/178893

## Description

### BACKGROUND

Single-nucleotide resolving assays to detect epigenetic variants or nucleoside modifications generally require a conversion of the modified nucleosides or corresponding unmodified nucleosides to change their base-pairing specificity. The conversion is then detected by sequencing. Examples of such methods include bisulfide and oxidative bisulfide and Tet-assisted bisulfide conversion, EM-seq, TAPS and TAPS β conversion and ACE-seq. See, e.g., Moss et al., Nat Commun. 2018; 9: 5068; Booth et al., Science 2012; 336: 934-937; Yu et al., Cell 2012; 149: 1368-80; Liu et al., Nature Biotechnology 2019; 37:424-429; Schutsky, E.K. et al.; and Vaisvila et al. Genome Research 2021 31(7): 1280-1289.

Bisulfite-based and EM-Seq methylation assays convert unmethylated cytosine to uracil, which is PCR-amplified and NGS-read as thymine. Incomplete (missed) conversion of a unmethylated base results in incorrectly identifying that base as methylated - i.e. a false positive signal. Conversely, erroneous conversion of methylated bases results in incorrectly identifying that base as unmethylated - i.e. a false negative signal. Since ~99% of non-CpG cytosines are unmethylated in the human genome, low/no conversion of CH cytosines (i.e., cytosines followed by a base other than guanine) in a given DNA molecule is sometimes used to filter out non-converted molecules prior to assessing (CpG) methylation levels.

In other methods, the epigenetic conversion is opposite, i.e. the modified nucleosides, rather than the unmodified nucleosides, are converted. For example, in the TAPS method from Song's lab at Ludwig Cancer Institute, methylated cytosines (5mCs and 5hmCs) are converted to DHU, PCR-amplified and NGS-read as thymine. Incomplete/missed conversion of methylated residues in TAPS results in incorrectly identifying that base as unmethylated - i.e., a false negative signal. Conversely, erroneous conversion of unmethylated bases results in incorrectly identifying that base as methylated - i.e., a false positive signal. In these methods, the nucleosides that are converted are generally much rarer in the sample, so ineffective conversion, and the consequent false negative signals, are more difficult to adequately detect.

Given the issues surrounding the false positive and false negative signals in these assays, there is a need for quality control methods which allow for the estimation of these false signals, so the data can be interpreted accordingly.

WO2021/178893 (Singular Genomics Systems, Inc.) discusses adapters containing a *"bisulfite conversion control region consisting of one or more unmethylated cytosine bases"* (see [0032] of WO2021/178893).

### SUMMARY

Described herein are methods that provide improved quality control for the conversion step in methods for detecting and/or identifying modified nucleosides in a DNA sample that rely on using a base pairing specificity conversion procedure that is sensitive to the modification status of nucleosides. The invention is set out in the appended set of claims. References to methods for treatment of the human or animal body by surgery or therapy and diagnostic methods practised on the human or animal body are to be read as products, e.g. substances or compositions, for use in such methods. The disclosure includes the following exemplary embodiments.

In some embodiments, the conversion procedure is selected to change the base pairing specificity of quality control nucleosides in the adapters, but not the base pairing specificity of DNA sample nucleosides having the same nucleoside identity and a different modification status; and wherein suboptimal conversion of the quality control nucleosides predicts false positive detection of DNA sample nucleosides having the different modification status.

Embodiment 8 is the method of the immediately preceding embodiment, wherein the quality control nucleosides in the adapters include cytosine and/or wherein conversion procedure comprises bisulfite conversion.

In some embodiments, the first quality control nucleoside is a modified cytosine and the second quality control nucleoside is an unmodified cytosine.

In some embodiments, the conversion procedure is selected to change the base pairing specificity of the first quality control nucleoside but not the second quality control nucleoside, or the conversion procedure is selected to change the base pairing specificity of the second quality control nucleoside but not the first quality control nucleoside

In some embodiments, the conversion procedure is selected to change the base pairing specificity of modified quality control nucleosides in the adapters, but not the base pairing specificity of DNA sample nucleosides having the same nucleoside identity but a different modification status and/or no modification; and wherein suboptimal conversion of the modified quality control nucleosides predicts false negative detection of DNA sample nucleosides having the same nucleoside identity and modification status as the quality control nucleosides or a different modification status and the same change in base pairing specificity on exposure to the conversion procedure.

Embodiment 15 is the method of any one of the preceding embodiments, wherein the method further comprises using the sequence data obtained in step (c) to (i) identify adapted DNA molecules with sub-optimal or erroneous conversion of quality control nucleosides in the adapter sequence; and (ii) infer sub-optimal or erroneous conversion of nucleosides having the same nucleoside identity and modification status in the full length molecules identified in step (i).

Embodiment 16 is the method of any one of the preceding embodiments, wherein the method further comprises determining the conversion rate for quality control nucleosides in the adapted DNA or in individual adapted DNA molecules and (i) applying a weighting to analysis of the modified nucleoside detection in (A) the DNA sample; or (B) individual adapted DNA molecules, wherein the weighting is dependent on the conversion rate; (ii) excluding DNA samples having (A) suboptimal conversion of adapter quality control nucleosides or a conversion rate for adapter quality control nucleosides that is below a pre-determined quality control threshold; and/or (B) erroneous conversion of adapter quality control nucleosides, or a conversion rate for adapter quality control nucleosides that is above a pre-determined quality control threshold, from further analysis for detecting modified nucleosides; and/or (iii) excluding adapted DNA molecules having (A) suboptimal conversion of adapter quality control nucleosides or a conversion rate for adapter quality control nucleosides that is below a pre-determined quality control threshold; and/or (B) erroneous conversion of adapter quality control nucleosides, or a conversion rate for adapter quality control nucleosides that is above a pre-determined quality control threshold, from further analysis for detecting modified nucleosides.

Embodiment 17 is the method of any one of the preceding embodiments, wherein the method further comprises enriching the DNA by capturing a target region set from the sample, wherein the capture step is before, after or in between the ligating step (a) and the conversion step (b).

Embodiment 18 is the method of any one of the preceding embodiments, further comprising (i) comparing the sequence data obtained in step (c) with (A) a pre-determined reference sequence; and/or (B) sequence data obtained by sequencing a sub-sample of the DNA that was not subjected to the conversion procedure; and (ii) identifying point differences between the converted DNA sequences and the reference sequence (A) or non-converted DNA sequence data (B) as nucleosides having a modification status that permits a change in base pairing specificity on exposure to the conversion procedure.

Embodiment 19 is the method of any one of the preceding embodiments, wherein the DNA comprises cell-free DNA (cfDNA), optionally cfDNA obtained from a test subject, optionally wherein the test subject is a patient having or suspected of having cancer.

Embodiment 20 is the method of any one of the preceding embodiments, further comprising using the detection of modified nucleosides in the DNA sample to determine or predict the presence of DNA produced by a cancer cell or tumor, to determine the probability that a test subject has a tumor or cancer, or to characterize a cancer or tumor of the subject.

Embodiment 21 is the method of any one of the preceding embodiments, wherein a sub-sample of the DNA is not subjected to the conversion procedure before sequencing, wherein the converted subsample and the non-converted subsample have different adapter sequences, and wherein the converted subsample and the non-converted subsample are recombined for sequencing step (c).

Embodiment 22 is the method of any one of the preceding embodiments, further comprising analyzing the DNA to detect copy number variation, single nucleotide variants, insertions, deletions, methylation, and/or fusions.

Embodiment 23 is the method of any one of the preceding embodiments, further comprising capturing epigenetic target regions from the adapter-ligated DNA and amplifying and sequencing the epigenetic target regions.

Embodiment 24 is the method of embodiment 23, wherein the captured epigenetic target regions form an epigenetic target region set.

Embodiment 25 is the method of embodiment 24, wherein the epigenetic target region set comprises a plurality of type-specific epigenetic target regions, and wherein the type-specific epigenetic target regions are type-specific differentially methylated regions and/or type specific fragments.

Embodiment 26 is the method of embodiment 25, wherein the plurality of type-specific epigenetic target regions comprises type-specific hypomethylated regions.

Embodiment 27 is the method of any one of the preceding embodiments, wherein the sample is a blood sample.

Embodiment 28 is the method of any one of embodiments 25-27, wherein the plurality of type-specific epigenetic target regions comprises target regions that are: hypermethylated in immune cells relative to non-immune cell types present in the blood sample; differentially methylated in colon relative to other tissue types; differentially methylated in breast relative to other tissue types; differentially methylated in liver relative to other tissue types; differentially methylated in kidney relative to other tissue types; differentially methylated in pancreas relative to other tissue types; differentially methylated in prostate relative to other tissue types; differentially methylated in skin relative to other tissue types; or differentially methylated in bladder relative to other tissue types.

Embodiment 29 is the method of any one of embodiments 25-28, wherein the plurality of type-specific epigenetic target regions comprises: target regions that are hypomethylated in non-immune blood cells relative to the methylation level of the target regions in a different cell or tissue type in the sample; fragments specific to immune cells relative to non-immune cell types present in the blood sample; or fragments specific to colon, lung, breast, liver, kidney, pancreas, prostate, skin, or bladder relative to other tissue types.

Embodiment 30 is the method of any one of embodiments 23-29, further comprising identifying at least one cell type or tissue type from which the type-specific epigenetic target regions originated.

Embodiment 31 is the method of any one of embodiments 23-30, wherein the level of type-specific epigenetic target regions that originated from a cell or tissue type is determined.

Embodiment 32 is the method of the immediately preceding embodiment, wherein the levels of type-specific epigenetic target regions that originated from immune cells, non-immune blood cells, colon, lung, breast, liver, kidney, prostate, skin, bladder, or pancreas are determined.

Embodiment 33 is the method of embodiment 32, wherein the type-specific epigenetic target regions comprise cell-type specific, tissue-type specific, and/or cancer-type specific epigenetic target regions.

Embodiment 34 is the method of any one of embodiments 27-33, wherein the blood sample is fractionated prior to capturing at least an epigenetic target region set of DNA.

Embodiment 35 is the method of any one of the preceding embodiments, further comprising: partitioning the sample or an aliquot thereof into a plurality of partitioned subsamples, including a first partitioned subsample and a second partitioned subsample, wherein the first subsample comprises DNA with a cytosine modification in a greater proportion than the second subsample; contacting the second partitioned subsample with a methylation-dependent nuclease, thereby degrading nonspecifically partitioned DNA in the second subsample to produce a treated second subsample and optionally contacting the first partitioned subsample with a methylation-sensitive endonuclease, thereby degrading nonspecifically partitioned DNA in the first partitioned subsample to produce a treated first subsample; wherein epigenetic target regions are captured from at least a portion of the first subsample or the treated first subsample, optionally wherein the DNA from the subject that is contacted with the one or more capture probes comprises DNA from the first partitioned subsample, the treated first subsample, the second partitioned subsample, and/or the treated second subsample.

Embodiment 36 is the method of the immediately preceding embodiment, wherein the cytosine modification is methylation, optionally wherein the cytosine modification is methylation at the 5 position of cytosine.

Embodiment 37 is the method of embodiment 35 or embodiment 36, wherein the first subsample is contacted with a methylation-sensitive endonuclease.

Embodiment 38 is the method of the immediately preceding embodiment, wherein the methylation-sensitive endonuclease cleaves an unmethylated CpG sequence.

Embodiment 39 is the method of any one of embodiments 35-38, wherein the methylation-sensitive endonuclease is one or more of AatII, AccII, AciI, Aor13HI, Aor15HI, BspT104I, BssHII, BstUI, Cfr10I, ClaI, CpoI, Eco52I, HaeII, HapII, HhaI, Hin6I, HpaII, HpyCH4IV, MluI, NaeI, NotI, NruI, NsbI, PmaCI, Psp1406I, PvuI, SacII, SalI, SmaI, and SnaBI.

Embodiment 40 is the method of any one of the preceding embodiments, wherein the conversion procedure comprises bisulfite conversion; protection of 5hmC; Tet-assisted bisulfite conversion; Tet-assisted conversion with a substituted borane reducing agent, optionally wherein the substituted borane reducing agent is 2-picoline borane, borane pyridine, tert-butylamine borane, or ammonia borane; protection of hmC followed by Tet-assisted conversion with a substituted borane reducing agent, optionally wherein the substituted borane reducing agent is 2-picoline borane, borane pyridine, tert-butylamine borane, or ammonia borane; protection of hmC followed by deamination of mC and/or C; chemical-assisted conversion with a substituted borane reducing agent, optionally wherein the substituted borane reducing agent is 2-picoline borane, borane pyridine, tert-butylamine borane, or ammonia borane; or enzymatic protection of modified cytosines followed by deamination of unprotected cytosines to uracil.

Embodiment 41 is the method of embodiment 40, wherein the substituted borane reducing agent is 2-picoline borane or borane pyridine.

Embodiment 42 is the method of embodiment 40, wherein the deamination of mC and/or C comprises treatment with an AID/APOBEC family DNA deaminase enzyme.

Embodiment 43 is the method of embodiment 40, wherein protection of hmC comprises glucosylation of hmC.

Embodiment 44 is the method of any one of the preceding embodiments, further comprising detecting the presence or absence of sequence variations and/or determining fragmentation patterns, wherein adapted DNA comprising quality control nucleosides indicative of sub-optimal or erroneous conversion of quality control nucleosides is included in detecting the presence or absence of sequence variations and/or determining fragmentation patterns.

Embodiment 49 is the method of any one of the preceding embodiments, wherein the oligonucleotide adapters comprise sequencing primer binding sites and the quality control nucleosides are located downstream of the sequencing primer binding sites. To be "downstream of" a primer binding site with respect to an adapter at the 5' end of a strand means located 3' of the primer binding site, and with respect to an adapter at the 3' end of a strand means located 5' of the primer binding site.

Embodiment 50 is the method of any one of the preceding embodiments, wherein the method further comprises amplifying the DNA using primers targeting the adapters, wherein the amplifying step is in between the conversion step (b) and the sequencing step (c).

In some embodiments, the method further comprises using the sequence data obtained in step (c) to: (i) identify adapted DNA molecules with sub-optimal or erroneous conversion of quality control nucleosides in the adapter sequence; and (ii) infer (additional) sub-optimal or erroneous conversion of nucleosides having the same nucleoside identity and modification status in the full length molecules identified in step (i).

In some embodiments, the method further comprises determining the conversion rate for quality control nucleosides in the adapted DNA or in individual adapted DNA molecules and (i) applying a weighting to analysis of the modified nucleoside detection in (A) the DNA sample; or (B) individual adapted DNA molecules, wherein the weighting is dependent on the conversion rate; (ii) excluding DNA samples having (A) suboptimal conversion of adapter quality control nucleosides or a conversion rate for adapter quality control nucleosides that is below quality control threshold or a pre-determined quality control threshold; and/or (B) erroneous conversion of adapter quality control nucleosides, or a conversion rate for adapter quality control nucleosides that is above a quality control threshold or pre-determined quality control threshold, from further analysis for detecting modified nucleosides; and/or (iii) excluding adapted DNA molecules having (A) suboptimal conversion of adapter quality control nucleosides or a conversion rate for adapter quality control nucleosides that is below a quality control threshold or pre-determined quality control threshold; and/or (B) erroneous conversion of adapter quality control nucleosides, or a conversion rate for adapter quality control nucleosides that is above a quality control threshold or pre-determined quality control threshold, from further analysis for detecting modified nucleosides.

Modified adapter quality control nucleosides can be used to detect false negatives (i.e. nucleosides which are incorrectly identified as being unmodified) when using conversion procedures which convert the base-pairing specificity of modified nucleosides. Accordingly, in some embodiments, the conversion procedure is selected to change the base pairing specificity of modified quality control nucleosides in the adapters, but not the base pairing specificity of DNA sample nucleosides of the same nucleoside identity but having a different modification status compared to the quality control nucleosides and/or no modification; and wherein suboptimal conversion of the modified quality control nucleosides predicts false negative detection of DNA sample nucleosides having the same identity and modification status as the quality control nucleosides or the same base pairing specificity change on exposure to the conversion procedure. That is, conversion of the base pairing specificity of the un-modified quality control nucleosides predicts that other unmodified nucleosides in the DNA sample having the same nucleoside identity (or DNA sample modified nucleosides having the same nucleoside identity as the quality control nucleosides but a different modification status that protects the nucleoside from the change in base-pairing specificity on exposure to the conversion procedure) will be falsely identified as having the modification status of the quality control nucleosides (or, where applicable, a different modification that still permits the same change in base-pairing specificity on exposure to the conversion procedure).

**In** some embodiments, quality control nucleosides in the adapters include 5-methylcytosine (5mC) and/or 5-hydroxymethyl-cytosine (5hmC). In some embodiments, the conversion procedure comprises Tet-assisted conversion with a substituted borane reducing agent, optionally wherein the substituted borane reducing agent is 2-picoline borane, borane pyridine, tert-butylamine borane, or ammonia borane.

Modified adapter quality control nucleosides can also be used to detect false negatives (i.e. nucleosides which are incorrectly identified as being unmodified) when using conversion procedures which convert unmodified nucleosides. Accordingly, in some embodiments, the conversion procedure is selected not to change the base pairing specificity of quality control nucleosides in the adapters, but to change the base pairing specificity of DNA sample nucleosides of the same nucleoside identity but having a different modification status compared to the quality control nucleosides and/or no modification; and wherein erroneous conversion of the quality control nucleosides predicts false negative detection/identification of DNA sample nucleosides having the same nucleoside identity and modification status as the quality control nucleosides or the same base pairing specificity change on exposure to the conversion procedure. That is, conversion of the base pairing specificity of modified quality control nucleosides predicts that other DNA sample nucleosides having the same nucleoside identity and the same modification (or a different modification status but the same expected change in base pairing specificity on exposure to the conversion procedure) will be falsely identified as having no modification (or, where applicable, a different modification that protects the nucleoside from a change in base-pairing specificity on exposure to the conversion procedure). In some embodiments, the quality control nucleosides in the adapters include 5-methylcytosine (5mC) and/or 5-hydroxymethyl-cytosine (5hmC). In some embodiments the conversion procedure comprises bisulfite conversion.

In other cases, unmodified adapter quality control nucleosides can be used to detect false positives (i.e., nucleosides which are incorrectly identified as being modified) when using conversion procedures which convert unmodified nucleosides. Accordingly, in some embodiments, the conversion procedure is selected to change the base pairing specificity of quality control nucleosides in the adapters, but not the base pairing specificity of DNA sample nucleosides having the same nucleoside identity but having a different modification status compared to the quality control nucleosides; and wherein suboptimal conversion of the quality control nucleosides predicts false positive detection of DNA sample nucleosides having the different modification status. That is, non-conversion of the base pairing specificity of un-modified quality control nucleosides predicts that other DNA sample unmodified nucleosides having the same nucleoside identity as the quality control nucleosides (and/or, where applicable, the same nucleoside identity and a different modification status, specifically one that does not protect the nucleoside from the change in base pairing specificity on exposure to the conversion procedure) will be falsely identified as being modified (or, where applicable, as having a modification that does not protect the nucleoside from a change in base-pairing specificity on exposure to the conversion procedure). In some embodiments, the quality control nucleosides in the adapters include cytosine. In some embodiments, the conversion procedure comprises bisulfite conversion.

Unmodified adapter quality control nucleosides can also be used to detect false positives (i.e., nucleosides which are incorrectly identified as being modified) when using conversion procedures which convert modified nucleosides. Accordingly, in some embodiments, the conversion procedure is selected not to change the base pairing specificity of quality control nucleosides in the adapters, but to change the base pairing specificity of DNA sample nucleosides of the same nucleoside identity but having a different modification status compared to the quality control nucleosides; and wherein erroneous conversion of the quality control nucleosides predicts false positive detection of DNA sample nucleosides having the different modification status. That is, conversion of the base pairing specificity of the unmodified quality control nucleosides predicts that other unmodified nucleosides in the DNA sample having the same nucleoside identity (or DNA sample modified nucleosides having the same nucleoside identity as the quality control nucleosides but a different modification status that protects the nucleoside from the change in base-pairing specificity on exposure to the conversion procedure) will be falsely identified as having the modification status of the quality control nucleosides (or, where applicable, a different modification that still permits the same change in base-pairing specificity on exposure to the conversion procedure). In some embodiments, the quality control nucleosides in the adapters include cytosine. In some embodiments, the conversion procedure comprises Tet-assisted conversion with a substituted borane reducing agent, optionally wherein the substituted borane reducing agent is 2-picoline borane, borane pyridine, tert-butylamine borane, or ammonia borane.

The method may further comprise enriching the DNA by capturing a target region set from the sample. The capture step may be before, after or in between the ligating step (a) and the conversion step (b).

Sequencing step (c) allows modified nucleosides in the initial sample to be identified as those that have been converted. For example, the method may comprise: (i) comparing the sequence data obtained in step (c) with (A) a (pre-determined) reference sequences; and/or (B) sequence data obtained by sequencing a sub-sample of the DNA that was not subjected to the conversion procedure; and (ii) identifying point differences between the converted DNA sequences and the reference sequence (A) or non-converted DNA sequence data (B) as nucleotides having a modification status that permits a change in base pairing specificity on exposure to the conversion procedure.

In some embodiments, the DNA comprises cell-free DNA (cfDNA). The cfDNA may, for example, be obtained from a test subject. In some cases, the test subject is a patient having or suspected of having cancer.

In some cases, the method may further comprise using the detection of modified nucleosides in the DNA sample to determine or predict the presence of DNA produced by a cancer cell or tumor, to determine the probability that a test subject has a tumor or cancer, or to characterize a cancer or tumor of the subject.

In some embodiments, a sub-sample of the DNA is not subjected to the conversion procedure before sequencing. In some cases, the converted subsample and the non-converted subsample may have different adapter sequences. The converted subsample and the non-converted subsample may be recombined for sequencing step (c). The different adapter sequences may be used to distinguish sequences or molecules from the exposed subsample and the non-exposed subsample in later steps or analysis.

In a further aspect, the disclosure provides a method of detecting modified nucleosides in a DNA sample. The method may comprise any set of steps (a) to (e) set out above. Other features set out above, where appropriate, are also applicable to such methods. For example, the method may further comprise (f) comparing the sequence data obtained in step (c) with (A) pre-determined reference sequences; and/or (B) sequence data obtained by sequencing a sub-sample of the DNA that was not subjected to the conversion procedure; and (g) identifying point differences between the converted DNA sequences and the reference or non-converted DNA sequences as modified nucleotides in the DNA sample. Step (e) provides a quality control measure for the method.

The conversion rate determined in step (d) provides a quality control measure for the conversion procedure, and can be used to estimate the rate of false negatives, i.e. modified residues in the initial sample that were not effectively converted by the conversion procedure and hence falsely identified.

Hence, in a further aspect the disclosure provides a quality control method for analyzing the modified nucleoside profile of DNA in a sample or for detecting modified nucleosides in a DNA sample. The method may comprises any set of steps (a) to (e) set out above. Other features set out above, where appropriate, are also applicable to such methods.

In some embodiments, the method further comprises applying a weighting in the analysis to the DNA sample or to individual adapted DNA molecules in the sample, wherein the weighting is dependent on the conversion rate determined in step (d). Typically, greater weighting is given to samples or molecules with higher determined conversion rates and lesser weighting given to samples or molecules with lower determined conversion rates. The weighting reflects the level of confidence that can be assigned to the modified nucleoside profile determined by sequencing the DNA molecules of the sample after conversion. In some cases, samples or molecules with sub-optimal conversion or a conversion rate that is below a (pre-determined) quality control threshold may be excluded from further analysis.

In some embodiments, the results of the methods disclosed herein are used as an input to generate a report. The report may be in a paper or electronic format. For example, the detection of false positives and/or false negatives, as obtained by the methods disclosed herein, or information derived therefrom, can be displayed directly in such a report. Alternatively, or additionally, diagnostic information or therapeutic recommendations which are at least in part based on the methods disclosed herein can be included in the report.

The various steps of the methods disclosed herein may be carried out at the same or different times, in the same or different geographical locations, e.g., countries, and/or by the same or different people.

Additional advantages will be set forth in part in the description which follows or may be learned by practice. The advantages will be realized and attained by means of the elements and combinations particularly pointed out in the appended claims.

### BRIEF DESCRIPTION OF THE DRAWINGS

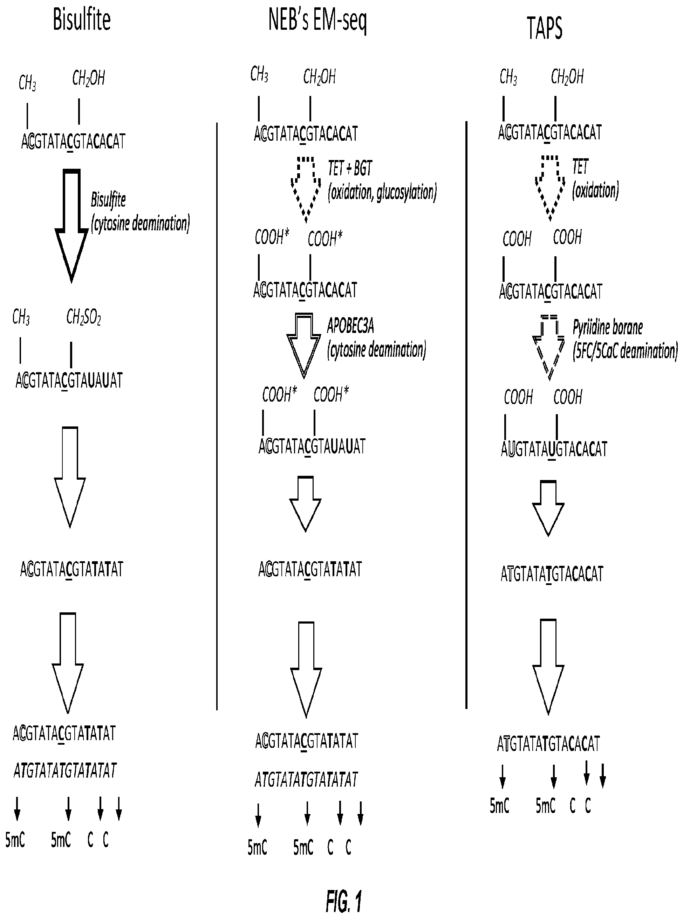

FIG. 1 illustrates the differences between three single-nucleotide resolution methylation assays, bisulfite-sequencing, NEB's EM-seq and TAPS. All three methods distinguish methyl-cytosine and hydroxymethyl-cytosine from non-methylated cytosine. However, TAPS has opposite conversion logic to the other two methods, changing base-pairing of the methylated cytosines (sequence read as "T") and not the non-methylated cytosines (still read as "C"), whilst bisulfite-sequencing and EM-seq change the base-pairing of non-methylated cytosine (sequence read as "T") and not the methylated cytosines (still read as "C").

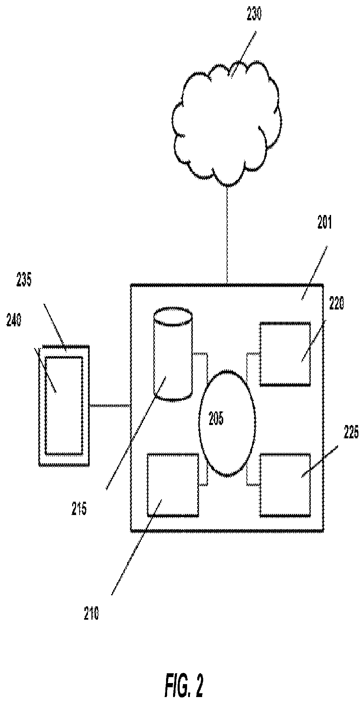

FIG. 2 is a schematic diagram of an example of a system suitable for use with some embodiments of the disclosure.

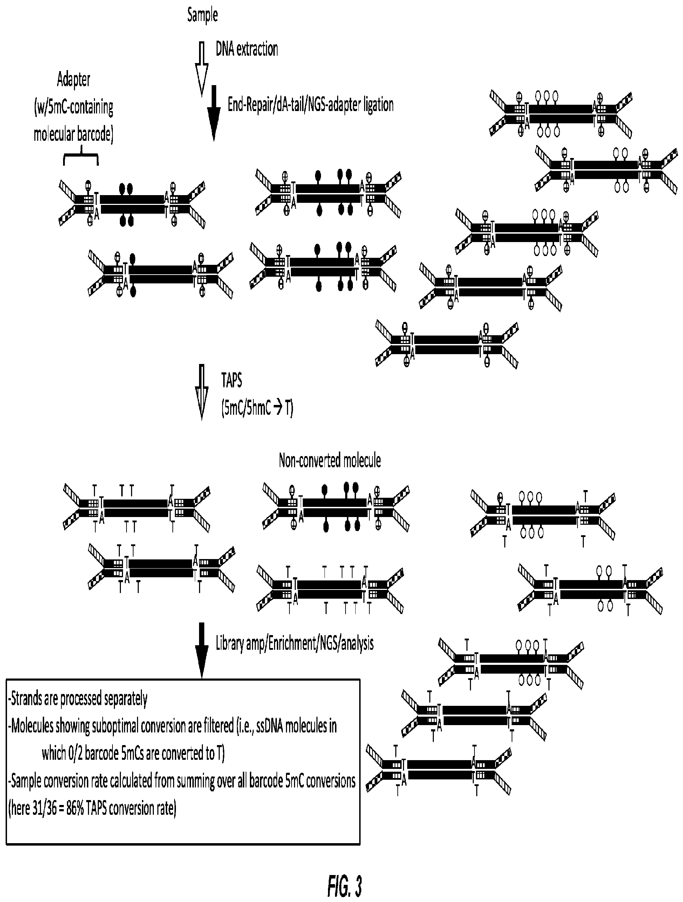

FIG. 3 illustrates an embodiment of a quality control method for monitoring false negative and/or false positive detection of DNA subjected to a TAPS base conversion procedure. Adapters containing a 5mC (e.g., in a molecular barcode) are ligated to DNA then subjected to a TAPS conversion procedure, changing the base-pairing specificity of the methylated cytosines (sequence read as "T") but not the non-methylated cytosines (still read as "C"). DNA molecules containing non-converted 5mC in the adapter can be used to infer suboptimal conversion of 5mC in the full-length molecule.

### DETAILED DESCRIPTION

Reference will now be made in detail to certain embodiments of the disclosure. While the disclosure will be described in conjunction with such embodiments, it will be understood that they are not intended to limit the invention to those embodiments. On the contrary, the disclosure is intended to cover all alternatives, modifications, and equivalents, which may be included within the invention as defined by the appended claims.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a nucleic acid" includes a plurality of nucleic acids.

Numeric ranges are inclusive of the numbers defining the range. Measured and measurable values are understood to be approximate, taking into account significant digits and the error associated with the measurement.

Unless specifically noted in the above specification, embodiments in the specification that recite "comprising" various components are also contemplated as "consisting of" or "consisting essentially of" the recited components.

The section headings used herein are for organizational purposes and are not to be construed as limiting the disclosed subject matter in any way.

If different versions of a publication, website or the like are published at different times, the version most recently published at the effective filing date of the application is meant, unless otherwise indicated.

### Definitions

As used herein, "base pairing specificity" refers to the standard DNA base (A, C, G, or T) for which a given base most preferentially pairs. Thus, for example, unmodified cytosine and 5-methylcytosine have the same base pairing specificity (i.e., specificity for G) whereas uracil and cytosine have different base pairing specificity because uracil has base pairing specificity for A while cytosine has base pairing specificity for G. The ability of uracil to form a wobble pair with G, for example, is irrelevant because uracil nonetheless most preferentially pairs with A among the four standard DNA bases.

Nucleosides of the "same identity" or "same nucleoside identity" refer to nucleosides with the same base, regardless of modification status of that base. For example, cytosine is considered to be the "same identity" as 5-methylcytosine (5mC) and/or 5-hydroxymethyl-cytosine (5hmC), despite them having different modification statuses.

A "conversion reagent" refers to a reagent that can be used to change the base pairing specificity of at least one modified or unmodified nucleoside in a nucleic acid. For example, bisulfite is a conversion reagent that can be used to change unmodified cytosine (having base pairing specificity for G) to uracil (having base pairing specificity for A), and Tet enzyme and pyridine borane are conversion reagents that can be used together to change methylated or hydroxymethylated cytosine (having base pairing specificity for G) to dihydrouracil (having base pairing specificity for A). A conversion procedure is a procedure that uses one or more conversion reagents to change the base pairing specificity of at least one modified or unmodified nucleoside in a nucleic acid.

A conversion reagent or procedure is "substantially not capable of changing the base pairing specificity" of a nucleoside with a first modification status (which may be modified or unmodified) if the conversion reagent or procedure preferentially changes the base pairing specificity of that nucleoside with a second, different modification status to the point that changes to the base pairing specificity of the nucleoside having the first modification status are properly considered erroneous. For example, bisulfite conversion is substantially not capable of changing the base pairing specificity of 5mC (nucleoside with a first modification status) but preferentially changes the base pairing specificity of unmodified cytosine (nucleoside with a second modification status). In contrast, TAPS preferentially changes the base pairing specificity of 5mC (and 5hmC) (either of which may be considered to have a second modification status), but TAPS is substantially not capable of changing the base pairing specificity of unmodified C (nucleoside with a first modification status).

"Capturing" one or more target nucleic acids refers to preferentially isolating or separating the one or more target nucleic acids from non-target nucleic acids.

A "captured set" of nucleic acids refers to nucleic acids that have undergone capture.

A "target-region set" or "set of target regions" refers to a plurality of genomic loci targeted for capture and/or targeted by a set of probes (e.g., through sequence complementarity).

"Corresponding to a target region set" means that a nucleic acid, such as cfDNA, originated from a locus in the target region set or specifically binds one or more probes for the target-region set.

"Sequence-variable target regions" refer to target regions that may exhibit changes in sequence such as nucleotide substitutions (i.e., single nucleotide variations), insertions, deletions, or gene fusions or transpositions in neoplastic cells (e.g., tumor cells and cancer cells) relative to normal cells. A sequence-variable target region set is a set of sequence-variable target regions. In some embodiments, the sequence-variable target regions are target regions that may exhibit changes that affect less than or equal to 50 contiguous nucleotides, e.g., less than or equal to 40, 30, 20, 10, 5, 4, 3, or 2 nucleotides, or that affect 1 nucleotide.

"Epigenetic target regions" refers to target regions that may show sequence-independent changes across tissue types (e.g., a target region having a different extent of methylation in a solid tissue type than in hematopoietic cells) or differences in neoplastic cells, such as tumor cells or cancer cells, relative to normal cells. In some embodiments, epigenetic target regions show sequence-independent differences in cfDNA originating from tissue types that ordinarily do not substantially contribute to cfDNA, such as lung, colon, etc., relative to background cfDNA, such as cfDNA that originated from hematopoietic cells. In some embodiments, epigenetic target regions show sequence-independent differences in cfDNA from subjects having cancer relative to cfDNA from healthy subjects. Examples of sequence-independent changes include, but are not limited to, changes in methylation (increases or decreases), nucleosome distribution, cfDNA fragmentation patterns, CCCTC-binding factor ("CTCF") binding, transcription start sites, and regulatory protein binding regions. An epigenetic target region set is a set of epigenetic target regions. Epigenetic target region sets thus include, but are not limited to, hypermethylation variable target region sets, hypomethylation variable target region sets, and fragmentation variable target region sets, such as CTCF binding sites and transcription start sites. For present purposes, loci susceptible to neoplasia-, tumor-, or cancer-associated focal amplifications and/or gene fusions may also be included in an epigenetic target region set because detection of a change in copy number by sequencing or a fused sequence that maps to more than one locus in a reference genome tends to be more similar to detection of exemplary epigenetic changes discussed above than detection of nucleotide substitutions, insertions, or deletions, e.g., in that the focal amplifications and/or gene fusions can be detected at a relatively shallow depth of sequencing because their detection does not depend on the accuracy of base calls at one or a few individual positions.

As used herein, an "epigenetic feature" refers to any feature of DNA or chromatin other than primary sequence (i.e., the sequence of A, C, G, and T bases). Epigenetic features include covalent modifications of bases, such as methylation, and modifications and positioning of histones and other stably DNA-associated proteins.

As used herein, a "differentially methylated region" refers to a region having a detectably different degree of methylation in at least one type of tissue relative to the degree of methylation in cell-free DNA from a healthy subject. In some embodiments, a differentially methylated region of DNA has a detectably different degree of methylation in at least one type of tissue relative to the degree of methylation in another type of tissue; or in a sample from a healthy subject relative to the degree of methylation in a subject having pre-cancer, cancer, or a neoplasm. In some embodiments, a differentially methylated region has a detectably higher degree of methylation in at least one type of tissue relative to the degree of methylation in cell-free DNA from a healthy subject. In some embodiments, a differentially methylated region has a detectably lower degree of methylation in at least one type of tissue relative to the degree of methylation in cell-free DNA from a healthy subject. In some embodiments, differentially methylated regions are hypomethylated in the erythrocyte lineage or in an immature red blood cell (e.g., reticulocyte) and hypermethylated in at least one non-erythrocyte cell or tissue type (e.g., a leukocyte or a solid tissue cell type, such as epithelial cells, muscle cells, etc.).

As used herein, "type-specific" in the context of an epigenetic variation means an epigenetic variation that is present at a detectably different degree in one cell or tissue type, or in a plurality of related cell or tissue types, relative to other cell or tissue types. Similarly, a "type-specific epigenetic target region" is an epigenetic target region that has a detectably different epigenetic characteristic in one cell or tissue type, or in a plurality of related cell or tissue types, relative to other cell or tissue types. Exemplary epigenetic characteristics are discussed in the definition of epigenetic target regions set forth above. For example, a "type-specific differentially methylated region" is a region of DNA that has a detectably different degree of methylation in one cell or tissue type, or in a plurality of related cell or tissue types, relative to other cell or tissue types. Examples of a type-specific differentially methylated region include tissue-specific differentially methylated regions, including those associated with copy-number gain in early cancer. In some embodiments, capturing, identification, and/or detection of type-specific differentially methylated regions facilitates identification of the cell or tissue type from which the DNA originated. The cell or tissue from which a type-specific differentially methylated region originated may be a wild type cell or tissue or a neoplastic cell or tissue. In another example, a "type-specific fragment" of DNA is a DNA fragment arising from a type-specific fragmentation pattern that is present at a detectably different degree in one cell or tissue type, or in a plurality of related cell or tissue types, relative to other cell or tissue types. In some embodiments, a type-specific fragment is only present in the specific cell or tissue type(s). In some embodiments, a type-specific fragment is present to a detectably greater extent in the specific cell or tissue type(s).

As used herein, a "blood sample" refers to a sample comprising whole blood or a component thereof (e.g., plasma, serum, buffy coat, plasma pellet).

"Buffy coat" refers to the portion of a blood (such as whole blood) or bone marrow sample that contains all or most of the white blood cells and platelets of the sample. The buffy coat fraction of a sample can be prepared from the sample using centrifugation, which separates sample components by density. For example, following centrifugation of a whole blood sample, the buffy coat fraction is situated between the plasma and erythrocyte (red blood cell) layers. The buffy coat can contain both mononuclear (e.g., T cells, B cells, NK cells, dendritic cells, and monocytes) and polymorphonuclear (e.g., granulocytes such as neutrophils and eosinophils) white blood cells.

As used herein, "primer-annealed DNA" means DNA to which at least one primer is annealed.

An "intronic region," of DNA herein encodes an intron or a portion thereof. Intronic regions include regions that encode introns removed during post-transcriptional splicing of pre-mRNA to generate mRNA and also "J intronic regions," which are sequences that intervene between germline J gene segments (e.g., in immunoglobulin or T cell receptor loci) and are removed during somatic V(D)J recombination. An "exonic region" of DNA herein encodes at least one exon or a portion thereof in pre-mRNA or mRNA. In some embodiments, an exonic region is a VDJ exonic region, meaning that it encodes one or more V, D, or J exons in pre-mRNA or mRNA. An "exon-exon junction region" of DNA herein encodes at least one exon-exon junction in pre-mRNA (e.g., immunoglobulin or T cell receptor pre-mRNA) before any introns are removed by splicing. Exon-exon junction regions can be formed by V(D)J recombination, e.g., when a J segment is joined to a D or V segment or a V segment is joined to a D or J segment.

As used herein, a DNA "structural variation" or "structural variant" is a mutation comprising a DNA sequence not present in the wild-type genome other than a point mutation (e.g., in which at least 5, 10, 20, or 50 contiguous nucleotides are different relative to the wild type sequence at the corresponding locus). Examples of DNA structural variations include rearrangements, such as translocations, insertions, deletions, duplications, copy-number variants, and inversions. As used herein, "structural variation sequence" or "structural variant sequence" is a DNA sequence that comprises or consists of a portion of or the entirety of a structural variation.

As used herein, "partitioning" of nucleic acids, such as DNA molecules, means separating, fractionating, sorting, or enriching a sample or population of nucleic acids into a plurality of subsamples or subpopulations of nucleic acids based on one or more modifications or features that is in different proportions in each of the plurality of subsamples or subpopulations. Partitioning may include physically partitioning nucleic acid molecules based on the presence or absence of one or more methylated nucleobases. A sample or population may be partitioned into one or more partitioned subsamples or subpopulations based on a characteristic that is indicative of a genetic or epigenetic change or a disease state.

As used herein, the form of the "originally isolated" sample refers to the composition or chemical structure of a sample at the time it was isolated and before undergoing any procedure that changes the chemical structure of the isolated sample. Similarly, a feature that is "originally present" in a molecule refers to a feature present in an "original molecule" or in molecules "originally comprising" the feature before the molecule undergoes any procedure that changes the chemical structure of the molecule.

As used herein, "without substantially altering base pairing specificity" of a given nucleobase means that a majority of molecules comprising that nucleobase that can be sequenced do not have alterations of the base pairing specificity of the given nucleobase relative to its base pairing specificity as it was in the originally isolated sample. In some embodiments, 75%, 90%, 95%, or 99% of molecules comprising that nucleobase that can be sequenced do not have alterations of the base pairing specificity relative to its base pairing specificity as it was in the originally isolated sample. As used herein, "altered base pairing specificity" of a given nucleobase means that a majority of molecules comprising that nucleobase that can be sequenced have a base pairing specificity at that nucleobase relative to its base pairing specificity in the originally isolated sample.

As used herein, a "combination" comprising a plurality of members refers to either of a single composition comprising the members or a set of compositions in proximity, e.g., in separate containers or compartments within a larger container, such as a multiwell plate, tube rack, refrigerator, freezer, incubator, water bath, ice bucket, machine, or other form of storage.

As used herein, a "label" is a capture moiety, fluorophore, oligonucleotide, or other moiety that facilitates detection, separation, or isolation of that to which it is attached.

As used herein, a "capture moiety" is a molecule that allows affinity separation of molecules linked to the capture moiety from molecules lacking the capture moiety. Exemplary capture moieties include biotin, which allows affinity separation by binding to streptavidin linked or linkable to a solid phase or an oligonucleotide, which allows affinity separation through binding to a complementary oligonucleotide linked or linkable to a solid phase.

As used herein, a "capture probe" means a probe comprising a capture moiety and that is generated by amplification and thus comprises an amplicon of the template DNA. In some embodiments, the amplification comprises polymerase chain reaction (PCR).

As used herein, "anti-parallel orientation" of two primers means that the primers anneal to a nucleic acid in opposite orientations relative to each other (e.g., to opposite strands of the nucleic acid) and/or in an orientation that is compatible with exponential PCR amplification. For example, primers that anneal to a rearrangement in an anti-parallel orientation can facilitate amplification of an amplicon comprising the rearrangement breakpoint.

As used herein, a "tag" is a molecule, such as a nucleic acid, label, fluorophore, or peptide, containing information that indicates a feature of the molecule to which the tag is associated. For example, molecules can bear a sample tag (which distinguishes molecules in one sample from those in a different sample), a molecular tag/molecular barcode/barcode (which distinguishes different molecules from one another (in both unique and non-unique tagging scenarios), a purification tag, and/or a detectable tag or label.

"Specifically binds" in the context of an probe or other oligonucleotide and a target sequence means that under appropriate hybridization conditions, the oligonucleotide or probe hybridizes to its target sequence, or replicates thereof, to form a stable probe:target hybrid, while at the same time formation of stable probe: non-target hybrids is minimized. Thus, a probe hybridizes to a target sequence or replicate thereof to a sufficiently greater extent than to a non-target sequence, to enable capture or detection of the target sequence. Appropriate hybridization conditions are well-known in the art, may be predicted based on sequence composition, or can be determined by using routine testing methods (see, e.g., Sambrook et al., Molecular Cloning, A Laboratory Manual, 2nd ed. (Cold Spring Harbor Laboratory Press, Cold Spring Harbor, NY, 1989) at §§ 1.90-1.91, 7.37-7.57, 9.47-9.51 and 11.47-11.57, particularly §§ 9.50-9.51, 11.12-11.13, 11.45-11.47 and 11.55-11.57).

A nucleic acid is "produced by a tumor" or ctDNA or circulating tumor DNA, if it originated from a tumor cell. Tumor cells are neoplastic cells that originated from a tumor, regardless of whether they remain in the tumor or become separated from the tumor (as in the cases, e.g., of metastatic cancer cells and circulating tumor cells).

A "target region" in the context of a nucleic acid refers to a genomic locus targeted for identification and/or capture, for example, by using probes (e.g., through sequence complementarity). A "target region set" or "set of target regions" refers to a plurality of genomic loci targeted for identification and/or capture, for example, by using a set of probes (e.g., through sequence complementarity).

The "capture yield" of a collection of probes for a given target region set refers to the amount (e.g., amount relative to another target region set or an absolute amount) of nucleic acid corresponding to the target region set that the collection of probes captures under typical conditions. Exemplary typical capture conditions are an incubation of the sample nucleic acid and probes at 65°C for 10-18 hours in a small reaction volume (about 20 µL) containing stringent hybridization buffer. The capture yield may be expressed in absolute terms or, for a plurality of collections of probes, relative terms. When capture yields for a plurality of sets of target regions are compared, they are normalized for the footprint size of the target region set (e.g., on a per-kilobase basis). Thus, for example, if the footprint sizes of first and second target regions are 50 kb and 500 kb, respectively (giving a normalization factor of 0.1), then the DNA corresponding to the first target region set is captured with a higher yield than DNA corresponding to the second target region set when the mass per volume concentration of the captured DNA corresponding to the first target region set is more than 0.1 times the mass per volume concentration of the captured DNA corresponding to the second target region set. As a further example, using the same footprint sizes, if the captured DNA corresponding to the first target region set has a mass per volume concentration of 0.2 times the mass per volume concentration of the captured DNA corresponding to the second target region set, then the DNA corresponding to the first target region set was captured with a two-fold greater capture yield than the DNA corresponding to the second target region set.

The term "methylation" or "DNA methylation" refers to addition of a methyl group to a nucleotide base in a nucleic acid molecule. In some embodiments, methylation refers to addition of a methyl group to a cytosine at a CpG site (cytosine-phosphate-guanine site (i.e., a cytosine followed by a guanine in a 5' → 3' direction of the nucleic acid sequence)). In some embodiments, DNA methylation refers to addition of a methyl group to adenine, such as in N⁶-methyladenine. In some embodiments, DNA methylation is 5-methylation (modification of the carbon in the 5th position of the cytosine ring). In some embodiments, 5-methylation refers to addition of a methyl group to the 5C position of the cytosine to create 5-methylcytosine (5mC). In some embodiments, methylation comprises a derivative of 5mC. Derivatives of 5mC include, but are not limited to, 5-hydroxymethylcytosine (5-hmC), 5-formylcytosine (5-fC), and 5-caryboxylcytosine (5-caC). In some embodiments, DNA methylation is 3C methylation (modification of the carbon in the 3^{rd} position of the cytosine ring). In some embodiments, 3C methylation comprises addition of a methyl group to the 3C position of the cytosine to generate 3-methylcytosine (3mC). Methylation can also occur at non CpG sites, for example, methylation can occur at a CpA, CpT, or CpC site. DNA methylation can change the activity of methylated DNA region. For example, when DNA in a promoter region is methylated, transcription of the gene may be repressed. DNA methylation is critical for normal development and abnormality in methylation may disrupt epigenetic regulation. The disruption, e.g., repression, in epigenetic regulation may cause diseases, such as cancer. Promoter methylation in DNA may be indicative of cancer.

The "modified nucleoside profile of DNA" means the position and identity of the nucleoside and the modification status of the nucleoside, such as methylations, within a DNA sequence. As described above, different methods of conversion followed by sequencing detect one or more different types of modified or unmodified nucleoside. For example, the TAPS method detects, but does not distinguish between, 5-methylcytosine (5mC) and 5-hydroxymethyl-cytosine (5hmC). Hence, a method for analyzing the modified nucleoside profile of DNA in a sample typically means identifying particular modifications or groups of modification, such as 5mC and/or 5hmC. Modified nucleosides are identified according to the specific method/conversion procedure being used as described above. This generally involves comparing sequence data obtained from DNA that has been subjected to a conversion procedure to a reference sequence. Typically, the method involves (i) comparing the sequence data with (A) one or more pre-determined reference sequence, typically corresponding to one or more epigenetic target regions where particular significance is attached to the modified nucleoside profile, e.g. in diagnosing, prognosing or characterizing a cancer; or (B) sequence data obtained by sequencing a sub-sample of the DNA that was not subjected to the conversion procedure, for example a subsample that was separated before subjecting a separate subsample to the conversion procedure, for example as described herein; and (ii) identifying point differences between the converted DNA sequences and the reference sequence(s) (A) or non-converted DNA sequences (B) as nucleosides (in the initial sample) having a modification status that permits a change in base pairing specificity on exposure to the conversion procedure.

It is recognized that the modified nucleoside profile determined by standard conversion and sequencing methods may contain errors due to incomplete or erroneous conversion of modified or unmodified nucleosides in the sample. The method of the disclosure provides a means for assessing the conversion rate on either a sample or molecular basis.

The term "hypermethylation" refers to an increased level or degree of methylation of nucleic acid molecule(s) relative to the other nucleic acid molecules within a population (e.g., sample) of nucleic acid molecules. In some embodiments, hypermethylated DNA can include DNA molecules comprising at least 1 methylated residue, at least 2 methylated residues, at least 3 methylated residues, at least 5 methylated residues, or at least 10 methylated residues. As used herein, "type-specific hypermethylation" means an increased level or degree of methylation of DNA in at one cell or tissue type, or in a plurality of related cell or tissue types, relative to other cell or tissue types. In some embodiments, capturing, identification, and/or detection of type-specific hypermethylated regions facilitates identification of the cell or tissue type from which the DNA originated. The cell or tissue from which a type-specific hypermethylated region originated may be a wild type cell or tissue or a neoplastic cell or tissue.

The term "hypomethylation" refers to a decreased level or degree of methylation of nucleic acid molecule(s) relative to the other nucleic acid molecules within a population (e.g., sample) of nucleic acid molecules. In some embodiments, hypomethylated DNA includes unmethylated DNA molecules. In some embodiments, hypomethylated DNA can include DNA molecules comprising 0 methylated residues, at most 1 methylated residue, at most 2 methylated residues, at most 3 methylated residues, at most 4 methylated residues, or at most 5 methylated residues. As used herein, "type-specific hypomethylation" means a decreased level or degree of methylation of DNA in at one cell or tissue type, or in a plurality of related cell or tissue types, relative to other cell or tissue types. In some embodiments, capturing, identification, and/or detection of type-specific hypomethylated regions facilitates identification of the cell or tissue type from which the DNA originated. The cell or tissue from which a type-specific hypomethylated region originated may be a wild type cell or tissue or a neoplastic cell or tissue.

The terms "agent that recognizes a modified nucleobase in DNA," such as an "agent that recognizes a modified cytosine in DNA" refers to a molecule or reagent that binds to or detects one or more modified nucleobases in DNA, such as methyl cytosine.

A "modified nucleoside" is a nucleoside that comprises a difference in chemical structure from an unmodified nucleoside. In the case of DNA, an unmodified nucleoside comprises a deoxyribosyl and one of adenine, cytosine, guanine, or thymine. In some embodiments, a modified nucleoside comprises a modified cytosine. In some embodiments, a modified nucleoside comprises a methylated nucleobase. In some embodiments, a modified cytosine is a methyl cytosine, e.g., a 5-methyl cytosine. In such embodiments, the cytosine modification is a methyl. Agents that recognize a methyl cytosine in DNA include but are not limited to "methyl binding reagents," which refer herein to reagents that bind to a methyl cytosine. Methyl binding reagents include but are not limited to methyl binding domains (MBDs) and methyl binding proteins (MBPs) and antibodies specific for methyl cytosine. In some embodiments, such antibodies bind to 5-methyl cytosine in DNA. In some such embodiments, the DNA may be single-stranded or double-stranded.

The terms "or a combination thereof" and "or combinations thereof" as used herein refers to any and all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

"Or" is used in the inclusive sense, i.e., equivalent to "and/or," unless the context requires otherwise.

### Samples and Subjects

The disclosure relates to methods of analyzing the modified nucleoside profile of nucleic acid or a quality control method for monitoring false negative and/or false positive detection of modified nucleosides in DNA in a sample, e.g. DNA in a sample. In some cases, the nucleic acid is obtained or has been obtained from a subject. In some embodiments, the nucleic acid sample may comprise or consist of nucleic acid , e.g. DNA, from a biological sample obtained from a subject. The subject may be a human, a mammal, an animal, a primate, rodent (including mice and rats), or other common laboratory, domestic, companion, service or agricultural animal, for example a rabbit, dog, cat, horse, cow, sheep, goat or pig. The subject may in some cases have or be suspected of having a cancer, tumor or neoplasm. In other cases, the subject may not have cancer or a detectable cancer symptom. The subject may have been treated with one or more cancer therapy, e.g., any one or more of chemotherapies, antibodies, vaccines or biologics. The subject may be in remission, e.g. from a tumor, cancer, or neoplasia (e.g., following treatment such as chemotherapy, surgical resection, radiation, or a combination thereof). The subject may or may not be diagnosed as being susceptible to cancer or any cancer-associated genetic mutations/disorders. In some embodiments, the sample is a polynucleotide sample obtained from a tumor tissue biopsy. The cancer, tumor or neoplasm may generally be of any type, for example a cancer tumor or neoplasm of the lung, colon, rectum (or colorectum), kidney, breast, prostate, or liver, or other type of cancer as described herein. In some embodiments, the sample is obtained from a subject in remission from a tumor, cancer, or neoplasia (e.g., following chemotherapy, surgical resection, radiation, or a combination thereof). In any of the foregoing embodiments, the pre-cancer, cancer, tumor, or neoplasia or suspected pre-cancer, cancer, tumor, or neoplasia may be of the bladder, head and neck, lung, colon, rectum, kidney, breast, prostate, skin, or liver. In some embodiments, the pre-cancer, cancer, tumor, or neoplasia or suspected pre-cancer, cancer, tumor, or neoplasia is of the lung. In some embodiments, the pre-cancer, cancer, tumor, or neoplasia or suspected pre-cancer, cancer, tumor, or neoplasia is of the colon or rectum. In some embodiments, the pre-cancer, cancer, tumor, or neoplasia or suspected pre-cancer, cancer, tumor, or neoplasia is of the breast. In some embodiments, the pre-cancer, cancer, tumor, or neoplasia or suspected pre-cancer, cancer, tumor, or neoplasia is of the prostate. In any of the foregoing embodiments, the subject may be a human subject. In some embodiments, the sample is obtained from a subject having a stage I cancer, stage II cancer, stage III cancer or stage IV cancer.

The sample can be any biological sample isolated from a subject. The sample can be a bodily sample. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another.

In some embodiments, a population of nucleic acids is obtained from a serum, plasma or blood sample from a subject suspected of having neoplasia, a tumor, precancer, or cancer or previously diagnosed with neoplasia, a tumor, precancer, or cancer. The population includes nucleic acids having varying levels of sequence variation, epigenetic variation, and/or post-replication or transcriptional modifications. Post-replication modifications include modifications of cytosine, particularly at the 5-position of the nucleobase, e.g., 5-methylcytosine, 5-hydroxymethylcytosine, 5-formylcytosine and 5-carboxylcytosine.

A sample can be isolated or obtained from a subject and transported to a site of sample analysis. The sample may be preserved and shipped at a desirable temperature, e.g., room temperature, 4°C, -20°C, and/or -80°C. A sample can be isolated or obtained from a subject at the site of the sample analysis. The subject can be a human, a mammal, an animal, a companion animal, a service animal, or a pet. The subject may have a cancer, precancer, infection, transplant rejection, or other disease or disorder related to changes in the immune system. The subject may not have cancer or a detectable cancer symptom. The subject may have been treated with one or more cancer therapy, e.g., any one or more of chemotherapies, antibodies, vaccines or biologies. The subject may be in remission. The subject may or may not be diagnosed of being susceptible to cancer or any cancer-associated genetic mutations/disorders.

The volume of plasma can depend on the desired read depth for sequenced regions. Exemplary volumes are 0.4-40 ml, 5-20 ml, 10-20 ml. For examples, the volume can be 0.5 mL, 1 mL, 5 mL 10 mL, 20 mL, 30 mL, or 40 mL. A volume of sampled plasma may be 5 to 20 mL.

A sample can comprise various amount of nucleic acid that contains genome equivalents. For example, a sample of about 30 ng DNA can contain about 10,000 (10⁴) haploid human genome equivalents and, in the case of cell free DNA (cfDNA), about 200 billion (2x10¹¹) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells and cell-free of the same subject, from cells and cell-free of different subjects. A sample can comprise nucleic acids carrying mutations. For example, a sample can comprise DNA carrying germline mutations and/or somatic mutations. Germline mutations refer to mutations existing in germline DNA of a subject. Somatic mutations refer to mutations originating in somatic cells of a subject, e.g., cancer cells. A sample can comprise DNA carrying cancer-associated mutations (e.g., cancer-associated somatic mutations). A sample can comprise an epigenetic variant (i.e. a chemical or protein modification), wherein the epigenetic variant associated with the presence of a genetic variant such as a cancer-associated mutation. In some embodiments, the sample comprises an epigenetic variant associated with the presence of a genetic variant, wherein the sample does not comprise the genetic variant.

The sample may be or comprise cell free nucleic acids or cfDNA. The cfDNA may be obtained from a test subject, for example as described above. For example, the sample for analysis may be plasma or serum containing cell-free nucleic acids. "Cell-free DNA" "cfDNA molecules," or "cfDNA", for example, include DNA molecules that naturally occur in a subject in extracellular form (e.g., in blood, serum, plasma, or other bodily fluids such as lymph, cerebrospinal fluid, urine, or sputum). While the cfDNA originally existed in a cell or cells in a large complex biological organism, e.g., a mammal, it has undergone release from the cell(s) *in vivo* into a fluid found in the organism, and may be obtained by obtaining a sample of the fluid without the need to perform an *in vitro* cell lysis step. In other words, cell-free nucleic acids or DNA are nucleic acids or DNA not contained within or otherwise bound to a cell, or the nucleic acids or DNA remaining in a sample after removing intact cells. Cell-free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells. In some embodiments, cfDNA is cell-free fetal DNA (cffDNA). In some embodiments, cell free nucleic acids are produced by tumor cells. In some embodiments, cell free nucleic acids are produced by a mixture of tumor cells and non-tumor cells.

Exemplary amounts of cell-free nucleic acids in a sample before amplification range from about 1 fg to about 1 µg, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng of cell-free nucleic acid molecules from samples.

Cell-free DNA refers to DNA not contained within a cell at the time of its isolation from a subject. For example, cfDNA can be isolated from a sample as the DNA remaining in the sample after removing intact cells, without lysing the cells or otherwise extracting intracellular DNA. Cell- free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells. In some embodiments, cfDNA is cell-free fetal DNA (cffDNA) In some embodiments, cell free nucleic acids are produced by tumor cells. In some embodiments, cell free nucleic acids are produced by a mixture of tumor cells and non-tumor cells.

Cell-free nucleic acids have an exemplary size distribution of about 100-500 nucleotides, with molecules of 110 to about 230 nucleotides representing about 90% of molecules, with a mode of about 168 nucleotides and a second minor peak in a range between 240 to 440 nucleotides.

Cell-free nucleic acids can be isolated from bodily fluids through a fractionation or partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica-based columns to remove contaminants or salts. Non-specific bulk carrier nucleic acids, such as C 1 DNA, DNA or protein for bisulfite sequencing, hybridization, and/or ligation, may be added throughout the reaction to optimize certain aspects of the procedure such as yield.

After such processing, samples can include various forms of nucleic acid including double stranded DNA, single stranded DNA and single stranded RNA. In some embodiments, single stranded DNA and RNA can be converted to double stranded forms so they are included in subsequent processing and analysis steps.

Double-stranded DNA molecules in a sample and single stranded nucleic acid molecules converted to double stranded DNA molecules can be linked to adapters at either one end or both ends. Typically, double stranded molecules are blunt ended by treatment with a polymerase with a 5'-3' polymerase and a 3 '-5' exonuclease (or proof reading function), in the presence of all four standard nucleotides. Klenow large fragment and T4 polymerase are examples of suitable polymerase. The blunt ended DNA molecules can be ligated with at least partially double stranded adapter (e.g., a Y shaped or bell-shaped adapter). Alternatively, complementary nucleotides can be added to blunt ends of sample nucleic acids and adapters to facilitate ligation. Contemplated herein are both blunt end ligation and sticky end ligation. In blunt end ligation, both the nucleic acid molecules and the adapter tags have blunt ends. In sticky-end ligation, typically, the nucleic acid molecules bear an "A" overhang and the adapters bear a "T" overhang.

### Ligation to Adapters

Double-stranded nucleic acids e.g., DNA molecules in a sample, and single stranded nucleic acid molecules converted to double stranded molecules, can be linked to adapters at either one end or both ends. In some cases, the DNA is made ligatable, e.g., by extending the end overhangs of the DNA molecules, and adding adenosine residues to the 3' ends of fragments and phosphorylating the 5' end of each DNA fragment. Typically, double stranded molecules are blunt ended by treatment with a polymerase with a 5'-3' polymerase and a 3'-5' exonuclease (or proof-reading function), in the presence of all four standard nucleotides. Klenow large fragment and T4 polymerase are examples of suitable polymerase.

The blunt ended DNA molecules can be ligated with at least partially double stranded adapter (e.g., a Y shaped or bell-shaped adapter). Alternatively, complementary nucleotides can be added to blunt ends of sample nucleic acids and adapters to facilitate ligation. Contemplated herein are both blunt end ligation and sticky end ligation. In blunt end ligation, both the nucleic acid molecules and the adapter tags have blunt ends. In sticky-end ligation, typically, the nucleic acid molecules bear an "A" overhang and the adapters bear a "T" overhang.

DNA ligase and adapters are added to ligate DNA molecules in the sample with an adapter on one or both ends, i.e. to form adapted DNA. As used herein, "adapter" refers to short nucleic acids (e.g., less than about 500, less than about 100 or less than about 50 nucleotides in length, or be 20-30, 20-40, 30-50, 30-60, 40-60, 40-70, 50-60, 50-70, 20-500, or 30-100 bases from end to end) that are typically at least partially double-stranded and can be ligated to the end of a given sample nucleic acid molecule. In some instances, two adapters can be ligated to a single sample nucleic acid molecule, with one adapter ligated to each end of the sample nucleic acid molecule.

Adapters can include nucleic acid primer binding sites to permit amplification of a sample nucleic acid molecule flanked by adapters at both ends, and/or a sequencing primer binding site, including primer binding sites for sequencing applications, such as various next generation sequencing (NGS) applications. Adapters can include a sequence for hybridizing to a solid support, e.g., a flow cell sequence. Adapters can also include binding sites for capture probes, such as an oligonucleotide attached to a flow cell support or the like. Adapters can also include sample indexes and/or molecular barcodes. These are typically positioned relative to amplification primer and sequencing primer binding sites, such that the sample index and/or molecular barcode is included in amplicons and sequencing reads of a given nucleic acid molecule. Adapters of the same or different sequence can be linked to the respective ends of a sample nucleic acid molecule. In some cases, adapters of the same or different sequence are linked to the respective ends of the nucleic acid molecule except that the sample index and/or molecular barcode differs in its sequence. In some embodiments, the adapter is a Y-shaped adapter in which one end is blunt ended or tailed as described herein, for joining to a nucleic acid molecule, which is also blunt ended or tailed with one or more complementary nucleotides to those in the tail of the adapter. In another exemplary embodiment, an adapter is a bell-shaped adapter that includes a blunt or tailed end for joining to a nucleic acid molecule to be analyzed. Other exemplary adapters include T-tailed, C-tailed or hairpin shaped adapters. For example, a hairpin shaped adaptor can comprise a complementary double stranded portion and a loop portion, where the double stranded portion can be attached {e.g. , ligated) to a double-stranded polynucleotide. Hairpin shaped sequencing adaptors can be attached to both ends of a polynucleotide fragment to generate a circular molecule, which can be sequenced multiple times. The adapters used in the methods of the present disclosure comprise one or more known modified nucleosides, such as methylated nucleosides. In instances where two adapters are ligated to a sample nucleic acid (one at each end), either or both of the adapters may comprise one or more known modified nucleosides. Typically, the primer binding site(s), sequencing primer binding site(s), sample index(es) and/or molecular barcode(s), if present, do not comprise the known modified nucleosides that change base pairing specificity as a result of the conversion procedure.

In some embodiments, sample nucleic acids flanked by adapters can be amplified by PCR and other amplification methods. Such amplification can be used to increase the amount of DNA available for subsequent steps, such as sequencing and may be performed in addition to selective amplification of DNA comprising a rearranged sequence. For example, this kind of amplification can be performed as part of library preparation (e.g., before selective amplification of DNA comprising a rearranged sequence) and/or after preparation of a targeted library (which would be after selective amplification of DNA comprising a rearranged sequence). Amplification can be primed by primers binding to primer binding sites in adapters flanking a DNA molecule to be amplified. Amplification methods can involve cycles of denaturation, annealing and extension, resulting from thermocycling or can be isothermal as in transcription-mediated amplification. Other amplification methods include the ligase chain reaction, strand displacement amplification, nucleic acid sequence-based amplification, and self-sustained sequence based replication.

In some embodiments, the present methods perform dsDNA ligations with T-tailed and C-tailed adapters, which result in amplification of at least 50, 60, 70 or 80% of double stranded nucleic acids before linking to adapters. Preferably the present methods increase the amount or number of amplified molecules relative to control methods performed with T-tailed adapters alone by at least 10, 15 or 20%.

In some embodiments, adapters may be added to the DNA or a subsample thereof. Adapters can be ligated to DNA at any point in the methods herein. In some embodiments, adapters are ligated to the DNA of a sample or subsample thereof prior to annealing primers to the DNA for capture probe generation. In some such embodiments, the adapter-ligated DNA is amplified prior to annealing primers to the DNA for capture probe generation. In some embodiments, adapters are ligated to the DNA of a sample or subsample thereof before the DNA is contacted with the capture probes. In some embodiments, the DNA to which the adapters are ligated is in the same sample or subsample as the DNA used as a template to generate capture probes. In some embodiments, the DNA to which the adapters are ligated is in a different sample or subsample, e.g., a second sample or a second subsample of a first sample, than the DNA used as a template to generate capture probes. In some embodiments, the adapters ligated to DNA captured by the capture probes.

In some embodiments, the primers used to generate capture probes are not complementary to adapters, and the resulting capture probes therefore do not comprise adapters. Adapter-ligated DNA can therefore be selectively amplified in the presence of capture probes that do not comprise adapters. Similarly, adapter-ligated DNA can be separated from DNA that does not comprise adapters.

In some embodiments, the disclosed methods comprise analyzing DNA in a sample. In such methods, adapters may be added to the DNA. This may be done concurrently with an amplification procedure, e.g., by providing the adapters in a 5' portion of a primer (where PCR is used, this can be referred to as library prep-PCR or LP-PCR), before, of after an amplification step. In some embodiments, adapters are added by other approaches, such as ligation. In some such methods, first adapters are added to the 3' ends of the nucleic acids by ligation, which may include ligation to single-stranded DNA. In some embodiments, prior to any partitioning or capturing steps, first adapters are added to the nucleic acids by ligation, which may include ligation to single-stranded DNA (e.g., to the 3' ends thereof). In some embodiments, the capture probes can be isolated after partitioning and ligation. For example, the hypomethylated partition can be ligated with adapters and a portion of the ligated hypomethylated partition can then be used to generate the capture probes for rearrangements. The adapter can be used as a priming site for second-strand synthesis, e.g., using a universal primer and a DNA polymerase. A second adapter can then be ligated to at least the 3' end of the second strand of the now double-stranded molecule. In some embodiments, the first adapter comprises an affinity tag, such as biotin, and nucleic acid ligated to the first adapter is bound to a solid support (e.g., bead), which may comprise a binding partner for the affinity tag such as streptavidin. For further discussion of a related procedure, see Gansauge et al., Nature Protocols 8:737-748 (2013). Commercial kits for sequencing library preparation compatible with single-stranded nucleic acids are available, e.g., the Accel-NGS^{®} Methyl-Seq DNA Library Kit from Swift Biosciences. In some embodiments, after adapter ligation, nucleic acids are amplified.

In some embodiments, the adapters include different tags of sufficient numbers that the number of combinations of tags results in a low probability e.g., 95, 99 or 99.9% of two nucleic acids with the same start and stop points receiving the same combination of tags. Adapters, whether bearing the same or different tags, can include the same or different primer binding sites, but preferably adapters include the same primer binding site.

In some embodiments, following attachment of adapters, the nucleic acids are subject to amplification. The amplification can use, e.g., universal primers that recognize primer binding sites in the adapters.

In some embodiments, following attachment of adapters, the DNA or a subsample or portion of the DNA is partitioned, comprising contacting the DNA with an agent that preferentially binds to nucleic acids bearing an epigenetic modification. The nucleic acids are partitioned into at least two partitioned subsamples differing in the extent to which the nucleic acids bear the modification from binding to the agents. For example, if the agent has affinity for nucleic acids bearing the modification, nucleic acids overrepresented in the modification (compared with median representation in the population) preferentially bind to the agent, whereas nucleic acids underrepresented for the modification do not bind or are more easily eluted from the agent. The nucleic acids can then be amplified from primers binding to the primer binding sites within the adapters. Partitioning may be performed instead before adapter attachment, in which case the adapters may comprise differential tags that include a component that identifies which partition a molecule occurred in.

In some embodiments, the nucleic acids are linked at both ends to Y-shaped adapters including primer binding sites and tags. The molecules are amplified.

### Molecular Tagging

In some embodiments, the nucleic acid molecules of the sample may be tagged with sample indexes and/or molecular barcodes (referred to generally as "tags").

Tags or indexes can be molecules, such as nucleic acids, containing information that indicates a feature of the molecule with which the tag is associated. For example, molecules can bear a sample tag or sample index (which distinguishes molecules in one sample from those in a different sample), a partition tag (which distinguishes molecules in one partition from those in a different partition) and/or a molecular tag/molecular barcode/barcode (which distinguishes different molecules from one another (in both unique and non-unique tagging scenarios).

Tagging strategies can be divided into unique tagging and non-unique tagging strategies. In unique tagging, all or substantially all of the molecules in a sample bear a different tag, so that reads can be assigned to original molecules based on tag information alone. Tags used in such methods are sometimes referred to as "unique tags". In non-unique tagging, different molecules in the same sample can bear the same tag, so that other information in addition to tag information is used to assign a sequence read to an original molecule. Such information may include start and stop coordinate, coordinate to which the molecule maps, start or stop coordinate alone, etc. Tags used in such methods are sometimes referred to as "non-unique tags". Accordingly, it is not necessary to uniquely tag every molecule in a sample. It suffices to uniquely tag molecules falling within an identifiable class within a sample. Thus, molecules in different identifiable families can bear the same tag without loss of information about the identity of the tagged molecule.

In certain embodiments, a tag can comprise one or a combination of barcodes. As used herein, the term "barcode" refers to a nucleic acid molecule having a particular nucleotide sequence, or to the nucleotide sequence, itself, depending on context. A barcode can have, for example, between 10 and 100 nucleotides. A collection of barcodes can have degenerate sequences or can have sequences having a certain Hamming distance, as desired for the specific purpose. So, for example, a molecular barcode can be comprised of one barcode or a combination of two barcodes, each attached to different ends of a molecule. Additionally, or alternatively, for different partitions and/or samples, different sets of molecular barcodes, molecular tags, or molecular indexes can be used such that the barcodes serve as a molecular tag through their individual sequences and also serve to identify the partition and/or sample to which they correspond based the set of which they are a member.

In some embodiments, two or more partitions, e.g., each partition, is/are differentially tagged. Tags can be used to label the individual polynucleotide population partitions so as to correlate the tag (or tags) with a specific partition. Alternatively, tags can be used in embodiments of the disclosure that do not employ a partitioning step. In some embodiments, a single tag can be used to label a specific partition. In some embodiments, multiple different tags can be used to label a specific partition. In embodiments employing multiple different tags to label a specific partition, the set of tags used to label one partition can be readily differentiated for the set of tags used to label other partitions. In some embodiments, the tags may have additional functions, for example the tags can be used to index sample sources or used as unique molecular identifiers (which can be used to improve the quality of sequencing data by differentiating sequencing errors from mutations, for example as in Kinde et al., Proc Nat'l Acad Sci USA 108: 9530-9535 (2011), Kou et al., PLoS ONE,11: e0146638 (2016)) or used as non-unique molecule identifiers, for example as described in US Pat. No. 9,598,731. Similarly, in some embodiments, the tags may have additional functions, for example the tags can be used to index sample sources or used as non-unique molecular identifiers (which can be used to improve the quality of sequencing data by differentiating sequencing errors from mutations).

Tags may be incorporated into or otherwise joined to adapters by chemical synthesis, ligation (e.g., as described above, e.g. by blunt-end ligation or sticky-end ligation), or overlap extension polymerase chain reaction (PCR), among other methods. Such adapters are ultimately joined to the target nucleic acid molecule. In other embodiments, one or more rounds of amplification cycles (e.g., PCR amplification) may be applied to introduce sample indexes to a nucleic acid molecule using conventional nucleic acid amplification methods. The amplifications may be conducted in one or more reaction mixtures (e.g., a plurality of microwells in an array). Molecular barcodes and/or sample indexes may be introduced simultaneously, or in any sequential order. In some embodiments, molecular barcodes and/or sample indexes are introduced prior to and/or after the conversion procedure. In some embodiments, molecular barcodes and/or sample indexes are introduced prior to and/or after sequence capturing steps, if present, are performed. In some embodiments, only the molecular barcodes are introduced prior to probe capturing and the sample indexes are introduced after sequence capturing steps are performed. In some embodiments, both the molecular barcodes and the sample indexes are introduced prior to performing probe-based capturing steps, if present. In some embodiments, the sample indexes are introduced after sequence capturing steps are performed, if present. In some embodiments, sample indexes are incorporated through overlap extension polymerase chain reaction (PCR).

In some embodiments, the tags may be located at one end or at both ends of the sample nucleic acid molecule. In some embodiments, tags are predetermined or random or semi-random sequence oligonucleotides. In some embodiments, the tag(s) may together be less than about 500, 200, 100, 50, 20, 10, 9, 8, 7, 6, 5, 4, 3, 2, or 1 nucleotides in length. Typically, tags are about 5 to 20 or 6 to 15 nucleotides in length. The tags may be linked to sample nucleic acids randomly or non-randomly.

In some embodiments, each sample or partition (discussed below) is uniquely tagged with a sample index or a combination of sample indexes. In some embodiments, each nucleic acid molecule of a sample or sub-sample is uniquely tagged with a molecular barcode or a combination of molecular barcodes. In other embodiments, a plurality of molecular barcodes may be used such that molecular barcodes are not necessarily unique to one another in the plurality (e.g., non-unique molecular barcodes). In these embodiments, molecular barcodes are generally attached (e.g., by ligation) to individual molecules such that the combination of the molecular barcode and the sequence it may be attached to creates a unique sequence that may be individually tracked. Detection of non-unique molecular barcodes in combination with endogenous sequence information (e.g., the beginning (start) and/or end (stop) genomic location/position corresponding to the sequence of the original nucleic acid molecule in the sample, start and stop genomic positions corresponding to the sequence of the original nucleic acid molecule in the sample, the beginning (start) and/or end (stop) genomic location/position of the sequence read that is mapped to the reference sequence, start and stop genomic positions of the sequence read that is mapped to the reference sequence, sub-sequences of sequence reads at one or both ends, length of sequence reads, and/or length of the original nucleic acid molecule in the sample) typically allows for the assignment of a unique identity to a particular molecule. In some embodiments, beginning region comprises the first 1, first 2, the first 5, the first 10, the first 15, the first 20, the first 25, the first 30 or at least the first 30 base positions at the 5' end of the sequencing read that align to the reference sequence. In some embodiments, the end region comprises the last 1, last 2, the last 5, the last 10, the last 15, the last 20, the last 25, the last 30 or at least the last 30 base positions at the 3' end of the sequencing read that align to the reference sequence. The length, or number of base pairs, of an individual sequence read are also optionally used to assign a unique identity to a given molecule. As described herein, fragments from a single strand of nucleic acid having been assigned a unique identity, may thereby permit subsequent identification of fragments from the parent strand, and/or a complementary strand.

In certain embodiments of non-unique tagging, the number of different tags used can be sufficient that there is a very high likelihood (e.g., at least 99%, at least 99.9%, at least 99.99% or at least 99.999% that all DNA molecules of a particular group bear a different tag. It is to be noted that when barcodes are used as tags, and when barcodes are attached, e.g., randomly, to both ends of a molecule, the combination of barcodes, together, can constitute a tag. This number, in term, is a function of the number of molecules falling into the calls. For example, the class may be all molecules mapping to the same start-stop position on a reference genome. The class may be all molecules mapping across a particular genetic locus, e.g., a particular base or a particular region (e.g., up to 100 bases or a gene or an exon of a gene). In certain embodiments, the number of different tags used to uniquely identify a number of molecules, z, in a class can be between any of 2*z, 3*z, 4*z, 5*z, 6*z, 7*z, 8*z, 9*z, 10*z, 11 *z, 12*z, 13*z, 14*z, 15*z, 16*z, 17*z, 18*z, 19*z, 20*z or 100*z (e.g., lower limit) and any of 100,000*z, 10,000*z, 1000*z or 100*z (e.g., upper limit). In some embodiments, molecular barcodes are introduced at an expected ratio of a set of identifiers (e.g., a combination of unique or non-unique molecular barcodes) to molecules in a sample. One example format uses from about 2 to about 1,000,000 different molecular barcode sequences, or from about 5 to about 150 different molecular barcode sequences, or from about 20 to about 50 different molecular barcode sequences, ligated to both ends of a target molecule. Alternatively, from about 25 to about 1,000,000 different molecular barcode sequences may be used. For example, 20-50 x 20-50 molecular barcode sequences (i.e., one of the 20-50 different molecular barcode sequences can be attached to each end of the target molecule) can be used. Such numbers of identifiers are typically sufficient for different molecules having the same start and stop points to have a high probability (e.g., at least 94%, 99.5%, 99.99%, or 99.999%) of receiving different combinations of identifiers. In some embodiments, about 80%, about 90%, about 95%, or about 99% of molecules have the same combinations of molecular barcodes.

For example, in a sample of about 5 ng to 30 ng of cell free DNA, one expects around 3000 molecules to map to a particular nucleotide coordinate, and between about 3 and 10 molecules having any start coordinate to share the same stop coordinate. Accordingly, about 50 to about 50,000 different tags (e.g., between about 6 and 220 barcode combinations) can suffice to uniquely tag all such molecules. To uniquely tag all 3000 molecules mapping across a nucleotide coordinate, about 1 million to about 20 million different tags would be required.

In some embodiments, the assignment of unique or non-unique molecular barcodes in reactions is performed using methods and systems described in, for example, U.S. Patent Application Nos. 20010053519, 20030152490, and 20110160078, and U.S. Patent Nos. 6,582,908, 7,537,898, 9,598,731, and 9,902,992. Alternatively, in some embodiments, different nucleic acid molecules of a sample may be identified using only endogenous sequence information (e.g., start and/or stop positions, sub-sequences of one or both ends of a sequence, and/or lengths).

In some embodiments, the tagged nucleic acids are sequenced after loading into a microwell plate. The microwell plate can have 96, 384, or 1536 microwells. In some cases, they are introduced at an expected ratio of unique tags to microwells. For example, the unique tags may be loaded so that more than about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the unique tags may be loaded so that less than about 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the average number of unique tags loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags per genome sample.

A preferred format uses 20-50 different tags (e.g., barcodes) ligated to both ends of target nucleic acids. For example, 35 different tags (e.g., barcodes) ligated to both ends of target molecules creating 35 x 35 permutations, which equals 1225 for 35 tags. Such numbers of tags are sufficient so that different molecules having the same start and stop points have a high probability (e.g., at least 94%, 99.5%, 99.99%, 99.999%) of receiving different combinations of tags. Other barcode combinations include any number between 10 and 500, e.g., about 15x15, about 35x35, about 75x75, about 100x100, about 250x250, about 500x500.

In some cases, unique tags may be predetermined or random or semi-random sequence oligonucleotides. In other cases, a plurality of barcodes may be used such that barcodes are not necessarily unique to one another in the plurality. In this example, barcodes may be ligated to individual molecules such that the combination of the barcode and the sequence it may be ligated to creates a unique sequence that may be individually tracked. As described herein, detection of non-unique barcodes in combination with sequence data of beginning (start) and end (stop) portions of sequence reads may allow assignment of a unique identity to a particular molecule. The length or number of base pairs, of an individual sequence read may also be used to assign a unique identity to such a molecule. As described herein, fragments from a single strand of nucleic acid having been assigned a unique identity, may thereby permit subsequent identification of fragments from the parent strand.

In some embodiments, the method includes adding one or more internal control DNAs and forward and reverse primers for amplifying the internal control DNAs. The internal control DNAs may be added before amplification using the primers that anneal upstream and downstream of the rearrangement breakpoints. The forward and reverse primers for amplifying the internal control DNAs may be included with, or added at the same time as, the primers that anneal upstream and downstream of the rearrangement breakpoints. The internal control DNAs may comprise or consist of sequences that do not occur in the genome of the subject, or that do not occur in the genome of the species of which the subject is a member (e.g., the human genome). The forward and/or reverse primers for amplifying the internal control DNAs may comprise sequences that are not complementary to any sequence in the genome of the subject, e.g., the human genome. The internal control DNAs may be used to ensure that the amplification process proceeded as designed. As such, the method may comprise detecting (e.g., sequencing) molecules amplified from and/or captured by the one or more internal control DNAs. The method can comprise comparing an amount of internal control DNAs (e.g., number of molecules or reads detected that correspond to an internal control DNA sequence) to a predetermined threshold, and either rejecting sequencing results if the predetermined threshold is not met or accepting sequencing results if the predetermined threshold is met. The predetermined threshold may be established, e.g., based on historical data or by testing the method on samples of DNA from test subjects, such as healthy volunteers. For example, amplification and detection of the one or more internal control DNAs provides confirmation that the amplification process proceeded properly, thus reducing the likelihood of a false negative.

### Conversion Procedure

The use of quality control nucleosides in the adapters, as described in the methods disclosed herein, can advantageously be used with conversion procedures which convert the base pairing specificity of modified nucleosides (e.g., Tet-assisted conversion with a substituted borane reducing agent) or conversion procedures which convert the base pairing specificity of unmodified nucleosides (e.g. bisulfite conversion). For example, in some embodiments, when a molecule comprising adapters containing two or more quality control nucleosides is exposed to a conversion procedure selected to change the base pairing specificity of quality control nucleosides, the base-pairing specificity of a first portion (e.g., at least one) of the quality control nucleosides is changed but the base-pairing specificity of a second portion (e.g., at least one) of the quality control nucleosides in the adapter is unaffected, which can indicate suboptimal conversion. The use of quality control nucleosides in the adapters, as described in the methods disclosed herein, can advantageously be used to predict/infer/indicate false negative detection and/or identification of modified nucleosides in the DNA sample (i.e., incorrectly identifying a base as being unmodified) and/or false positive detection and/or identification of modified nucleosides in the DNA sample (i.e. incorrectly identifying a base as being modified). Quality control nucleosides as described herein for use to detect the occurrence of false positive detection of modified nucleosides may be referred to as "false positive quality control nucleosides". Quality control nucleosides as described herein for use to detect the occurrence of false negative detection of modified nucleosides may be referred to as "false negative quality control nucleosides". A nucleoside having a modification status that means that its base pairing specificity is not changed when exposed to a particular conversion procedure may in some cases be referred to as a "protected" nucleoside or as having a "protected modification status" or similar.

In the case of detecting false negatives using conversion procedures which convert the base pairing specificity of modified nucleosides, the quality control nucleosides in the adapters may comprise modified nucleosides such that the conversion efficiency of the conversion procedure/sub-optimal conversion can measured, and thus the frequency of false negatives predicted. Sub-optimal conversion refers to conversion of fewer than all nucleosides of the type that the reagent used in a conversion procedure normally converts; for example, sub-optimal conversion by Tet enzyme and pyridine borane (e.g., in TAPS) results in conversion of some but not all 5mCs and 5hmCs to dihydrouracil. The terms sub-optimal and suboptimal have equivalent meanings. Sub-optimal conversion may also be referred to as incomplete conversion in the sense that some nucleosides (modified or unmodified) that should have been converted by the conversion procedure in a complete reaction were not actually converted.

In the case of detecting false positives using conversion procedures which convert the base pairing specificity of unmodified nucleosides, the quality control nucleosides in the adapters may comprise modified nucleosides such that the erroneous conversion frequency of modified nucleosides can measured, and thus the frequency of false positives predicted. Erroneous conversion refers to conversion of a nucleoside other than the nucleosides that are typically converted by a conversion procedure. Conversion of a methylated cytosine by bisulfite conversion (which typically converts only unmodified cytosines) is an example of erroneous conversion.

In the case of detecting false positives using conversion procedures which convert the base pairing specificity of modified nucleosides, the quality control nucleosides in the adapters may comprise unmodified nucleosides such that the erroneous conversion frequency of unmodified nucleosides can measured, and thus the frequency of false positives predicted.

In the case of detecting false positives using conversion procedures which convert the base pairing specificity of unmodified nucleosides, the quality control nucleosides in the adapters may comprise unmodified nucleosides such that the conversion efficiency of the conversion procedure/sub-optimal conversion can measured, and thus the frequency of false positives predicted.

There are various methods of detecting and/or identifying modified nucleosides that rely on a conversion procedure that changes the base-pairing specificity of a nucleoside, based on the modification status of the nucleosides. These changes of base-pairing specificity can then be detected, and thus the modification status of the nucleoside inferred, by sequencing.

In some cases, the conversion procedure used in the methods of the disclosure is one that changes the base pairing specificity of a modified nucleoside (e.g., methylated cytosine), but does not change the base pairing specificity of the corresponding unmodified nucleoside (e.g. cytosine) or does not change the base pairing specificity of any un-modified nucleoside (e.g. cytosine, adenosine, guanosine and thymidine (or uracil)). Advantages of methods that do not convert the base-pairing specificity of unmodified nucleosides include reduced loss of sequence complexity, higher sequencing efficiency and reduced alignment losses. Additionally, methods such as TAPS may in some cases be preferred over methods such as bisulfite sequencing and EM-seq (see FIG. 1) because they are less destructive (especially important for low yield samples such as cfDNA) and do not require denaturation, meaning that non-conversion errors are theoretically more likely to be random. In methods that require denaturation for conversion, failure to denature a DNA molecule will result in non-conversion of all bases in the DNA molecule. As biological changes in methylation are predominantly concerted to a localized region of interest, these non-random (localized) conversion can appear as false negatives (non-methylated regions). Random non-conversion methods can maximally affect a low percent of bases within a region, and thus the specificity of methylation change detection can be maximized (reduce false positives) by placing a threshold on % of bases within a region that are methylated/non-methylated. Hence, in some cases, a conversion procedure that does not involve denaturation is preferred.

According to the claimed invention, an adapter comprises a first quality control nucleoside with a first modification status (modified, such as methylated) and a second quality control nucleoside with a second modification status (unmodified). Such adapters can be used to detect both suboptimal conversion and erroneous conversion.

FIG. 3 shows an embodiment of a quality control method for monitoring false negative and/or false positive detection of DNA subjected to a TAPS base conversion procedure. Adapters containing a 5mC (e.g., in a molecular barcode) are ligated to DNA and then subjected to a TAPS conversion procedure, changing base-pairing of the methylated cytosines (sequence read as "T") and not the non-methylated cytosines (still read as "C"). Each strand is sequenced. Molecules that underwent sub-optimal conversion are identified and can be filtered out at least for purposes of determining methylation. In some embodiments, molecules that underwent sub-optimal conversion include ssDNA molecules in which 0/2 barcode 5mCs are converted to T. A sample conversion rate can be calculated by dividing all converted barcode 5mCs by the total of barcode 5mCs, here 31/36 = 86% TAPS conversion rate.

In other cases, the conversion procedure used in the methods of the disclosure is one that changes the base pairing specificity of an unmodified nucleoside (e.g., cytosine), but does not change the base pairing specificity of the corresponding modified nucleoside (e.g., methylated cytosine). Such methods include, for example, bisulfite sequencing.

The skilled person can select a suitable method according to their needs, including which nucleoside modifications are to be detected and/or identified.

In some embodiments, the conversion procedure converts modified nucleosides. In some embodiments, the conversion procedure which converts modified nucleosides comprises Tet-assisted conversion with a substituted borane reducing agent, optionally wherein the substituted borane reducing agent is 2-picoline borane, borane pyridine, tert-butylamine borane, ammonia borane or pyridine borane. In Tet-assisted pic-borane conversion with a substituted borane reducing agent conversion, a TET protein is used to convert mC and hmC to caC, without affecting unmodified C. caC, and fC if present, are then converted to dihydrouracil (DHU) by treatment with 2-picoline borane (pic-borane) or another substituted borane reducing agent such as borane pyridine, tert-butylamine borane, or ammonia borane, also without affecting unmodified C. *See, e.g.,* Liu et al., Nature Biotechnology 2019; 37:424-429 (e.g., at Supplementary Fig. 1 and Supplementary Note 7). DHU is read as a T in sequencing. Thus, when this type of conversion is used, the first nucleobase comprises one or more of mC, fC, caC, or hmC, and the second nucleobase comprises unmodified cytosine. Sequencing of the converted DNA identifies positions that are read as cytosine as being unmodified C positions. Meanwhile, positions that are read as T are identified as being T, mC, fC, caC, or hmC. Performing TAP conversion thus facilitates identifying positions containing unmodified C using the sequence reads obtained. Hence, in these embodiments, the quality control nucleosides in the adapters used in the method comprise methyl-cytosine (5mC) and/or hydroxymethyl-cytosine (hmC). This procedure encompasses Tet-assisted pyridine borane sequencing (TAPS), described in further detail in Liu et al. 2019, supra. In this method Tet enzyme is used to progressively oxidize 5mC and 5hmC to 5fC or 5CaC, then pyridine borane deaminates 5fC, 5CaC to DHU, amplified as T. This procedure encompasses Tet-assisted pyridine borane sequencing (TAPS), described in further detail in Liu et al. 2019, supra.

Alternatively, protection of hmC (e.g., using βGT) can be combined with Tet-assisted conversion with a substituted borane reducing agent, e.g., as described above. In this method (TAPS-β), hmC can be protected from conversion, for example through glucosylation using β-glucosyl transferase (βGT), forming (forming 5-glucosylhydroxymethylcytosine) ghmC. This is described in Yu et al., Cell 2012; 149: 1368-80. Treatment with a TET protein such as mTet1 then converts mC to caC but does not convert C or ghmC. caC is then converted to DHU by treatment with pic-borane or another substituted borane reducing agent such as borane pyridine, tert-butylamine borane, or ammonia borane, also without affecting unmodified C or ghmC. Sequencing of the converted DNA identifies positions that are read as cytosine as being either hmC or unmodified C positions. Meanwhile, positions that are read as T are identified as being T, fC, caC, or mC. Performing TAPSβ conversion on a sample as described herein thus facilitates distinguishing positions containing unmodified C or hmC on the one hand from positions containing mC using the sequence reads obtained. Hence, in these embodiments, the quality control nucleosides in the adapters used in the method may typically comprise both methyl-cytosine (5mC) and hydroxymethyl-cytosine (hmC). This allows the efficiency of each of the two steps to be determined separately. For example, if sequencing of the adapter indicates that both the 5mC and the hmC nucleoside(s) have converted base-pairing specificity, this indicates that the hmC-protecting step was ineffective. If neither the 5mC nor the hmC nucleoside(s) have converted base-pairing specificity, this indicates that (at least) the Tet-assisted conversion was ineffective. If base-pairing of the mC nucleosides, but not the hmC nucleosides, in the adapter have converted base-pairing specificity, then both steps were effective. Alternatively, the efficiency of just one step or the other could be determined by including just mC or hmC as the quality control nucleosides in the adapters. Determining the efficiencies of conversions and/or whether a protecting step was ineffective can be used to determine whether there is sub-optimal conversion, as defined in the methods disclosed herein. For an exemplary description of this type of conversion, *see, e.g.,* Liu et al., Nature Biotechnology 2019; 37:424-429.

In addition to controlling for sub-optimal conversion of modified nucleosides, quality control nucleosides in the adapters can also be used to predict false positives (i.e., nucleosides erroneously classified as being modified). In this case, the quality control nucleosides in the adapters comprise, for appropriate conversion procedures, unmodified C. If sequencing of the adapter indicates that quality control nucleoside(s) have converted the base-pairing specificity, this indicates that the unmodified base (e.g., unmodified C) has been erroneously converted. This information can then be used to predict false positive detection of modified nucleosides (e.g., modified C) in the DNA sample.

In some embodiments, the conversion procedure which converts modified nucleosides comprises chemical-assisted conversion with a substituted borane reducing agent, optionally wherein the substituted borane reducing agent is 2-picoline borane, borane pyridine, tert-butylamine borane, borane pyridine or ammonia borane. In chemical-assisted conversion with a substituted borane reducing agent, an oxidizing agent such as potassium perruthenate (KRuO₄) (also suitable for use in ox-BS conversion) is used to specifically oxidize hmC to fC. Treatment with pic-borane or another substituted borane reducing agent such as borane pyridine, tert-butylamine borane, or ammonia borane converts fC and caC to DHU but does not affect mC or unmodified C. Sequencing of the converted DNA identifies positions that are read as cytosine as being either mC or unmodified C positions. Meanwhile, positions that are read as T are identified as being T, fC, caC, or hmC. Performing this type of conversion as described herein thus facilitates distinguishing positions containing unmodified C or mC on the one hand from positions containing hmC using the sequence reads obtained. Hence, in these embodiments, and when controlling for false negatives (i.e., nucleosides erroneously classified as being unmodified), the adapters used in the method comprise hydroxymethyl-cytosine (hmC). For an exemplary description of this type of conversion, *see, e.g.,* Liu et al., Nature Biotechnology 2019; 37:424-429.

Exemplary conversion procedures that change the base-pairing specificity of modified cytosines have been described. However, the methods described herein could in principle use any modified nucleoside and suitable conversion procedure (i.e., single-base epigenetic conversion assay) that changes the base-pairing specificity of the modified nucleoside and thereby allows the modified base to be distinguished from the corresponding unmodified nucleoside and/or other types of modification when sequenced. For example, any conversion procedure could be used allowing any one of N⁶-methyladenine (mA), N⁶-hydroxymethyladenine (hmA), or N⁶-formyladenine (fA) to be distinguished from any other one or more of N⁶-methyladenine (mA), N⁶-hydroxymethyladenine (hmA), or N⁶-formyladenine (fA) and/or from the corresponding unmodified nucleoside, i.e., unmodified adenosine.

In some embodiments, the conversion procedure converts unmodified nucleosides. In some embodiments, the conversion procedure which converts unmodified nucleosides comprises bisulfite conversion. Treatment with bisulfite converts unmodified cytosine and certain modified cytosine nucleotides (e.g., 5-formyl cytosine (fC) or 5-carboxylcytosine (caC)) to uracil whereas other modified cytosines (e.g., 5-methylcytosine, 5-hydroxylmethylcystosine) are not converted. Thus, where bisulfite conversion is used, the converted nucleobases are inferred as comprising one or more of unmodified cytosine, 5-formyl cytosine, 5-carboxylcytosine, or other cytosine forms affected by bisulfite. The unconverted nucleobases are inferred as comprising one or more of mC and hmC. Sequencing of bisulfite-treated DNA identifies positions that are read as cytosine as being mC or hmC positions. Meanwhile, positions that are read as T are identified as being T or a bisulfite-susceptible form of C, such as unmodified cytosine, 5-formyl cytosine, or 5-carboxylcytosine. Thus, performing bisulfite conversion as described herein thus facilitates identifying positions containing mC or hmC. For an exemplary description of bisulfite conversion, *see, e.g.,* Moss et al., Nat Commun. 2018; 9: 5068.

In some embodiments, the procedure which converts unmodified nucleosides comprises oxidative bisulfite (Ox-BS) conversion. This procedure first converts hmC to fC, which is bisulfite susceptible, followed by bisulfite conversion. Thus, when oxidative bisulfite conversion is used, the converted nucleobases are inferred as comprising one or more of unmodified cytosine, fC, caC, hmC, or other cytosine forms affected by bisulfite. The unconverted nucleobases are inferred as comprising mC. Sequencing of Ox-BS converted DNA identifies positions that are read as cytosine as being mC positions. Meanwhile, positions that are read as T are identified as being T, hmC, or a bisulfite-susceptible form of C, such as unmodified cytosine, fC, or hmC. Performing Ox-BS conversion thus facilitates identifying positions containing mC. For an exemplary description of oxidative bisulfite conversion, *see, e.g.,* Booth et al., Science 2012; 336: 934-937.

In some embodiments, the procedure which converts unmodified nucleosides comprises Tet-assisted bisulfite (TAB) conversion. In TAB conversion, hmC is protected from conversion and mC is oxidized in advance of bisulfite treatment, so that positions originally occupied by mC are converted to U while positions originally occupied by hmC remain as a protected form of cytosine. For example, as described in Yu et al., Cell 2012; 149: 1368-80, β-glucosyl transferase can be used to protect hmC (forming 5-glucosylhydroxymethylcytosine (ghmC)), then a TET protein such as mTet1 can be used to convert mC to caC, and then bisulfite treatment can be used to convert C and caC to U while ghmC remains unaffected. Thus, when TAB conversion is used, the converted nucleobases are inferred as comprising one or more of unmodified cytosine, fC, caC, mC, or other cytosine forms affected by bisulfite The unconverted nucleobases are inferred as comprising hmC. Sequencing of TAB-converted DNA identifies positions that are read as cytosine as being hmC positions. Meanwhile, positions that are read as T are identified as being T, mC, or a bisulfite-susceptible form of C, such as unmodified cytosine, fC, or caC. Performing TAB conversion on a first subsample as described herein thus facilitates identifying positions containing hmC.

In some embodiments, the conversion procedure which converts unmodified nucleosides comprises APOBEC-coupled epigenetic (ACE) conversion. In ACE conversion, an AID/APOBEC family DNA deaminase enzyme such as APOBEC3A (A3A) is used to deaminate unmodified cytosine and mC without deaminating hmC, fC, or caC. Sequencing of ACE-converted DNA identifies positions that are read as cytosine as being hmC, fC, or caC positions. Meanwhile, positions that are read as T are identified as being T, unmodified C, or mC. Performing ACE conversion as described herein thus facilitates distinguishing positions containing hmC from positions containing mC or unmodified C using the sequence reads obtained from the first subsample. For an exemplary description of ACE conversion, *see, e.g.,* Schutsky et al., Nature Biotechnology 2018; 36: 1083-1090.

In some embodiments, the conversion procedure includes enzymatic protection of modified cytosines followed by deamination of unprotected cytosines to uracil, which is then read as T. Prior to enzymatic protection of modified cytosines and deamination, hairpins are ligated to the DNA, where the hairpin connects the complementary DNA strands. The DNA strands are then separated and the missing strand is synthesized. Adapters are ligated to the DNA, the hairpin opened, and the PCR amplified. Each side of the hairpin is read and represents the same stretch of DNA, and a set of resolution rules resolves reads into one of A, unmodified C, modified C, G, or T. For an exemplary description of this conversion procedure, *see, e.g.,* Fullgrabe et al., bioRxiv 2022; 07.08.499285.

In particular embodiments, methods of the present disclosure have utility in providing a quality control method for the identification of methylated cytosines which are not present in any sequence context (i.e. CpG and CpH cytosines). Methylated CpH or non-CpG cytosines are infrequent and thus require high levels of sensitivity to reliably detect. Additionally methylated CpGs that co-locate with methylated non-CpGs cannot be detected by methods that use methylation status of non-CpG cytosines as indicator of sub-optimal molecular conversion. The methods of the present disclosure achieve this by providing quality control nucleosides which are known to have a particular modification status, and thus provide a reliable measure of the frequency of erroneous conversion and/or sub-optimal conversion.

In some embodiments, methods of the present disclosure comprise analysis of sequence variations and/or fragmentation patterns, and do not exclude adapted DNA with sub-optimal or erroneous conversion of quality control nucleosides from analysis of sequence variations and/or fragmentation patterns. For example, the methods can comprise detecting the presence or absence of sequence variations and/or determining fragmentation patterns, wherein adapted DNA comprising quality control nucleosides indicative of sub-optimal or erroneous conversion of quality control nucleosides is included in detecting the presence or absence of sequence variations and/or determining fragmentation patterns. In this way, the present methods can reduce the likelihood of false negatives and/or false positives in detecting modified nucleosides (e.g., mC or hmC) by excluding adapted DNA unsuitable for that purpose due to sub-optimal or erroneous conversion, while retaining such adapted DNA for analyses of sequence variations and/or fragmentation patterns (which are not impacted by suboptimal or erroneous conversion) and therefore avoiding impacting sensitivity.

### Analyzing and/or Partitioning DNA

**In** some instances, a heterogeneous nucleic acid sample is partitioned into two or more partitions (sub-samples). In some embodiments, each partition is differentially tagged. Tagged partitions can then be pooled together for collective sample prep and/or sequencing. The partitioning-tagging-pooling steps can occur more than once, with each round of partitioning occurring based on a different characteristics, and tagged using differential tags that are distinguished from other partitions and partitioning means.

Examples of characteristics that can be used for partitioning include sequence length, methylation level, nucleosome binding, sequence mismatch, immunoprecipitation, and/or proteins that bind to DNA. Resulting partitions can include one or more of the following nucleic acid forms: single-stranded DNA (ssDNA), double-stranded DNA (dsDNA), shorter DNA fragments and longer DNA fragments. In some embodiments, partitioning based on a cytosine modification (e.g., cytosine methylation) or methylation generally is performed and is optionally combined with at least one additional partitioning step, which may be based on any of the foregoing characteristics or forms of DNA. In some embodiments, a heterogeneous population of nucleic acids is partitioned into nucleic acids with one or more epigenetic modifications and without the one or more epigenetic modifications. Examples of epigenetic modifications include presence or absence of methylation; level of methylation; type of methylation (e.g., 5-methylcytosine versus other types of methylation, such as adenine methylation and/or cytosine hydroxymethylation); and association and level of association with one or more proteins, such as histones. Alternatively or additionally, a heterogeneous population of nucleic acids can be partitioned into nucleic acid molecules associated with nucleosomes and nucleic acid molecules devoid of nucleosomes. Alternatively or additionally, a heterogeneous population of nucleic acids may be partitioned into single-stranded DNA (ssDNA) and double-stranded DNA (dsDNA). Alternatively, or additionally, a heterogeneous population of nucleic acids may be partitioned based on nucleic acid length (e.g., molecules of up to 160 bp and molecules having a length of greater than 160 bp).

In some cases, different procedures are applied to different partitions to determine different characteristics of the initial sample. The nucleic acid, e.g. DNA of at least one partition is subjected to a conversion procedure according to the methods of the disclosure described herein. In some embodiments at least one partition is not subjected to the conversion procedure. Corresponding sequences from the converted and non-converted partitions can be compared to identify single nucleotides that have undergone conversion and therefore identify corresponding modified nucleosides in the initial sample.

For methods that involve a partitioning step, a partition tag (which distinguishes molecules in one partition from those in a different partition) may be included in the adapters or may be added to the sample molecules.

In some embodiments, two or more partitions, e.g., each partition, is/are differentially tagged. Tags can be used to label the individual polynucleotide population partitions so as to correlate the tag (or tags) with a specific partition. In some embodiments, a single tag can be used to label a specific partition. In some embodiments, multiple different tags can be used to label a specific partition. In embodiments employing multiple different tags to label a specific partition, the set of tags used to label one partition can be readily differentiated for the set of tags used to label other partitions. In some embodiments, the tags may have additional functions, for example the tags can be used to index sample sources or used as unique molecular identifiers (which can be used to improve the quality of sequencing data by differentiating sequencing errors from mutations, for example as in Kinde et al., Proc Nat'l Acad Sci USA 108: 9530-9535 (2011), Kou et al., PLoS ONE,11: e0146638 (2016)) or used as non-unique molecule identifiers, for example as described in US Pat. No. 9,598,731. Similarly, in some embodiments, the tags may have additional functions, for example the tags can be used to index sample sources or used as non-unique molecular identifiers (which can be used to improve the quality of sequencing data by differentiating sequencing errors from mutations).

In some embodiments, partition tagging comprises tagging molecules in each partition with a partition tag. After re-combining partitions (e.g., to reduce the number of sequencing runs needed and avoid unnecessary cost) and sequencing molecules, the partition tags identify the source partition. In another embodiment, different partitions are tagged with different sets of molecular tags, e.g., comprised of a pair of barcodes. In this way, each molecular barcode indicates the source partition as well as being useful to distinguish molecules within a partition. For example, a first set of 35 barcodes can be used to tag molecules in a first partition, while a second set of 35 barcodes can be used tag molecules in a second partition.

In some embodiments, after partitioning and tagging with partition tags, the molecules may be pooled for sequencing in a single run. In some embodiments, a sample tag is added to the molecules, e.g., in a step subsequent to addition of partition tags and pooling. Sample tags can facilitate pooling material generated from multiple samples for sequencing in a single sequencing run.

Alternatively, in some embodiments, partition tags may be correlated to the sample as well as the partition. As a simple example, a first tag can indicate a first partition of a first sample; a second tag can indicate a second partition of the first sample; a third tag can indicate a first partition of a second sample; and a fourth tag can indicate a second partition of the second sample.

While tags may be attached to molecules already partitioned based on one or more characteristics, the final tagged molecules in the library may no longer possess that characteristic. For example, while single stranded DNA molecules may be partitioned and tagged, the final tagged molecules in the library are likely to be double stranded. Similarly, while DNA may be subject to partition based on different levels of methylation, in the final library, tagged molecules derived from these molecules are likely to be unmethylated. Accordingly, the tag attached to a molecule in the library typically indicates the characteristic of the "parent molecule" from which the ultimate tagged molecule is derived, not necessarily to characteristic of the tagged molecule, itself.

As an example, barcodes 1, 2, 3, 4, etc. are used to tag and label molecules in the first partition; barcodes A, B, C, D, etc. are used to tag and label molecules in the second partition; and barcodes a, b, c, d, etc. are used to tag and label molecules in the third partition. Differentially tagged partitions can be pooled prior to sequencing. Differentially tagged partitions can be separately sequenced or sequenced together concurrently, e.g., in the same flow cell of an Illumina sequencer.

After sequencing, analysis of reads can be performed on a partition-by-partition level, as well as a whole DNA population level. Tags are used to sort reads from different partitions. Analysis can include in silico analysis to determine genetic and epigenetic variation (one or more of methylation, chromatin structure, etc.) using sequence information, genomic coordinates length, coverage, and/or copy number. In some embodiments, higher coverage can correlate with higher nucleosome occupancy in genomic region while lower coverage can correlate with lower nucleosome occupancy or a nucleosome depleted region (NDR).

Disclosed methods herein comprise analyzing DNA in a sample. In some embodiments described herein, the disclosed methods comprise partitioning DNA. In such methods, different forms of DNA (e.g., hypermethylated and hypomethylated DNA) can be physically partitioned based on one or more characteristics of the DNA. This approach can be used to determine, for example, whether certain sequences are hypermethylated or hypomethylated. In some embodiments, a first subsample or aliquot of a sample is subjected to steps for making capture probes as described elsewhere herein and a second subsample or aliquot of a sample is subjected to partitioning. In some embodiments, a sample or subsample or aliquot thereof is subjected to partitioning and differential tagging, followed by a capture step using capture probes for rearranged sequences and optionally additional capture probes, e.g., for sequence-variable and/or epigenetic target regions.

Methylation profiling can involve determining methylation patterns across different regions of the genome. For example, after partitioning molecules based on extent of methylation (e.g., relative number of methylated nucleobases per molecule) and sequencing, the sequences of molecules in the different partitions can be mapped to a reference genome. This can show regions of the genome that, compared with other regions, are more highly methylated or are less highly methylated. In this way, genomic regions, in contrast to individual molecules, may differ in their extent of methylation.

Partitioning nucleic acid molecules in a sample can increase a rare signal, e.g., by enriching rare nucleic acid molecules that are more prevalent in one partition of the sample. For example, a genetic variation present in hypermethylated DNA but less (or not) present in hypomethylated DNA can be more easily detected by partitioning a sample into hypermethylated and hypomethylated nucleic acid molecules. By analyzing multiple partitions of a sample, a multi-dimensional analysis of a single molecule can be performed and hence, greater sensitivity can be achieved. Partitioning may include physically partitioning nucleic acid molecules into partitions or subsamples based on the presence or absence of one or more methylated nucleobases. A sample may be partitioned into partitions or subsamples based on a characteristic that is indicative of differential gene expression or a disease state. A sample may be partitioned based on a characteristic, or combination thereof that provides a difference in signal between a normal and diseased state during analysis of nucleic acids, e.g., cell free DNA (cfDNA), non-cfDNA, tumor DNA, circulating tumor DNA (ctDNA) and cell free nucleic acids (cfNA).

In some embodiments, hypermethylation and/or hypomethylation variable epigenetic target regions are analyzed to determine whether they show differential methylation characteristic of tumor cells or cells of a type that does not normally contribute to the DNA sample being analyzed (such as cfDNA), and/or particular immune cell types.

In some instances, heterogeneous DNA in a sample is partitioned into two or more partitions (e.g., at least 3, 4, 5, 6 or 7 partitions). In some embodiments, each partition is differentially tagged. Tagged partitions can then be pooled together for collective sample prep and/or sequencing. The partitioning-tagging-pooling steps can occur more than once, with each round of partitioning occurring based on a different characteristic (examples provided herein), and tagged using differential tags that are distinguished from other partitions and partitioning means. In other instances, the differentially tagged partitions are separately sequenced.

In some embodiments, sequence reads from differentially tagged and pooled DNA are obtained and analyzed in silico. Tags are used to sort reads from different partitions. Analysis to detect genetic variants can be performed on a partition-by-partition level, as well as whole nucleic acid population level. For example, analysis can include in silico analysis to determine genetic variants, such as CNV, SNV, indel, fusion in nucleic acids in each partition. In some instances, in silico analysis can include determining chromatin structure. For example, coverage of sequence reads can be used to determine nucleosome positioning in chromatin. Higher coverage can correlate with higher nucleosome occupancy in genomic region while lower coverage can correlate with lower nucleosome occupancy or nucleosome depleted region (NDR).

Examples of characteristics that can be used for partitioning include sequence length, methylation level, nucleosome binding, sequence mismatch, immunoprecipitation, and/or proteins that bind to DNA. Resulting partitions can include one or more of the following nucleic acid forms: single-stranded DNA (ssDNA), double-stranded DNA (dsDNA), shorter DNA fragments and longer DNA fragments. In some embodiments, partitioning based on a cytosine modification (e.g., cytosine methylation) or methylation generally is performed and is optionally combined with at least one additional partitioning step, which may be based on any of the foregoing characteristics or forms of DNA. In some embodiments, a heterogeneous population of nucleic acids is partitioned into nucleic acids with one or more epigenetic modifications and without the one or more epigenetic modifications. Examples of epigenetic modifications include presence or absence of methylation; level of methylation; type of methylation (e.g., 5-methylcytosine versus other types of methylation, such as adenine methylation and/or cytosine hydroxymethylation); and association and level of association with one or more proteins, such as histones. Alternatively or additionally, a heterogeneous population of nucleic acids can be partitioned into nucleic acid molecules associated with nucleosomes and nucleic acid molecules devoid of nucleosomes. Alternatively or additionally, a heterogeneous population of nucleic acids may be partitioned into single-stranded DNA (ssDNA) and double-stranded DNA (dsDNA). Alternatively, or additionally, a heterogeneous population of nucleic acids may be partitioned based on nucleic acid length (e.g., molecules of up to 160 bp and molecules having a length of greater than 160 bp).

The agents used to partition populations of nucleic acids within a sample can be affinity agents, such as antibodies with the desired specificity, natural binding partners or variants thereof (Bock et al., Nat Biotech 28: 1106-1114 (2010); Song et al., Nat Biotech 29: 68-72 (2011)), or artificial peptides selected e.g., by phage display to have specificity to a given target. In some embodiments, the agent used in the partitioning is an agent that recognizes a modified nucleobase. In some embodiments, the modified nucleobase recognized by the agent is a modified cytosine, such as a methylcytosine (e.g., 5-methylcytosine). In some embodiments, the modified nucleobase recognized by the agent is a product of a procedure that affects the first nucleobase in the DNA differently from the second nucleobase in the DNA of the sample. In some embodiments, the modified nucleobase may be a "converted nucleobase," meaning that its base pairing specificity was changed by a procedure. For example, certain procedures convert unmethylated or unmodified cytosine to dihydrouracil, or more generally, at least one modified or unmodified form of cytosine undergoes deamination, resulting in uracil (considered a modified nucleobase in the context of DNA) or a further modified form of uracil. Examples of partitioning agents include antibodies, such as antibodies that recognize a modified nucleobase, which may be a modified cytosine, such as a methylcytosine (e.g., 5-methylcytosine). In some embodiments, the partitioning agent is an antibody that recognizes a modified cytosine other than 5-methylcytosine, such as 5-carboxylcytosine (5caC). Alternative partitioning agents include methyl binding domain (MBDs) and methyl binding proteins (MBPs) as described herein, including proteins such as MeCP2.

Additional, non-limiting examples of partitioning agents are histone binding proteins which can separate nucleic acids bound to histones from free or unbound nucleic acids. Examples of histone binding proteins that can be used in the methods disclosed herein include RBBP4, RbAp48 and SANT domain peptides.

In some embodiments, partitioning can comprise both binary partitioning and partitioning based on degree/level of modifications. For example, methylated fragments can be partitioned by methylated DNA immunoprecipitation (MeDIP), or all methylated fragments can be partitioned from unmethylated fragments using methyl binding domain proteins (e.g., MethylMinder Methylated DNA Enrichment Kit (ThermoFisher Scientific). Subsequently, additional partitioning may involve eluting fragments having different levels of methylation by adjusting the salt concentration in a solution with the methyl binding domain and bound fragments. As salt concentration increases, fragments having greater methylation levels are eluted.

Analyzing DNA may comprise detecting or quantifying DNA of interest. Analyzing DNA can comprise detecting genetic variants and/or epigenetic features (e.g., DNA methylation and/or DNA fragmentation).

In some embodiments, methylation levels can be determined using partitioning, modification-sensitive conversion such as bisulfite conversion, direct detection during sequencing, methylation-sensitive restriction enzyme digestion, methylation-dependent restriction enzyme digestion, or any other suitable approach. For example, different forms of DNA (e.g., hypermethylated and hypomethylated DNA) can be physically partitioned based on one or more characteristics of the DNA. For example, a methylated DNA binding protein (e.g., an MBD such as MBD2, MBD4, or MeCP2) or an antibody specific for 5-methylcytosine (as in MeDIP) can be used to partition the DNA. This approach can be used to determine, for example, whether certain sequences are hypermethylated or hypomethylated. In some embodiments, DNA fragmentation pattern can be determined based on endpoints and/or centerpoints of DNA molecules, such as cfDNA molecules.

In some instances, the final partitions are enriched in nucleic acids having different extents of modifications (overrepresentative or underrepresentative of modifications). Overrepresentation and underrepresentation can be defined by the number of modifications born by a nucleic acid relative to the median number of modifications per strand in a population. For example, if the median number of 5-methylcytosine residues in nucleic acid in a sample is 2, a nucleic acid including more than two 5-methylcytosine residues is overrepresented in this modification and a nucleic acid with 1 or zero 5-methylcytosine residues is underrepresented. The effect of the affinity separation is to enrich for nucleic acids overrepresented in a modification in a bound phase and for nucleic acids underrepresented in a modification in an unbound phase (i.e. in solution). The nucleic acids in the bound phase can be eluted before subsequent processing.

When using MeDIP or MethylMiner^{®}Methylated DNA Enrichment Kit (ThermoFisher Scientific) various levels of methylation can be partitioned using sequential elutions. For example, a hypomethylated partition (no methylation) can be separated from a methylated partition by contacting the nucleic acid population with the MBD from the kit, which is attached to magnetic beads. The beads are used to separate out the methylated nucleic acids from the non-methylated nucleic acids. Subsequently, one or more elution steps are performed sequentially to elute nucleic acids having different levels of methylation. For example, a first set of methylated nucleic acids can be eluted at a salt concentration of 160 mM or higher, e.g., at least 150 mM, at least 200 mM, 300 mM, 400 mM, 500 mM, 600 mM, 700 mM, 800 mM, 900 mM, 1000 mM, or 2000 mM. After such methylated nucleic acids are eluted, magnetic separation is once again used to separate higher level of methylated nucleic acids from those with lower level of methylation. The elution and magnetic separation steps can be repeated to create various partitions such as a hypomethylated partition (enriched in nucleic acids comprising no methylation), a methylated partition (enriched in nucleic acids comprising low levels of methylation), and a hyper methylated partition (enriched in nucleic acids comprising high levels of methylation).

In some methods, nucleic acids bound to an agent used for affinity separation based partitioning are subjected to a wash step. The wash step washes off nucleic acids weakly bound to the affinity agent. Such nucleic acids can be enriched in nucleic acids having the modification to an extent close to the mean or median (i.e., intermediate between nucleic acids remaining bound to the solid phase and nucleic acids not binding to the solid phase on initial contacting of the sample with the agent).

The affinity separation results in at least two, and sometimes three or more partitions of nucleic acids with different extents of a modification. While the partitions are still separate, the nucleic acids of at least one partition, and usually two or three (or more) partitions are linked to nucleic acid tags, usually provided as components of adapters, with the nucleic acids in different partitions receiving different tags that distinguish members of one partition from another. The tags linked to nucleic acid molecules of the same partition can be the same or different from one another. But if different from one another, the tags may have part of their code in common so as to identify the molecules to which they are attached as being of a particular partition.

For further details regarding portioning nucleic acid samples based on characteristics such as methylation, see WO2018/119452.

In some embodiments, the partitioning comprises contacting the DNA with a methylation sensitive restriction enzyme (MSRE) and/or a methylation dependent restriction enzyme (MDRE). Following the treatment of the DNA with a MSRE or a MDRE, the DNA may be partitioned based on size to generate hypermethylated (longest DNA molecules following MSRE treatment and shortest DNA fragments following MDRE treatment), intermediate (intermediate length DNA molecules following MSRE or MDRE treatment), and hypomethylated (shortest DNA molecules following MSRE treatment and longest DNA fragments following MDRE treatment) subsamples.

In some embodiments, the partitioning is performed by contacting the nucleic acids with a methyl binding domain ("MBD") of a methyl binding protein ("MBP"). In some such embodiments, the nucleic acids are contacted with an entire MBP. In some embodiments, an MBD binds to 5-methylcytosine (5mC), and an MBP comprises an MBD and is referred to interchangeably herein as a methyl binding protein or a methyl binding domain protein. In some embodiments, MBD is coupled to paramagnetic beads, such as Dynabeads^{®} M-280 Streptavidin via a biotin linker. Partitioning into fractions with different extents of methylation can be performed by eluting fractions by increasing the NaCl concentration.

In some embodiments, bound DNA is eluted by contacting the antibody or MBD with a protease, such as proteinase K. This may be performed instead of or in addition to elution steps using NaCl as discussed above.

Examples of agents that recognize a modified nucleobase contemplated herein include, but are not limited to:
(a) MeCP2 is a protein that preferentially binds to 5-methyl-cytosine over unmodified cytosine.
(b) RPL26, PRP8 and the DNA mismatch repair protein MHS6 preferentially bind to 5-hydroxymethyl-cytosine over unmodified cytosine.
(c) FOXK1, FOXK2, FOXP1, FOXP4 and FOXI3 preferably bind to 5-formyl-cytosine over unmodified cytosine (Iurlaro et al., Genome Biol. 14: R119 (2013)).
(d) Antibodies specific to one or more methylated or modified nucleobases or conversion products thereof, such as 5mC, 5caC, or DHU.

In general, elution is a function of the number of modifications, such as the number of methylated sites per molecule, with molecules having more methylation eluting under increased salt concentrations. To elute the DNA into distinct populations based on the extent of methylation, one can use a series of elution buffers of increasing NaCl concentration. Salt concentration can range from about 100 nm to about 2500 mM NaCl. In one embodiment, the process results in three (3) partitions. Molecules are contacted with a solution at a first salt concentration and comprising a molecule comprising an agent that recognizes a modified nucleobase, which molecule can be attached to a capture moiety, such as streptavidin. At the first salt concentration a population of molecules will bind to the agent and a population will remain unbound. The unbound population can be separated as a "hypomethylated" population. For example, a first partition enriched in hypomethylated form of DNA is that which remains unbound at a low salt concentration, e.g., 100 mM or 160 mM. A second partition enriched in intermediate methylated DNA is eluted using an intermediate salt concentration, e.g., between 100 mM and 2000 mM concentration. This is also separated from the sample. A third partition enriched in hypermethylated form of DNA is eluted using a high salt concentration, e.g., at least about 2000 mM.

In some embodiments, a monoclonal antibody raised against 5-methylcytidine (5mC) is used to purify methylated DNA. DNA is denatured, e.g., at 95°C in order to yield single-stranded DNA fragments. Protein G coupled to standard or magnetic beads as well as washes following incubation with the anti-5mC antibody are used to immunoprecipitate DNA bound to the antibody. Such DNA may then be eluted. Partitions may comprise unprecipitated DNA and one or more partitions eluted from the beads.

In some embodiments, the partitions of DNA are desalted and concentrated in preparation for enzymatic steps of library preparation.

Sequences that comprise aberrantly high copy numbers may tend to be hypermethylated. Accordingly, in some embodiments, the DNA contacted with capture probes specific for members of an epigenetic target region set comprising a plurality of target regions that are both type-specific differentially methylated regions and copy number variants comprises at least a portion of a hypermethylated partition. The DNA from or comprising at least a portion of the hypermethylated partition may or may not be combined with DNA from or comprising at least a portion of one or more other partitions, such as an intermediate partition or a hypomethylated partition.

In some embodiments, methylation is detected using a conversion procedure. Conversion procedures include any technique that differentially alters a first nucleobase but not a second nucleobase in a modification-dependent manner, e.g., being methylated (or hydroxymethylated, or formylated, or carboxylated, etc.) versus unmodified, and/or being modified in one way versus another way (e.g., methylated versus hydroxymethylated). Examples of such conversion procedures include bisulfite conversion, which converts unmodified cytosine and certain modified cytosines (e.g. 5-formyl cytosine (fC) or 5-carboxylcytosine (caC)) to uracil whereas other modified cytosines (e.g., 5-methylcytosine, 5-hydroxylmethylcystosine) are not converted. Performing bisulfite conversion can facilitate identifying positions containing mC or hmC using the sequence reads. For an exemplary description of bisulfite conversion, see, e.g., Moss et al., Nat Commun. 2018; 9: 5068.

Examples of such conversion procedures also include oxidative bisulfite (Ox-BS) conversion. Performing Ox-BS conversion can facilitate identifying positions containing mC using the sequence reads. For an exemplary description of oxidative bisulfite conversion, see, e.g., Booth et al., Science 2012; 336: 934-937.

Examples of such conversion procedures also include Tet-assisted bisulfite (TAB) conversion. For example, as described in Yu et al., Cell 2012; 149: 1368-80, β-glucosyl transferase can be used to protect hmC (forming 5-glucosylhydroxymethylcytosine (ghmC)), then a TET protein such as mTet1 can be used to convert mC to caC, and then bisulfite treatment can be used to convert C and caC to U while ghmC remains unaffected. Thus, when TAB conversion is used, the first nucleobase comprises one or more of unmodified cytosine, fC, caC, mC, or other cytosine forms affected by bisulfite, and the second nucleobase comprises hmC. Performing TAB conversion can facilitate identifying positions containing hmC using the sequence reads.

Examples of such conversion procedures also include Tet-assisted conversion with a substituted borane reducing agent, optionally wherein the substituted borane reducing agent is 2-picoline borane, borane pyridine, tert-butylamine borane, or ammonia borane. See, e.g., Liu et al., Nature Biotechnology 2019; 37:424-429 (e.g., at Supplementary Fig. 1 and Supplementary Note 7). Performing TAP conversion can facilitate identifying positions containing unmodified C using the sequence reads. This procedure encompasses Tet-assisted pyridine borane sequencing (TAPS), described in further detail in Liu et al. 2019, supra.

Alternatively, protection of hmC (e.g., using βGT) can be combined with Tet-assisted conversion with a substituted borane reducing agent. Performing such TAPSβ conversion can facilitate distinguishing positions containing unmodified C or hmC on the one hand from positions containing mC using the sequence reads. For an exemplary description of this type of conversion, see, e.g., Liu et al., Nature Biotechnology 2019; 37:424-429.

Examples of such conversion procedures also include APOBEC-coupled epigenetic (ACE) conversion. Performing ACE conversion can facilitate distinguishing positions containing hmC from positions containing mC or unmodified C using the sequence reads. For an exemplary description of ACE conversion, see, e.g., Schutsky et al., Nature Biotechnology 2018; 36: 1083-1090.

Examples of such conversion procedures also include enzymatic conversion of a nucleobase, e.g., as in EM-Seq. See, e.g., Vaisvila R, et al. (2019) EM-seq: Detection of DNA methylation at single base resolution from picograms of DNA. bioRxiv; DOI: 10.1101/2019.12.20.884692, available at www.biorxiv.org/content/10.1101/2019.12.20.884692v1.

In some embodiments, methylation is detected using a methylation-sensitive restriction enzyme (MSRE). For example, a sample, subsample, or portion of a sample can be subjected to digestion with one or more MSREs to cleave unmethylated sequences. Exemplary MSREs include AatII, AccII, AciI, Aor13HI, Aor15HI, BspT104I, BssHII, BstUI, Cfr10I, ClaI, CpoI, Eco52I, HaeII, HapII, HhaI, Hin6I, HpaII, HpyCH4IV, MluI, MspI, NaeI, NotI, NruI, NsbI, PmaCI, Psp1406I, PvuI, SacII, SalI, SmaI, and SnaBI. In some embodiments, at least two methylation-sensitive nucleases are used. In some embodiments, at least three methylation-sensitive nucleases are used. In some embodiments, the methylation-sensitive nucleases comprise BstUI and HpaII. In some embodiments, the two methylation-sensitive nucleases comprise HhaI and AccII. In some embodiments, the methylation-sensitive nucleases comprise BstUI, HpaII and Hin6I. In some embodiments, the portion of the sample that is contacted with one or more MSREs comprises hypermethylated DNA, or is or comprises a hypermethylated DNA partition, which may be obtained as described elsewhere herein.

In some embodiments, DNA fragmentation is detected by determining the endpoints and/or midpoints of sequenced fragments of DNA (e.g., cfDNA). For example, differences in fragmentation patterns may occur depending on whether the fragments originated from a tumor or from healthy cells. To detect tumor-cell derived DNA of cfDNA based on fragmentation, the presence or absence of an increased level of abnormal fragments can be determined at regions with copy-number amplifications, (e.g., proportional to the degree of amplification), e.g., where the increase and abnormality are relative to control or healthy samples.

In some embodiments, a sample or subsample (e.g., a first, second, or third subsample prepared by partitioning a sample as described herein, such as on the basis of a level of a cytosine modification, such as methylation, e.g., 5-methylation, such as of cytosine) is contacted with a methylation-dependent nuclease or methylation-sensitive nuclease. Unless otherwise indicated, where partitioning is performed on the basis of a cytosine modification, the first subsample is the subsample with a higher level of the modification; the second subsample is the subsample with a lower level of the modification; and, when present, the third subsample has a level of the modification intermediate between the first and second subsamples.

As discussed above, partitioning procedures may result in imperfect sorting of DNA molecules among the subsamples. The choice of a methylation-dependent nuclease or methylation-sensitive nuclease can be made so as to degrade nonspecifically partitioned DNA. For example, the second subsample can be contacted with a methylation-dependent nuclease, such as a methylation-dependent restriction enzyme. This can degrade nonspecifically partitioned DNA in the second subsample (e.g., methylated DNA) to produce a treated second subsample. Alternatively or in addition, the first subsample can be contacted with a methylation-sensitive endonuclease, such as a methylation-sensitive restriction enzyme, thereby degrading nonspecifically partitioned DNA in the first subsample to produce a treated first subsample. Degradation of nonspecifically partitioned DNA in either or both of the first or second subsamples is proposed as an improvement to the performance of methods that rely on accurate partitioning of DNA on the basis of a cytosine modification, e.g., to detect the presence of aberrantly modified DNA in a sample, to determine the tissue of origin of DNA, and/or to determine whether a subject has cancer. For example, such degradation may provide improved sensitivity and/or simplify downstream analyses. In general, where nonspecifically partitioned DNA would be hypermethylated, such as in a hypomethylated partition, a methylation-dependent nuclease, such as a methylation-dependent restriction enzyme, should be used. Conversely, where nonspecifically partitioned DNA would be hypomethylated, such as in a hypermethylated partition, a methylation-sensitive nuclease, such as a methylation-sensitive restriction enzyme, should be used. Methylation-dependent nucleases, such as methylation-dependent restriction enzymes, preferentially cut methylated DNA relative to unmethylated DNA, while methylation-sensitive nucleases, such as methylation-sensitive restriction enzymes, preferentially cut unmethylated DNA relative to methylated DNA.

In contacting a subsample with a nuclease, one or more nucleases can be used. In some embodiments, a subsample is contacted with a plurality of nucleases. The subsample may be contacted with the nucleases sequentially or simultaneously. Simultaneous use of nucleases may be advantageous when the nucleases are active under similar conditions (e.g., buffer composition) to avoid unnecessary sample manipulation. Contacting the second subsample with more than one methylation-dependent restriction enzyme can more completely degrade nonspecifically partitioned hypermethylated DNA. Similarly, contacting the first subsample with more than one methylation-sensitive restriction enzyme can more completely degrade nonspecifically partitioned hypomethylated and/or unmethylated DNA.

In some embodiments, a methylation-dependent nuclease comprises one or more of MspJI, LpnPI, FspEI, or McrBC. In some embodiments, at least two methylation-dependent nucleases are used. In some embodiments, at least three methylation-dependent nucleases are used. In some embodiments, the methylation-dependent nuclease comprises FspEI. In some embodiments, the methylation-dependent nuclease comprises FspEI and MspJI, e.g., used sequentially.

In some embodiments, a methylation-sensitive nuclease comprises one or more of AatII, AccII, AciI, Aor13HI, Aor15HI, BspT1041, BssHII, BstUI, Cfr10I, ClaI, CpoI, Eco52I, HaeII, HapII, HhaI, Hin6I, HpaII, HpyCH4IV, MluI, MspI, NaeI, NotI, NruI, NsbI, PmaCI, Psp1406I, PvuI, SacII, SalI, SmaI, and SnaBI. In some embodiments, at least two methylation-sensitive nucleases are used. In some embodiments, at least three methylation-sensitive nucleases are used. In some embodiments, the methylation-sensitive nucleases comprise BstUI and HpaII. In some embodiments, the two methylation-sensitive nucleases comprise HhaI and AccII. In some embodiments, the methylation-sensitive nucleases comprise BstUI, HpaII and Hin6I.

In some embodiments, FspEI is used for digesting the nucleic acid molecules in at least one subsample (e.g., a hypomethylated partition). In some embodiments, BstUI, HpaII and Hin6I are used for digesting the nucleic acid molecules in at least one subsample (e.g., a hypermethylated partition) and FspEI is used for digesting the nucleic acid molecules in at least one other subsample (e.g., a hypomethylated partition). In embodiments involving an intermediately methylated partition, the nucleic acid molecules therein may be digested with a methylation-sensitive nuclease or a methylation-dependent nuclease. In some embodiments, the nucleic acid molecules in an intermediately methylated partition are digested with the same nuclease(s) as the hypermethylated partition. For example, the intermediately methylated partition may be pooled with the hypermethylated partition and then the pooled partitions may be subjected to digestion. In some embodiments, the nucleic acid molecules in an intermediately methylated partition are digested with the same nuclease(s) as the hypomethylated partition. For example, the intermediately methylated partition may be pooled with the hypomethylated partition and then the pooled partitions may be subjected to digestion.

In some embodiments, a subsample is contacted with a nuclease as described above after a step of tagging or attaching adapters to both ends of the DNA. The tags or adapters can be resistant to cleavage by the nuclease using any of the approaches described above. In this approach, cleavage can prevent the nonspecifically partitioned molecule from being carried through the analysis because the cleavage products lack tags or adapters at both ends.

Alternatively, a step of tagging or attaching adapters can be performed after cleavage with a nuclease as described above. Cleaved molecules can be then identified in sequence reads based on having an end (point of attachment to tag or adapter) corresponding to a nuclease recognition site. Processing the molecules in this way can also allow the acquisition of information from the cleaved molecule, e.g., observation of somatic mutations. When tagging or attaching adapters after contacting the subsample with a nuclease, and low molecular weight DNA such as cfDNA is being analyzed, it may be desirable to remove high molecular weight DNA (such as contaminating genomic DNA) from the sample before the contacting step. It may also be desirable to use nucleases that can be heat-inactivated at a relatively low temperature (e.g., 65°C or less, or 60°C or less) to avoid denaturing DNA, in that denaturation may interfere with subsequent ligation steps.

Where a sample is partitioned into three subsamples, including a third subsample containing intermediately methylated molecules, the third subsample is in some embodiments contacted with a methylation-sensitive nuclease. Such a step may have any of the features described elsewhere herein with respect to contacting steps, and may be performed before or after a step of tagging or attaching adapters as discussed above. In some embodiments, the first and third subsamples are combined before being contacted with a methylation-sensitive nuclease. Such a step may have any of the features described elsewhere herein with respect to contacting steps, and may be performed before or after a step of tagging or attaching adapters as discussed above. In some embodiments, the first and third subsamples are differentially tagged before being combined.

Alternatively, where a sample is partitioned into three subsamples, including a third subsample containing intermediately methylated molecules, the third subsample is in some embodiments contacted with a methylation-dependent nuclease. Such a step may have any of the features described elsewhere herein with respect to contacting steps, and may be performed before or after a step of tagging or attaching adapters as discussed above. In some embodiments, the second and third subsamples are combined before being contacted with a methylation-dependent nuclease. Such a step may have any of the features described elsewhere herein with respect to contacting steps, and may be performed before or after a step of tagging or attaching adapters as discussed above. In some embodiments, the second and third subsamples are differentially tagged before being combined.

In some embodiments, the DNA is purified after being contacted with the nuclease, e.g., using SPRI beads. Such purification may occur after heat inactivation of the nuclease. Alternatively, purification can be omitted; thus, for example, a subsequent step such as amplification can be performed on the subsample containing heat-inactivated nuclease. In another embodiment, the contacting step can occur in the presence of a purification reagent such as SPRI beads, e.g., to minimize losses associated with tube transfers. After cleavage and heat inactivation, the SPRI beads can be re-used for cleanup by adding molecular crowding reagents (e.g., PEG) and salt.

In some embodiments, where a conversion procedure is performed on a sample or subsample, the subsequent capturing of one or more target region sets (e.g., at least an epigenetic target region set) from that sample or subsample uses capture probes that comprise probes specific for a modification state (e.g., of at least one base in the sequence to which the probe hybridizes), e.g., complementary to target sequences that have undergone conversion (e.g., conversion of modified or unmodified cytosines to uracils or analogs thereof, such as DHU, that preferentially pair with adenine) or that have not undergone conversion, as desired. As such, the probes can be specific for sequences in which a modification of interest, such as methylation, was or was not present. In some embodiments, where a modification sensitive conversion is performed on a sample or subsample, the subsequent capturing of one or more target region sets (e.g., at least an epigenetic target region set) from that sample or subsample uses capture probes that comprise probes that can hybridize to target sequences regardless of modification state (e.g., comprise a promiscuously pairing nucleobase at a position that may or may not have undergone conversion of modified or unmodified cytosines to uracils or analogs thereof, such as DHU, that preferentially pair with adenine; for example, inosine can pair with C or U).

In some embodiments, the methods comprise preparing a pool comprising at least a portion of the DNA of the second subsample (also referred to as the hypomethylated partition) and at least a portion of the DNA of the first subsample (also referred to as the hypermethylated partition). Target regions, e.g., including epigenetic target regions and/or sequence-variable target regions, may be captured from the pool. The steps of capturing a target region set from at least a portion of a subsample described elsewhere herein encompass capture steps performed on a pool comprising DNA from the first and second subsamples. A step of amplifying DNA in the pool may be performed before capturing target regions from the pool. The capturing step may have any of the features described elsewhere herein.

The epigenetic target regions may show differences in methylation levels and/or fragmentation patterns depending on whether they originated from a tumor or from healthy cells, or what type of tissue they originated from, as discussed elsewhere herein. The sequence-variable target regions may show differences in sequence depending on whether they originated from a tumor or from healthy cells.

Analysis of epigenetic target regions from the hypomethylated partition may be less informative in some applications than analysis of sequence-variable target-regions from the hypermethylated and hypomethylated partitions and epigenetic target regions from the hypermethylated partition. As such, in methods where sequence-variable target-regions and epigenetic target regions are being captured, the latter may be captured to a lesser extent than one or more of the sequence-variable target-regions from the hypermethylated and hypomethylated partitions and epigenetic target regions from the hypermethylated partition. For example, sequence-variable target regions can be captured from the portion of the hypomethylated partition not pooled with the hypermethylated partition, and the pool can be prepared with some (e.g., a majority, substantially all, or all) of the DNA from the hypermethylated partition and none or some (e.g., a minority) of the DNA from the hypomethylated partition. Such approaches can reduce or eliminate sequencing of epigenetic target regions from the hypomethylated partition, thereby reducing the amount of sequencing data that suffices for further analysis.

In some embodiments, including a minority of the DNA of the hypomethylated partition in the pool facilitates quantification of one or more epigenetic features (e.g., methylation or other epigenetic feature(s) discussed in detail elsewhere herein), e.g., on a relative basis.

In some embodiments, the pool comprises a minority of the DNA of the hypomethylated partition, e.g., less than about 50% of the DNA of the hypomethylated partition, such as less than or equal to about 45%, 40%, 35%, 30%, 25%, 20%, 15%, 10%, or 5% of the DNA of the hypomethylated partition. In some embodiments, the pool comprises about 5%-25% of the DNA of the hypomethylated partition. In some embodiments, the pool comprises about 10%-20% of the DNA of the hypomethylated partition. In some embodiments, the pool comprises about 10% of the DNA of the hypomethylated partition. In some embodiments, the pool comprises about 15% of the DNA of the hypomethylated partition. In some embodiments, the pool comprises about 20% of the DNA of the hypomethylated partition.

In some embodiments, the pool comprises a portion of the hypermethylated partition, which may be at least about 50% of the DNA of the hypermethylated partition. For example, the pool may comprise at least about 55%, 60%, 65%, 70%, 75%, 80%, 85%, 90%, or 95% of the DNA of the hypermethylated partition. In some embodiments, the pool comprises 50-55%, 55-60%, 60-65%, 65-70%, 70-75%, 75-80%, 80-85%, 85-90%, 90-95%, or 95-100% of the DNA of the hypermethylated partition. In some embodiments, the second pool comprises all or substantially all of the hypermethylated partition.

In some embodiments, the methods comprise preparing a first pool comprising at least a portion of the DNA of the hypomethylated partition. In some embodiments, the methods comprise preparing a second pool comprising at least a portion of the DNA of the hypermethylated partition. In some embodiments, the first pool further comprises a portion of the DNA of the hypermethylated partition. In some embodiments, the second pool further comprises a portion of the DNA of the hypomethylated partition. In some embodiments, the first pool comprises a majority of the DNA of the hypomethylated partition, and optionally and a minority of the DNA of the hypermethylated partition. In some embodiments, the second pool comprises a majority of the DNA of the hypermethylated partition and a minority of the DNA of the hypomethylated partition. In some embodiments involving an intermediately methylated partition, the second pool comprises at least a portion of the DNA of the intermediately methylated partition, e.g., a majority of the DNA of the intermediately methylated partition. In some embodiments, the first pool comprises a majority of the DNA of the hypomethylated partition, and the second pool comprises a majority of the DNA of the hypermethylated partition and a majority of the DNA of the intermediately methylated partition.

In some embodiments, the methods comprise capturing at least a first set of target regions from the first pool, e.g., wherein the first pool is as set forth in any of the embodiments above. In some embodiments, the first set comprises sequence-variable target regions. In some embodiments, the first set comprises hypomethylation variable target regions and/or fragmentation variable target regions. In some embodiments, the first set comprises sequence-variable target regions and fragmentation variable target regions. In some embodiments, the first set comprises sequence-variable target regions, hypomethylation variable target regions and fragmentation variable target regions. A step of amplifying DNA in the first pool may be performed before this capture step. In some embodiments, capturing the first set of target regions from the first pool comprises contacting the DNA of the first pool with a first set of capture probes. In some embodiments, the first set of capture probes comprises target-binding probes specific for the sequence-variable target regions. In some embodiments, the first set of capture probes comprises target-binding probes specific for the sequence-variable target regions, hypomethylation variable target regions and/or fragmentation variable target regions.

In some embodiments, the methods comprise capturing a second set of target regions or plurality of sets of target regions from the second pool, e.g., wherein the first pool is as set forth in any of the embodiments above. In some embodiments, the second plurality comprises epigenetic target regions, such as hypermethylation variable target regions and/or fragmentation variable target regions. In some embodiments, the second plurality comprises sequence-variable target regions and epigenetic target regions, such as hypermethylation variable target regions and/or fragmentation variable target regions. A step of amplifying DNA in the second pool may be performed before this capture step. In some embodiments, capturing the second plurality of sets of target regions from the second pool comprises contacting the DNA of the first pool with a second set of capture probes, wherein the second set of capture probes comprises target-binding probes specific for the sequence-variable target regions and target-binding probes specific for the epigenetic target regions. In some embodiments, the first set of target regions and the second set of target regions are not identical. For example, the first set of target regions may comprise one or more target regions not present in the second set of target regions. Alternatively or in addition, the second set of target regions may comprise one or more target regions not present in the first set of target regions. In some embodiments, at least one hypermethylation variable target region is captured from the second pool but not from the first pool. In some embodiments, a plurality of hypermethylation variable target regions are captured from the second pool but not from the first pool. In some embodiments, the first set of target regions comprises sequence-variable target regions and/or the second set of target regions comprises epigenetic target regions. In some embodiments, the first set of target regions comprises sequence-variable target regions, and fragmentation variable target regions; and the second set of target regions comprises epigenetic target regions, such as hypermethylation variable target regions and fragmentation variable target regions. In some embodiments, the first set of target regions comprises sequence-variable target regions, fragmentation variable target regions, and comprises hypomethylation variable target regions; and the second set of target regions comprises epigenetic target regions, such as hypermethylation variable target regions and fragmentation variable target regions.

In some embodiments, the first pool comprises a majority of the DNA of the hypomethylated partition and a portion of the DNA of the hypermethylated partition (e.g., about half), and the second pool comprises a portion of the DNA of the hypermethylated partition (e.g., about half). In some such embodiments, the first set of target regions comprises sequence-variable target regions and/or the second set of target regions comprises epigenetic target regions. The sequence-variable target regions and/or the epigenetic target regions may be as set forth in any of the embodiments described elsewhere herein.

In some embodiments, the partitions of DNA are desalted and concentrated in preparation for enzymatic steps of library preparation. Sequences that comprise structural variations may tend to be hypomethylated. Accordingly, in some embodiments, the DNA contacted with a plurality of primers comprising at least two primers that anneal in an anti-parallel orientation to a rearranged sequence of DNA is from or comprises at least a portion of a hypomethylated partition. The DNA from or comprising at least a portion of hypomethylated partition may or may not be combined with DNA from or comprising at least a portion of one or more other partitions, such as an intermediate partition or a hypermethylated partition.

### Amplification

Sample nucleic acids flanked by adapters can be amplified by PCR and other amplification methods. Amplification is typically primed by primers binding to primer binding sites in adapters flanking a DNA molecule to be amplified. Amplification methods can involve cycles of denaturation, annealing and extension, resulting from thermocycling or can be isothermal as in transcription-mediated amplification. Other amplification methods include the ligase chain reaction, strand displacement amplification, nucleic acid sequence-based amplification, and self-sustained sequence based replication.

In some embodiments, the present methods perform dsDNA ligations with T-tailed and C-tailed adapters, which result in amplification of at least 50, 60, 70 or 80% of double stranded nucleic acids. Preferably the present methods increase the amount or number of amplified molecules relative to control methods performed with T-tailed adapters alone by at least 10, 15 or 20%.

In some embodiments, sample nucleic acids are amplified before sequencing. Amplification may be before and/or after the conversion step. Amplification may in some cases be before one or more capture steps. In some embodiments, the ligating occurs before or simultaneously with amplification.

In some embodiments, amplification is primed by primer binding to primer binding site(s) in the adapter oligonucleotide(s). The known modified nucleosides of the adapter(s) may be outside of the primer binding site(s), i.e., the known modified nucleosides used for the quality control method are not in the primer-binding sites or bound by the amplification primers.

### Enriching, Capturing and Using Capture Probes; Target Regions

Nucleic acids in a sample can be subject to a capture step, in which molecules having target sequences are captured for subsequent analysis. Capture may be performed using any suitable approach known in the art. Target capture can involve use of a bait set comprising oligonucleotide baits labeled with a capture moiety, such as biotin or the other examples noted below. The probes can have sequences selected to tile across a panel of regions, such as genes. Such bait sets are combined with a sample under conditions that allow hybridization of the target molecules with the baits. Then, captured molecules are isolated using the capture moiety. For example, a biotin capture moiety by bead-based streptavidin. Such methods are further described in, for example, U.S. patent 9,850,523, issuing December 26, 2017.

Capture moieties include, without limitation, biotin, avidin, streptavidin, a nucleic acid comprising a particular nucleotide sequence, a hapten recognized by an antibody, and magnetically attractable particles. The extraction moiety can be a member of a binding pair, such as biotin/streptavidin or hapten/antibody. In some embodiments, a capture moiety that is attached to an analyte is captured by its binding pair which is attached to an isolatable moiety, such as a magnetically attractable particle or a large particle that can be sedimented through centrifugation. The capture moiety can be any type of molecule that allows affinity separation of nucleic acids bearing the capture moiety from nucleic acids lacking the capture moiety. Exemplary capture moieties are biotin which allows affinity separation by binding to streptavidin linked or linkable to a solid phase or an oligonucleotide, which allows affinity separation through binding to a complementary oligonucleotide linked or linkable to a solid phase.

In some embodiments, DNA is captured that comprises a region comprising a type-specific epigenetic variation. In some embodiments, the variations are present in healthy cells but not normally present in the sample type, such as a blood sample. In some embodiments, the variations are present in aberrant cells (e.g., hyperplastic, metaplastic, or neoplastic cells).

In some embodiments, a first captured epigenetic target region set captured from a sample or first subsample comprises hypermethylation variable target regions. In some embodiments, the hypermethylation variable target regions show type-specific hypermethylation in healthy cfDNA from one or more related cell or tissue types. Without wishing to be bound by any particular theory, the presence of cancer cells may increase the shedding of DNA into the bloodstream (e.g., from the cancer and/or the surrounding tissue). As such, the distribution of tissue of origin of cfDNA may change upon carcinogenesis. Thus, an increase in the level of hypermethylation variable target regions in the first subsample can be an indicator of the presence (or recurrence, depending on the history of the subject) of cancer.

In some embodiments, the methods herein comprise capturing a second captured epigenetic target region set from a sample or second subsample. In some embodiments, the second epigenetic target region set comprises hypomethylation variable target regions. Without wishing to be bound by any particular theory, cancer cells may shed more DNA into the bloodstream than healthy cells of the same tissue type. As such, the distribution of tissue of origin of cfDNA may change upon carcinogenesis. Thus, an increase in the level of hypomethylation variable target regions in the second subsample can be an indicator of the presence (or recurrence, depending on the history of the subject) of cancer.

Additionally, captured target region sets may comprise DNA corresponding to a sequence-variable target region set. The captured sets may be combined to provide a combined captured set.

In some embodiments in which a captured set comprising DNA corresponding to a sequence-variable target region set and the epigenetic target region set includes a combined captured set as discussed above, the DNA corresponding to the sequence-variable target region set may be present at a greater concentration than the DNA corresponding to the epigenetic target region set, e.g., a 1.1 to 1.2-fold greater concentration, a 1.2- to 1.4-fold greater concentration, a 1.4- to 1.6-fold greater concentration, a 1.6- to 1.8-fold greater concentration, a 1.8- to 2.0-fold greater concentration, a 2.0- to 2.2-fold greater concentration, a 2.2- to 2.4-fold greater concentration a 2.4- to 2.6-fold greater concentration, a 2.6- to 2.8-fold greater concentration, a 2.8- to 3.0-fold greater concentration, a 3.0- to 3.5-fold greater concentration, a 3.5- to 4.0, a 4.0- to 4.5-fold greater concentration, a 4.5- to 5.0-fold greater concentration, a 5.0-to 5.5-fold greater concentration, a 5.5- to 6.0-fold greater concentration, a 6.0- to 6.5-fold greater concentration, a 6.5- to 7.0-fold greater, a 7.0- to 7.5-fold greater concentration, a 7.5- to 8.0-fold greater concentration, an 8.0- to 8.5-fold greater concentration, an 8.5- to 9.0-fold greater concentration, a 9.0- to 9.5-fold greater concentration, 9.5- to 10.0-fold greater concentration, a 10- to 11-fold greater concentration, an 11- to 12-fold greater concentration a 12- to 13-fold greater concentration, a 13- to 14-fold greater concentration, a 14- to 15-fold greater concentration, a 15- to 16-fold greater concentration, a 16- to 17-fold greater concentration, a 17- to 18-fold greater concentration, an 18- to 19-fold greater concentration, a 19- to 20-fold greater concentration, a 20- to 30-fold greater concentration, a 30- to 40-fold greater concentration, a 40- to 50-fold greater concentration, a 50- to 60-fold greater concentration, a 60- to 70-fold greater concentration, a 70- to 80-fold greater concentration, a 80-to 90-fold greater concentration, or a 90- to 100-fold greater concentration. The degree of difference in concentrations accounts for normalization for the footprint sizes of the target regions, as discussed in the definition section.

In some embodiments, the DNA that is captured comprises intronic regions. In some embodiments, the intronic regions comprise one or more introns likely to differentiate DNA from neoplastic (e.g., tumor or cancer) cells and from healthy cells, e.g., non-neoplastic circulating cells. For example, an intron comprising a rearrangement known to be present in some neoplastic cells and absent from healthy cells can be used to differentiate DNA from neoplastic (e.g., tumor or cancer) cells and from healthy cells. In some embodiments, the rearrangement is a translocation.

In some embodiments, captured intronic regions have a footprint of at least 30 bp, e.g., at least 100 bp, at least 200 bp, at least 500 bp, at least 1 kb, at least 2 kb, at least 5 kb, at least 10 kb, at least 20 kb, at least 50 kb, at least 200 kb, at least 300 kb, or at least 400 kb. In some embodiments, the intronic target region set has a footprint in the range of 30 bp-1000 kb, e.g., 30 bp-100 bp, 100 bp-200 bp, 200 bp-500 bp, 500 bp-1kb, 1 kb-2 kb, 2 kb-5 kb, 5 kb-10 kb, 10 kb-20 kb, 20 kb-50 kb, 50 kb-100 kb, 100-200 kb, 200-300 kb, 300-400 kb, 400-500 kb, 500-600 kb, 600-700 kb, 700-800 kb, 800-900 kb, and 900-1,000 kb.

Exemplary rearrangements, such as intronic translocations that can be detected using the methods described herein include but are not limited to translocations wherein at least one of the two genes involved in the translocation is a receptor tyrosine kinase. Exemplary translocation products are the BCR-ABL fusion. and fusions comprising any of ALK, FGFR2, FGFR3, NTRK1, RET, or ROS1.

In some embodiments, the DNA that is captured comprises target regions having a type-specific epigenetic variation. In some embodiments, an epigenetic target region set consists of target regions having a type-specific epigenetic variation. In some embodiments, the type-specific epigenetic variations, e.g., differential methylation or a type-specific fragmentation pattern, are likely to differentiate DNA from one or more related cell or tissue types cells from DNA from other cell or tissue types present in a sample or in a subject.

In some embodiments, nucleic acids captured or enriched using a method described herein comprise captured DNA, such as one or more captured sets of DNA. In some embodiments, the captured DNA comprise target regions that are differentially methylated in different immune cell types. In some embodiments, the immune cell types comprise rare or closely related immune cell types, such as activated and naive lymphocytes or myeloid cells at different stages of differentiation.

In some embodiments, a captured epigenetic target region set captured from a sample or first subsample comprises hypermethylation variable target regions. In some embodiments, the hypermethylation variable target regions are differentially or exclusively hypermethylated in one or more related cell or tissue types. In some embodiments, the hypermethylation variable target regions are differentially or exclusively hypermethylated in one cell type or in one immune cell type, or in one immune cell type within a cluster. In some embodiments, the hypermethylation variable target regions are hypermethylated to an extent that is distinguishably higher or exclusively present in one cell type or one immune cell type or one immune cell type within a cluster. Such hypermethylation variable target regions may be hypermethylated in other cell or tissue types but not to the extent observed in the one or more related cell or tissue types. In some embodiments, the hypermethylation variable target regions show lower methylation in healthy cfDNA than in at least one other tissue type. In some embodiments, the hypermethylation variable target regions show even higher methylation in cfDNA from a diseased cell of the one or more related cell or tissue types. In some embodiments, target regions comprise hypermethylated regions with aberrantly high copy number. In some such embodiments, the target regions are hypermethylated in healthy and diseased colon tissue and have aberrantly high copy number in pre-cancerous or cancerous colon tissue. Examples of such target regions are shown in Table 1 below.

**Table 1: Hypermethylated target regions with aberrantly high copy number in colon cancer or pre-cancer**

| Chromosomal region with copy number gain | Genes comprising DMRs within the chromosomal region |
|---|---|
| 7p21.3-15.1 | VWDE, TWIST1, DNAH1, GPNMB, NPY, GSDME, NFE2L3, HOXA1, HOXA7, EVX1, CREB5 |
| 8q13.3-24.3 | PRDM14, MSC, TRPA1, SBSPON, CRISPLD1, ZFHX4, RALYL, MMP16, C8orf88, RUNX1T1, CDH17, GDF6, SDC2, OSR2, PABPC1, ZNF706, RIMS2, ZFPM2, SYBU, CSMD3, TRPS1, AARD, TNFRSF11B, MAL2, COL14A1, POUSF1B, ADCY8, ST3GAL1, TRAPPC9, AGO2, ADGRB1, LY6E, LY6H, GLI4, MAFA, TSTA3, PLEC, OPLAH, FOXH1 |
| 13q14.1-33.3 | ELF1, RGCC, TNFSF11, SERP2, RB1, CNMD, PCDH17, EDNRB, POU4F1, SLITRKS, GPC6, SOX21, CLDN10, STK24, ZIC2, NALCN, FGF14, MYO16, COL4A1, COL4A2, SOX1, F7 |
| 20q12-13.33 | MAFB, LPIN3, EMILIN3, PTPRT, TOX2, GDAP1L1, HNF4A, MMP9, CDH22, PREX1, ZNFX1, PARD6B, ZFP64, DOK5, FAM210B, TFAP2C, RBM38, CTCFL, GNAS, PHACTR3, CDH4, GATA5, BHLHE23, YTHDF1, NKAIN4, CHRNA4, KCNQ2, GMEB2 |

**Table 2. Exemplary Hypermethylation Target Regions based on Lung Cancer studies**

| **Gene Name** | **Chromosome** |
|---|---|
| MARCH11 | chr5 |
| TAC1 | chr7 |
| TCF21 | chr6 |
| SHOX2 | chr3 |
| p16 | chr3 |
| Casp8 | chr2 |
| CDH13 | chr16 |
| MGMT | chr10 |
| MLH1 | chr3 |
| MSH2 | chr2 |
| TSLC1 | chr11 |
| APC | chr5 |
| DKK1 | chr10 |
| DKK3 | chr11 |
| LKB1 | chr11 |
| WIF1 | chr12 |
| RUNX3 | chr1 |
| GATA4 | chr8 |
| GATA5 | chr20 |
| PAX5 | chr9 |
| E-Cadherin | chr16 |
| H-Cadherin | chr16 |

In some embodiments, a captured epigenetic target region set captured from a sample or subsample comprises hypomethylation variable target regions. In some embodiments, the hypomethylation variable target regions are exclusively hypomethylated in one or more related cell or tissue types. In some embodiments, the hypomethylation variable target regions are exclusively hypomethylated in one cell type or in one immune cell type or in one immune cell type within a cluster. In some embodiments, the hypomethylation variable target regions are hypomethylated to an extent that is exclusively present in one cell type or one immune cell type or in one immune cell type within a cluster. Such hypomethylation variable target regions may be hypomethylated in other cell or tissue types but not to the extent observed in the one or more cell or tissue types. In some embodiments, the hypomethylation variable target regions show higher methylation in healthy cfDNA than in at least one other tissue type.

Without wishing to be bound by any particular theory, in an individual with cancer, proliferating or activated immune cells and/or dying cancer cells may shed more DNA into the bloodstream than immune cells in a healthy individual and/or healthy cells of the same tissue type, respectively. As such, the distribution of cell type and/or tissue of origin of cfDNA may change upon carcinogenesis. Thus, the presence and/or levels of cfDNA originating from certain cell or tissue types can be an indicator of disease. Variations in hypermethylation and/or hypomethylation can be an indicator of disease. For example, an increase in the level of hypermethylation variable target regions and/or hypomethylation variable target regions in a subsample following a partitioning step can be an indicator of the presence (or recurrence, depending on the history of the subject) of cancer.

Exemplary hypermethylation variable target regions and hypomethylation variable target regions useful for distinguishing between various cell types, including but not limited to immune cell types, have been identified by analyzing DNA obtained from various cell types via whole genome bisulfite sequencing, as described, e.g., in Scott, C.A., Duryea, J.D., MacKay, H. et al., "Identification of cell type-specific methylation signals in bulk whole genome bisulfite sequencing data," Genome Biol 21, 156 (2020) (doi.org/10.1186/s13059-020-02065-5). Whole-genome bisulfite sequencing data is available from the Blueprint consortium, available on the internet at dcc.blueprint-epigenome.eu.

In some embodiments, first and second captured target region sets comprise, respectively, DNA corresponding to a sequence-variable target region set and DNA corresponding to an epigenetic target region set, for example, as described in WO 2020/160414. The first and second captured sets may be combined to provide a combined captured set. The sequence-variable target region set and epigenetic target region set may have any of the features described for such sets in WO 2020/160414. In some embodiments, the epigenetic target region set comprises a hypermethylation variable target region set. In some embodiments, the epigenetic target region set comprises a hypomethylation variable target region set. In some embodiments, the epigenetic target region set comprises CTCF binding regions. In some embodiments, the epigenetic target region set comprises fragmentation variable target regions. In some embodiments, the epigenetic target region set comprises transcriptional start sites. In some embodiments, the epigenetic target region set comprises regions that may show focal amplifications in cancer, e.g., one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the epigenetic target region set comprises at least 2, 3, 4, 5, 6, 7, 8, 9, 10, 11, 12, 13, 14, 15, 16, 17, or 18 of the foregoing targets.

In some embodiments, the sequence-variable target region set comprises a plurality of regions known to undergo somatic mutations in cancer. In some aspects, the sequence-variable target region set targets a plurality of different genes or genomic regions ("panel") selected such that a determined proportion of subjects having a cancer exhibits a genetic variant or tumor marker in one or more different genes or genomic regions in the panel. The panel may be selected to limit a region for sequencing to a fixed number of base pairs. The panel may be selected to sequence a desired amount of DNA, e.g., by adjusting the affinity and/or amount of the probes as described elsewhere herein. The panel may be further selected to achieve a desired sequence read depth. The panel may be selected to achieve a desired sequence read depth or sequence read coverage for an amount of sequenced base pairs. The panel may be selected to achieve a theoretical sensitivity, a theoretical specificity, and/or a theoretical accuracy for detecting one or more genetic variants in a sample.

Probes for detecting the panel of regions can include those for detecting genomic regions of interest (hotspot regions). Information about chromatin structure can be taken into account in designing probes, and/or probes can be designed to maximize the likelihood that particular sites (e.g., KRAS codons 12 and 13) can be captured, and may be designed to optimize capture based on analysis of cfDNA coverage and fragment size variation impacted by nucleosome binding patterns and GC sequence composition. Regions used herein can also include non-hotspot regions optimized based on nucleosome positions and GC models.

Probes for detecting the panel of regions can include those for detecting genomic regions of interest (hotspot regions). Information about chromatin structure can be taken into account in designing probes, and/or probes can be designed to maximize the likelihood that particular sites (e.g., KRAS codons 12 and 13) can be captured, and may be designed to optimize capture based on analysis of cfDNA coverage and fragment size variation impacted by nucleosome binding patterns and GC sequence composition. Regions used herein can also include non-hotspot regions optimized based on nucleosome positions and GC models.

Examples of listings of genomic locations of interest may be found in Table 3 and Table 4 of WO 2020/160414. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, or 70 of the genes of Table 3 of WO 2020/160414. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, at least 70, or 73 of the genes of Table 4 of WO 2020/160414. Additionally or alternatively, suitable target region sets are available from the literature. For example, Gale et al., PLoS One 13: e0194630 (2018) describes a panel of 35 cancer-related gene targets that can be used as part or all of a sequence-variable target region set. These 35 targets are AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

In some embodiments, the sequence-variable target region set comprises target regions from at least 10, 20, 30, or 35 cancer-related genes, such as the cancer-related genes listed above and in WO 2020/160414.

In some embodiments, a collection of capture probes is used in methods described herein, e.g., comprising capture probes prepared by any method disclosed herein for doing so. In some embodiments, the collection of capture probes further comprises target-binding probes specific for a sequence-variable target region set and/or target-binding probes specific for an epigenetic target region set. In some embodiments, the capture yield of the target-binding probes specific for the sequence-variable target region set is higher (e.g., at least 2-fold higher) than the capture yield of the target-binding probes specific for the epigenetic target region set. In some embodiments, the collection of capture probes is configured to have a capture yield specific for the sequence-variable target region set higher (e.g., at least 2-fold higher) than its capture yield specific for the epigenetic target region set.

In some embodiments, the capture yield of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the capture yield of the target-binding probes specific for the epigenetic target region set. In some embodiments, the capture yield of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than the capture yield of the target-binding probes specific for the epigenetic target region set.

In some embodiments, the collection of capture probes is configured to have a capture yield specific for the sequence-variable target region set at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than its capture yield for the epigenetic target region set. In some embodiments, the collection of capture probes is configured to have a capture yield specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than its capture yield specific for the epigenetic target region set.

The collection of probes can be configured to provide higher capture yields for the sequence-variable target region set in various ways, including concentration, different lengths and/or chemistries (e.g., that affect affinity), and combinations thereof. Affinity can be modulated by adjusting probe length and/or including nucleotide modifications as discussed below.

In some embodiments, the capture probes specific for the sequence-variable target region set are present at a higher concentration than the capture probes specific for the epigenetic target region set. In some embodiments, concentration of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the concentration of the target-binding probes specific for the epigenetic target region set. In some embodiments, the concentration of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10-to 11-, 11- to 12-, 13- to 14-, or 14- to 15-fold higher than the concentration of the target-binding probes specific for the epigenetic target region set. In such embodiments, concentration may refer to the average mass per volume concentration of individual probes in each set.

In some embodiments, the capture probes specific for the sequence-variable target region set have a higher affinity for their targets than the capture probes specific for the epigenetic target region set. Affinity can be modulated in any way known to those skilled in the art, including by using different probe chemistries. For example, certain nucleotide modifications, such as cytosine 5-methylation (in certain sequence contexts), modifications that provide a heteroatom at the 2' sugar position, and LNA nucleotides, can increase stability of double-stranded nucleic acids, indicating that oligonucleotides with such modifications have relatively higher affinity for their complementary sequences. See, e.g., Severin et al., Nucleic Acids Res. 39: 8740-8751 (2011); Freier et al., Nucleic Acids Res. 25: 4429-4443 (1997); US Patent No. 9,738,894. Also, longer sequence lengths will generally provide increased affinity. Other nucleotide modifications, such as the substitution of the nucleobase hypoxanthine for guanine, reduce affinity by reducing the amount of hydrogen bonding between the oligonucleotide and its complementary sequence. In some embodiments, the capture probes specific for the sequence-variable target region set have modifications that increase their affinity for their targets. In some embodiments, alternatively or additionally, the capture probes specific for the epigenetic target region set have modifications that decrease their affinity for their targets. In some embodiments, the capture probes specific for the sequence-variable target region set have longer average lengths and/or higher average melting temperatures than the capture probes specific for the epigenetic target region set. These embodiments may be combined with each other and/or with differences in concentration as discussed above to achieve a desired fold difference in capture yield, such as any fold difference or range thereof described above.

In some embodiments, the capture probes comprise a capture moiety. The capture moiety may be any of the capture moieties described herein, e.g., biotin. In some embodiments, the capture probes are linked to a solid support, e.g., covalently or non-covalently such as through the interaction of a binding pair of capture moieties. In some embodiments, the solid support is a bead, such as a magnetic bead.

In some embodiments, the capture probes specific for the sequence-variable target region set and/or the capture probes specific for the epigenetic target region set are a capture probe set as discussed above, e.g., probes comprising capture moieties and sequences selected to tile across a panel of regions, such as genes.

In some embodiments, the capture probes are provided in a single composition. The single composition may be a solution (liquid or frozen). Alternatively, it may be a lyophilizate.

Alternatively, the capture probes may be provided as a plurality of compositions, e.g., comprising a first composition comprising probes specific for the epigenetic target region set and a second composition comprising probes specific for the sequence-variable target region set. These probes may be mixed in appropriate proportions to provide a combined probe composition with any of the foregoing fold differences in concentration and/or capture yield. Alternatively, they may be used in separate capture procedures (e.g., with aliquots of a sample or sequentially with the same sample) to provide first and second compositions comprising captured epigenetic target regions and sequence-variable target regions, respectively.

### Probes specific for epigenetic target regions

The probes for the epigenetic target region set may comprise probes specific for one or more types of target regions likely to differentiate DNA from neoplastic (e.g., tumor or cancer) cells from healthy cells, e.g., non-neoplastic circulating cells. Exemplary types of such regions are discussed in detail herein, e.g., in the sections above concerning captured sets. The probes for the epigenetic target region set may also comprise probes for one or more control regions, e.g., as described herein.

In some embodiments, the probes for the epigenetic target region set have a footprint of at least 100 kbp, e.g., at least 200 kbp, at least 300 kbp, or at least 400 kbp. In some embodiments, the epigenetic target region set has a footprint in the range of 100-20 Mbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp, 1.5-2 Mbp, 2-3 Mbp, 3-4 Mbp, 4-5 Mbp, 5-6 Mbp, 6-7 Mbp, 7-8 Mbp, 8-9 Mbp, 9-10 Mbp, or 10-20 Mbp. In some embodiments, the epigenetic target region set has a footprint of at least 20 Mbp.

### Hypermethylation variable target regions

In some embodiments, the probes for the epigenetic target region set comprise probes specific for one or more hypermethylation variable target regions. Hypermethylation variable target regions may also be referred to herein as hypermethylated DMRs (differentially methylated regions). The hypermethylation variable target regions may be any of those set forth above. For example, in some embodiments, the probes specific for hypermethylation variable target regions comprise probes specific for a plurality of loci listed in Table 1, e.g., at least 10%, 20%, 30%, 40%, 50%, 60%, 70%, 80%, 90%, or 100% of the loci listed in Table 1. In some embodiments, the probes specific for hypermethylation variable target regions comprise probes specific for a plurality of loci listed in Table 2, e.g., at least 10%, 20%, 30%, 40%, 50%, 60%, 70%, 80%, 90%, or 100% of the loci listed in Table 2. In some embodiments, the probes specific for hypermethylation variable target regions comprise probes specific for a plurality of loci listed in Table 1 or Table 2, e.g., at least 10%, 20%, 30%, 40%, 50%, 60%, 70%, 80%, 90%, or 100% of the loci listed in Table 1 or Table 2. In some embodiments, for each locus included as a target region, there may be one or more probes with a hybridization site that binds between the transcription start site and the stop codon (the last stop codon for genes that are alternatively spliced) of the gene. In some embodiments, the one or more probes bind within 300 bp of the listed position, e.g., within 200 or 100 bp. In some embodiments, a probe has a hybridization site overlapping the position listed above. In some embodiments, the probes specific for the hypermethylation target regions include probes specific for one, two, three, four, or five subsets of hypermethylation target regions that collectively show hypermethylation in one, two, three, four, or five of breast, colon, kidney, liver, and lung cancers.

### Hypomethylation variable target regions

In some embodiments, the probes for the epigenetic target region set comprise probes specific for one or more hypomethylation variable target regions. Hypomethylation variable target regions may also be referred to herein as hypomethylated DMRs (differentially methylated regions). The hypomethylation variable target regions may be any of those set forth above. For example, the probes specific for one or more hypomethylation variable target regions may include probes for regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells.

In some embodiments, probes specific for hypomethylation variable target regions include probes specific for repeated elements and/or intergenic regions. In some embodiments, probes specific for repeated elements include probes specific for one, two, three, four, or five of LINE1 elements, Alu elements, centromeric tandem repeats, pericentromeric tandem repeats, and/or satellite DNA.

Exemplary probes specific for genomic regions that show cancer-associated hypomethylation include probes specific for nucleotides 8403565-8953708 and/or 151104701-151106035 of human chromosome 1. In some embodiments, the probes specific for hypomethylation variable target regions include probes specific for regions overlapping or comprising nucleotides 8403565-8953708 and/or 151104701-151106035 of human chromosome 1.

### CTCF binding regions

In some embodiments, the probes for the epigenetic target region set include probes specific for CTCF binding regions. In some embodiments, the probes specific for CTCF binding regions comprise probes specific for at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above. In some embodiments, the probes for the epigenetic target region set comprise at least 100 bp, at least 200 bp at least 300 bp, at least 400 bp, at least 500 bp, at least 750 bp, or at least 1000 bp upstream and downstream regions of the CTCF binding sites.

### Transcription start sites

In some embodiments, the probes for the epigenetic target region set include probes specific for transcriptional start sites. In some embodiments, the probes specific for transcriptional start sites comprise probes specific for at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, the probes for the epigenetic target region set comprise probes for sequences at least 100 bp, at least 200 bp, at least 300 bp, at least 400 bp, at least 500 bp, at least 750 bp, or at least 1000 bp upstream and downstream of the transcriptional start sites.

### Focal amplifications

As noted above, although focal amplifications are somatic mutations, they can be detected by sequencing based on read frequency in a manner analogous to approaches for detecting certain epigenetic changes such as changes in methylation. As such, regions that may show focal amplifications in cancer can be included in the epigenetic target region set, as discussed above. In some embodiments, the probes specific for the epigenetic target region set include probes specific for focal amplifications. In some embodiments, the probes specific for focal amplifications include probes specific for one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the probes specific for focal amplifications include probes specific for one or more of at least 2, 3, 4, 5, 6, 7, 8, 9, 10, 11, 12, 13, 14, 15, 16, 17, or 18 of the foregoing targets.

### Control regions

It can be useful to include control regions to facilitate data validation. In some embodiments, the probes specific for the epigenetic target region set include probes specific for control methylated regions that are expected to be methylated in essentially all samples. In some embodiments, the probes specific for the epigenetic target region set include probes specific for control hypomethylated regions that are expected to be hypomethylated in essentially all samples.

### Probes specific for sequence-variable target regions

The probes for the sequence-variable target region set may comprise probes specific for a plurality of regions known to undergo somatic mutations in cancer. The probes may be specific for any sequence-variable target region set described herein. Exemplary sequence-variable target region sets are discussed in detail herein, e.g., in the sections above concerning captured sets.

In some embodiments, the sequence-variable target region probe set has a footprint of at least 0.5 kb, e.g., at least 1 kb, at least 2 kb, at least 5 kb, at least 10 kb, at least 20 kb, at least 30 kb, or at least 40 kb. In some embodiments, the epigenetic target region probe set has a footprint in the range of 0.5-100 kb, e.g., 0.5-2 kb, 2-10 kb, 10-20 kb, 20-30 kb, 30-40 kb, 40-50 kb, 50-60 kb, 60-70 kb, 70-80 kb, 80-90 kb, and 90-100 kb. In some embodiments, the sequence-variable target region probe set has a footprint of at least 50 kbp, e.g., at least 100 kbp, at least 200 kbp, at least 300 kbp, or at least 400 kbp. In some embodiments, the sequence-variable target region probe set has a footprint in the range of 100-2000 kbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp or 1.5-2 Mbp. In some embodiments, the sequence-variable target region set has a footprint of at least 2 Mbp.

In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, or at 70 of the genes of Table 3. In some embodiments, probes specific for the sequence-variable target region set comprise probes specific for the at least 5, at least 10, at least 15, at least 20, at least 25, at least 30, at least 35, at least 40, at least 45, at least 50, at least 55, at least 60, at least 65, or 70 of the SNVs of Table 3. In some embodiments, probes specific for the sequence-variable target region set comprise probes specific for at least 1, at least 2, at least 3, at least 4, at least 5, or 6 of the fusions of Table 3. In some embodiments, probes specific for the sequence-variable target region set comprise probes specific for at least a portion of at least 1, at least 2, or 3 of the indels of Table 3. In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, probes specific for the sequence-variable target region set comprise probes specific for at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, probes specific for the sequence-variable target region set comprise probes specific for at least 1, at least 2, at least 3, at least 4, at least 5, or 6 of the fusions of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5.

**Table 3**

| **Point Mutations (SNVs)** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | CDKN2B | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K 1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | SRC | STK11 | |
| TERT | TP53 | TSC1 | VHL | | | |

**Table 4**

| **Point Mutations (SNVs)** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | DDR2 | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K 1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | MAPK1 | STK11 | |
| TERT | TP53 | TSC1 | VHL | MAPK3 | MTOR | |
| NTRK3 | | | | | | |

**Table 5**

| **Gene** | **Chromosome** | **Start Position** | **Stop Position** | **Length (bp)** | **Exons Covered** | **Critical Feature** |
|---|---|---|---|---|---|---|
| ALK | chr2 | 29446405 | 29446655 | 250 | intron 19 | Fusion |
| ALK | chr2 | 29446062 | 29446197 | 135 | intron 20 | Fusion |
| ALK | chr2 | 29446198 | 29446404 | 206 | 20 | Fusion |
| ALK | chr2 | 29447353 | 29447473 | 120 | intron 19 | Fusion |
| ALK | chr2 | 29447614 | 29448316 | 702 | intron 19 | Fusion |
| ALK | chr2 | 29448317 | 29448441 | 124 | 19 | Fusion |
| ALK | chr2 | 29449366 | 29449777 | 411 | intron 18 | Fusion |
| ALK | chr2 | 29449778 | 29449950 | 172 | 18 | Fusion |
| BRAF | chr7 | 140453064 | 140453203 | 139 | 15 | BRAF V600 |
| CTNNB1 | chr3 | 41266007 | 41266254 | 247 | 3 | S37 |
| EGFR | chr7 | 55240528 | 55240827 | 299 | 18 and 19 | G719 and deletions |
| EGFR | chr7 | 55241603 | 55241746 | 143 | 20 | Insertions/T790M |
| EGFR | chr7 | 55242404 | 55242523 | 119 | 21 | L858R |
| ERBB2 | chr17 | 37880952 | 37881174 | 222 | 20 | Insertions |
| ESR1 | chr6 | 152419857 | 152420111 | 254 | 10 | V534, P535, L536, Y537, D538 |
| FGFR2 | chr10 | 123279482 | 123279693 | 211 | 6 | S252 |
| GATA3 | chr10 | 8111426 | 8111571 | 145 | 5 | SS / Indels |
| GATA3 | chr10 | 8115692 | 8116002 | 310 | 6 | SS / Indels |
| GNAS | chr20 | 57484395 | 57484488 | 93 | 8 | R844 |
| IDH1 | chr2 | 209113083 | 209113394 | 311 | 4 | R132 |
| IDH2 | chr15 | 90631809 | 90631989 | 180 | 4 | R140, R172 |
| KIT | chr4 | 55524171 | 55524258 | 87 | 1 | |
| KIT | chr4 | 55561667 | 55561957 | 290 | 2 | |
| KIT | chr4 | 55564439 | 55564741 | 302 | 3 | |
| KIT | chr4 | 55565785 | 55565942 | 157 | 4 | |
| KIT | chr4 | 55569879 | 55570068 | 189 | 5 | |
| KIT | chr4 | 55573253 | 55573463 | 210 | 6 | |
| KIT | chr4 | 55575579 | 55575719 | 140 | 7 | |
| KIT | chr4 | 55589739 | 55589874 | 135 | 8 | |
| KIT | chr4 | 55592012 | 55592226 | 214 | 9 | |
| KIT | chr4 | 55593373 | 55593718 | 345 | 10 and 11 | 557, 559, 560, 576 |
| KIT | chr4 | 55593978 | 55594297 | 319 | 12 and 13 | V654 |
| KIT | chr4 | 55595490 | 55595661 | 171 | 14 | T670, S709 |
| KIT | chr4 | 55597483 | 55597595 | 112 | 15 | D716 |
| KIT | chr4 | 55598026 | 55598174 | 148 | 16 | L783 |
| KIT | chr4 | 55599225 | 55599368 | 143 | 17 | C809, R815, D816, L818, D820, S821F, N822, Y823 |
| KIT | chr4 | 55602653 | 55602785 | 132 | 18 | A829P |
| KIT | chr4 | 55602876 | 55602996 | 120 | 19 | |
| KIT | chr4 | 55603330 | 55603456 | 126 | 20 | |
| KIT | chr4 | 55604584 | 55604733 | 149 | 21 | |
| KRAS | chr12 | 25378537 | 25378717 | 180 | 4 | A146 |
| KRAS | chr12 | 25380157 | 25380356 | 199 | 3 | Q61 |
| KRAS | chr12 | 25398197 | 25398328 | 131 | 2 | G12/G13 |
| MET | chr7 | 116411535 | 116412255 | 720 | 13, 14, intron 13, intron 14 | MET exon 14 SS |
| NRAS | chr1 | 115256410 | 115256609 | 199 | 3 | Q61 |
| NRAS | chr1 | 115258660 | 115258791 | 131 | 2 | G12/G13 |
| PIK3CA | chr3 | 178935987 | 178936132 | 145 | 10 | E545K |
| PIK3CA | chr3 | 178951871 | 178952162 | 291 | 21 | H1047R |
| PTEN | chr10 | 89692759 | 89693018 | 259 | 5 | R130 |
| SMAD4 | chr18 | 48604616 | 48604849 | 233 | 12 | D537 |
| TERT | chr5 | 1294841 | 1295512 | 671 | promoter | chr5:1295228 |
| TP53 | chr17 | 7573916 | 7574043 | 127 | 11 | Q331, R337, R342 |
| TP53 | chr17 | 7577008 | 7577165 | 157 | 8 | R273 |
| TP53 | chr17 | 7577488 | 7577618 | 130 | 7 | R248 |
| TP53 | chr17 | 7578127 | 7578299 | 172 | 6 | R213/Y220 |
| TP53 | chr17 | 7578360 | 7578564 | 204 | 5 | R175 / Deletions |
| TP53 | chr17 | 7579301 | 7579600 | 299 | 4 | |
| | | | | 12574 (total target region) | | |
| | | | | 16330 (total probe coverage) | | |

In some embodiments, the probes specific for the sequence-variable target region set comprise probes specific for target regions from at least 10, 20, 30, or 35 cancer-related genes, such as AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

### Sequencing

In general, sample nucleic acids flanked by adapters with or without prior amplification can be subject to sequencing. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, Digital Gene Expression (Helicos), Next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, and sequencing using PacBio, SOLiD, Ion Torrent, or Nanopore platforms. Sequencing reactions can be performed in a variety of sample processing units, which may include multiple lanes, multiple channels, multiple wells, or other mean of processing multiple sample sets substantially simultaneously. Sample processing unit can also include multiple sample chambers to enable processing of multiple runs simultaneously.

In some embodiments, sequence coverage of the genome may be, for example, less than 5%, 10%, 15%, 20%, 25%, 30%, 40%, 50%, 60%, 70%, 80%, 90%, 95%, 99%, 99.9% or 100%. In some embodiments, the sequence reactions may provide for sequence coverage of, for example, at least 5%, 10%, 15%, 20%, 25%, 30%, 40%, 50%, 60%, 70%, or 80% of the genome. Sequence coverage can performed on, for example, at least 5, 10, 20, 70, 100, 200 or 500 different genes, or up to, for example, 5000, 2500, 1000, 500 or 100 different genes.

Simultaneous sequencing reactions may be performed using multiplex sequencing. In some cases, cell-free nucleic acids may be sequenced with at least, for example, 1000, 2000, 3000, 4000, 5000, 6000, 7000, 8000, 9000, 10000, 50000, or 100,000 sequencing reactions. In other cases, cell-free nucleic acids may be sequenced with less than, for example, 1000, 2000, 3000, 4000, 5000, 6000, 7000, 8000, 9000, 10000, 50000, or 100,000 sequencing reactions. Sequencing reactions may be performed sequentially or simultaneously. Subsequent data analysis may be performed on all or part of the sequencing reactions. In some cases, data analysis may be performed on at least, for example, 1000, 2000, 3000, 4000, 5000, 6000, 7000, 8000, 9000, 10000, 50000, or 100,000 sequencing reactions. In other cases, data analysis may be performed on less than, for example, 1000, 2000, 3000, 4000, 5000, 6000, 7000, 8000, 9000, 10000, 50000, or 100,000 sequencing reactions. An exemplary read depth is 1000-50000 or 1000-10000 or 1000-20000 reads per locus (base).

In general, sequencing of epigenetic target regions, e.g., to analyze a modified nucleoside profile of DNA, requires a lesser depth of sequencing than sequencing of a sequence-variable target region, e.g. for analysis of mutations. Hence, lesser sequencing depths, as described herein, may in some cases be adequate for the methods described herein.

### Analysis

### Detecting false negatives with conversion procedures which convert modified nucleosides

In these embodiments, the adapter sequences used in the methods of the disclosure include quality control nucleosides which have a known modification. When the modified quality control nucleosides are used in conjunction with a conversion procedure that changes the base pairing specificity of modified nucleosides, it is expected to change the base pairing specificity of the quality control nucleosides as a result of the conversion procedure applied. Hence, whether or not the conversion procedure has been effective for a particular known modified nucleoside is determined simply by reading how the modified nucleotides have been sequenced. For example, if an adapter molecule comprising mC in the quality control nucleosides has been subjected to a TAPS procedure and is read when sequenced as T, then conversion was effective. If a known mC in the quality control nucleosides is read as C, then the conversion was ineffective. Hence, it is possible to identify (individual/single) adapted DNA molecules with sub-optimal conversion of quality conversion nucleosides in the adapters. If the adapter (or adapters, if one is included at each end) includes multiple known modified quality control nucleosides, then some of the modified quality control nucleosides may have been correctly converted and others not. Therefore, it is possible to determine the conversion rate for a particular molecule equal to the number of correctly converted known modified quality control nucleosides divided by the total number of known modified nucleosides in the quality control nucleosides of the molecule adapters. Likewise, the conversion rate for the whole sample (or partition/subsample thereof) may be determined by dividing the total number of correctly converted known modified quality control nucleosides in the uniquely identified molecules of the sample by the total number of known modified nucleosides in the same set of uniquely identified sample molecules. In other words, the conversion rate for the sample or molecule, including the nucleosides with unknown modification status, is inferred from the conversion rate of the known modified nucleosides in the adapter sequences.

The conversion rate can be used as a quality control measure of the conversion rate at the sample level or the molecular level. In some cases, individual molecules or samples that have sub-optimal conversion rates, e.g., a conversion rate that is below a pre-determined threshold, may be excluded from further analysis. In other cases, the conversion rate may be partial or sub-optimal, but can still be used for analysis, taking into account the known incomplete conversion of modified nucleosides in the molecule or sample. For example, a score or weighting may be applied to each molecule and/or the sample that is dependent on the determined conversion rate(s). Molecules or samples with higher conversion rates are assigned a higher weighting, i.e., are attributed a greater significance, in downstream analysis or determination of the modified nucleoside profile of the sample DNA. Molecules or samples with lower relative conversion rates are assigned a lower weighting, i.e., are attributed a lesser significance in downstream analysis. For example, the DNA may be from a subject suspected of having cancer. The analysis comprises determining the modified nucleoside profile of multiple epigenetic target regions where an altered modified nucleoside profile has been associated with cancer. The method of the disclosure may determine different conversion rates for the DNA molecules from each of the target regions. The diagnostic value of the modified nucleoside profiles determined by sequencing molecules within the regions having lower conversion rates is decreased compared to the diagnostic value of the modified nucleoside profiles determined by sequencing molecules within the regions having higher conversion rates. Hence, the lower conversion rate regions are assigned a lesser weighting when the modified nucleoside profile analysis from all of the regions is combined, typically also with other indicators such as the detection of cancer-associated sequence mutations, to provide the overall diagnostic determination, e.g., as a summation of risk scores.

### Detecting false negatives with conversion procedures which convert unmodified nucleosides

When the modified quality control nucleosides are used in conjunction with a conversion procedure that changes the base pairing specificity of unmodified nucleosides, it is typically not expected to change the base pairing specificity of the quality control nucleosides as a result of the conversion procedure applied. Hence, whether or not the conversion procedure has been effective for a particular known modified nucleoside is determined simply by reading how the modified nucleotides have been sequenced. For example, if an adapter molecule comprising mC in the quality control nucleosides has been subjected to a bisulfite procedure and is read when sequenced as C, then conversion procedure did not erroneously convert the mC and the known mC will be correctly identified as methylated. If the known mC in the quality control nucleosides is read as T, then the conversion procedure erroneously converted the mC and the known mC will be incorrectly identified as unmethylated (i.e., a false negative). Hence, it is possible to identify (individual/single) adapted DNA molecules with erroneous conversion of quality conversion nucleosides in the adapters. If the adapter (or adapters, if one is included at each end) includes multiple known modified nucleosides, then some of the modified nucleosides may have been erroneously converted and others not. Therefore, it is possible to determine the erroneous conversion rate for a particular molecule equal to the number of erroneously converted known modified nucleosides divided by the total number of known modified nucleosides in the quality control nucleosides of the molecule adapters. Likewise, the erroneous conversion rate for the whole sample (or partition/subsample thereof) may be determined by dividing the total number of erroneously converted known modified nucleosides in the uniquely identified molecules of the sample by the total number of known modified nucleosides in the same set of uniquely identified sample molecules. In other words, the erroneous conversion rate for the whole sample or molecule, including the nucleosides with unknown modification status, is inferred from the erroneous conversion rate of the known modified nucleosides in the adapter sequences.

The erroneous conversion rate can be used as a quality control measure of the erroneous conversion rate at the sample level or the molecular level. In some cases, individual molecules or samples that have high erroneous conversion rates, e.g., a conversion rate that is above a pre-determined threshold, may be excluded from further analysis. In other cases, the erroneous conversion rate may be above a pre-determined threshold, but can still be used for analysis, taking into account the known erroneous conversion of modified nucleosides in the molecule or sample. For example, a score or weighting may be applied to each molecule and/or the sample that is dependent on the determined erroneous conversion rate(s). Molecules or samples with higher erroneous conversion rates are assigned a lower weighting, i.e., are attributed a lower significance, in downstream analysis or determination of the modified nucleoside profile of the sample DNA. Molecules or samples with lower relative erroneous conversion rate are assigned a higher weighting, i.e., are attributed a higher significance in downstream analysis. For example, the DNA may be from a subject suspected of having cancer. The analysis comprises determining the modified nucleoside profile of multiple epigenetic target regions where an altered modified nucleoside profile has been associated with cancer. The method of the disclosure may determine different erroneous conversion rates for the DNA molecules from each of the target regions. The diagnostic value of the modified nucleoside profiles determined by sequencing molecules within the regions having higher erroneous conversion rates is decreased compared to the diagnostic value of the modified nucleoside profiles determined by sequencing molecules within the regions having lower erroneous conversion rates. Hence, the higher erroneous conversion rate regions are assigned a lesser weighting when the modified nucleoside profile analysis from all of the regions is combined, typically also with other indicators such as the detection of cancer-associated sequence mutations, to provide the overall diagnostic determination, e.g., as a summation of risk scores.

### Detecting false positives with conversion procedures which convert modified nucleosides

When the unmodified quality control nucleosides are used in conjunction with a conversion procedure that changes the base pairing specificity of modified nucleosides, it is not expected to change the base pairing specificity of the quality control nucleosides as a result of the conversion procedure applied. Hence, whether or not the conversion procedure has been effective for a particular known unmodified nucleoside is determined simply by reading how the unmodified nucleotides have been sequenced. For example, if an adapter molecule comprising an unmodified C in the quality control nucleosides has been subjected to a TAPS procedure and is read when sequenced as C, then conversion procedure did not erroneously convert the unmodified C and the known unmodified C will be correctly identified as unmethylated. If the known unmodified C in the quality control nucleosides is read as T, then the conversion procedure erroneously converted the unmodified C and the known unmodified C will be incorrectly identified as methylated (i.e. a false positive). Hence, it is possible to identify (individual/single) adapted DNA molecules with erroneous conversion of quality conversion nucleosides in the adapters. If the adapter (or adapters, if one is included at each end) includes multiple known unmodified nucleosides, then some of the unmodified nucleosides may have been erroneously converted and others not. Therefore, it is possible to determine the erroneous conversion rate for a particular molecule equal to the number of erroneously converted known unmodified nucleosides divided by the total number of known unmodified nucleosides in the quality control nucleosides of the molecule adapters. Likewise, the erroneous conversion rate for the whole sample (or partition/subsample thereof) may be determined by dividing the total number of erroneously converted known unmodified nucleosides in the uniquely identified molecules of the sample by the total number of known unmodified nucleosides in the same set of uniquely identified sample molecules. In other words, the erroneous conversion rate for the whole sample or molecule, including the nucleosides with unknown modification status, is inferred from the erroneous conversion rate of the known unmodified nucleosides in the adapter sequences.

The erroneous conversion rate can be used as a quality control measure of the erroneous conversion rate at the sample level or the molecular level. In some cases, individual molecules or samples that have high erroneous conversion rates, e.g. a conversion rate that is above a pre-determined threshold, may be excluded from further analysis. In other cases, the erroneous conversion rate may be above a pre-determined threshold, but can still be used for analysis, taking into account the known erroneous conversion of unmodified nucleosides in the molecule or sample. For example, a score or weighting may be applied to each molecule and/or the sample that is dependent on the determined erroneous conversion rate(s). Molecules or samples with higher erroneous conversion rates are assigned a lower weighting, i.e. are attributed a lower significance, in downstream analysis or determination of the modified nucleoside profile of the sample DNA. Molecules or samples with lower relative erroneous conversion rate are assigned a higher weighting, i.e. are attributed a higher significance in downstream analysis. For example, the DNA may be from a subject suspected of having cancer. The analysis comprises determining the modified nucleoside profile of multiple epigenetic target regions where an altered modified nucleoside profile has been associated with cancer. The method of the disclosure may determine different erroneous conversion rates for the DNA molecules from each of the target regions. The diagnostic value of the modified nucleoside profiles determined by sequencing molecules within the regions having higher erroneous conversion rates is decreased compared to the diagnostic value of the modified nucleoside profiles determined by sequencing molecules within the regions having lower erroneous conversion rates. Hence, the higher erroneous conversion rate regions are assigned a lesser weighting when the modified nucleoside profile analysis from all of the regions is combined, typically also with other indicators such as the detection of cancer-associated sequence mutations, to provide the overall diagnostic determination, e.g. as a summation of risk scores.

### Detecting false positives with conversion procedures which convert unmodified nucleosides

When the unmodified quality control nucleosides are used in conjunction with a conversion procedure that changes the base pairing specificity of unmodified nucleosides, it is expected to change the base pairing specificity of the quality control nucleosides as a result of the conversion procedure applied. Hence, whether or not the conversion procedure has been effective for a particular known unmodified nucleoside is determined simply by reading how the unmodified nucleotides have been sequenced. For example, if an adapter molecule comprising an unmodified C in the quality control nucleosides has been subjected to a bisulfite procedure and is read when sequenced as T, then conversion was effective. If the known unmodified C in the quality control nucleosides is read as C, then the conversion was ineffective. In such a case, this would normally lead to the unmodified C incorrectly being identified as modified (i.e. a false positive). Hence, it is possible to identify (individual/single) adapted DNA molecules with sub-optimal conversion of quality conversion nucleosides in the adapters. If the adapter (or adapters, if one is included at each end) includes multiple known unmodified nucleosides, then some of the unmodified nucleosides may have been correctly converted and others not. Therefore, it is possible to determine the conversion rate for a particular molecule equal to the number of correctly converted known unmodified nucleosides divided by the total number of known unmodified nucleosides in the quality control nucleosides of the molecule adapters. Likewise, the conversion rate for the whole sample (or partition/subsample thereof) may be determined by dividing the total number of correctly converted known unmodified nucleosides in the uniquely identified molecules of the sample by the total number of known unmodified nucleosides in the same set of uniquely identified sample molecules. In other words, the conversion rate for the whole sample or molecule, including the nucleosides with unknown modification status, is inferred from the conversion rate of the known unmodified nucleosides in the adapter sequences. The conversion rate can be used as a quality control measure of the conversion rate at the sample level or the molecular level. In some cases, individual molecules or samples that have sub-optimal conversion rates, e.g. a conversion rate that is below a pre-determined threshold, may be excluded from further analysis. In other cases, the conversion rate may be partial or sub-optimal, but can still be used for analysis, taking into account the known incomplete conversion of unmodified nucleosides in the molecule or sample. For example, a score or weighting may be applied to each molecule and/or the sample that is dependent on the determined conversion rate(s). Molecules or samples with higher conversion rates are assigned a higher weighting, i.e. are attributed a greater significance, in downstream analysis or determination of the modified nucleoside profile of the sample DNA. Molecules or samples with lower relative conversion rates are assigned a lower weighting, i.e. are attributed a lesser significance in downstream analysis. For example, the DNA may be from a subject suspected of having cancer. The analysis comprises determining the modified nucleoside profile of multiple epigenetic target regions where an altered modified nucleoside profile has been associated with cancer. The method of the disclosure may determine different conversion rates for the DNA molecules from each of the target regions. The diagnostic value of the modified nucleoside profiles determined by sequencing molecules within the regions having lower conversion rates is decreased compared to the diagnostic value of the modified nucleoside profiles determined by sequencing molecules within the regions having higher conversion rates. Hence, the lower conversion rate regions are assigned a lesser weighting when the modified nucleoside profile analysis from all of the regions is combined, typically also with other indicators such as the detection of cancer-associated sequence mutations, to provide the overall diagnostic determination, e.g. as a summation of risk scores.

### Exemplary Applications

The methods presented herein may be used as part of any method that benefits from obtaining an accurate modified nucleoside profile of DNA in any sample.

### Samples

A sample can be any biological sample isolated from a subject. A sample can be a bodily sample. Samples can include body tissues or fluids, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, gingival crevicular fluid, bone marrow, pleural effusions, pleura fluid, cerebrospinal fluid, saliva, mucous, sputum, semen, sweat, and urine. Samples are preferably body fluids, particularly blood and fractions thereof, cerebrospinal fluid, pleura fluid, saliva, sputum, or urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids.

In some embodiments, a population of nucleic acids is obtained from a serum, plasma or blood sample from a subject suspected of having neoplasia, a tumor, precancer, or cancer or previously diagnosed with neoplasia, a tumor, precancer, or cancer. The population includes nucleic acids having varying levels of sequence variation, epigenetic variation, and/or post-replication or transcriptional modifications. Post-replication modifications include modifications of cytosine, particularly at the 5-position of the nucleobase, e.g., 5-methylcytosine, 5-hydroxymethylcytosine, 5-formylcytosine and 5-carboxylcytosine.

A sample can be isolated or obtained from a subject and transported to a site of sample analysis. The sample may be preserved and shipped at a desirable temperature, e.g., room temperature, 4°C, -20°C, and/or -80°C. A sample can be isolated or obtained from a subject at the site of the sample analysis. The subject can be a human, a mammal, an animal, a companion animal, a service animal, or a pet. The subject may have a cancer, precancer, infection, transplant rejection, or other disease or disorder related to changes in the immune system. The subject may not have cancer or a detectable cancer symptom. The subject may have been treated with one or more cancer therapy, e.g., any one or more of chemotherapies, antibodies, vaccines or biologies. The subject may be in remission. The subject may or may not be diagnosed of being susceptible to cancer or any cancer-associated genetic mutations/disorders.

In some embodiments, the sample comprises plasma. The volume of plasma obtained can depend on the desired read depth for sequenced regions. Exemplary volumes are 0.4-40 ml, 5-20 ml, 10-20 ml. For examples, the volume can be 0.5 mL, 1 mL, 5 mL 10 mL, 20 mL, 30 mL, or 40 mL. A volume of sampled plasma may be 5 to 20 mL.

A sample can comprise various amount of nucleic acid that contains genome equivalents. For example, a sample of about 30 ng DNA can contain about 10,000 (10⁴) haploid human genome equivalents and, in the case of cfDNA, about 200 billion (2x10¹¹) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells and cell-free of the same subject, from cells and cell-free of different subjects. A sample can comprise nucleic acids carrying mutations. For example, a sample can comprise DNA carrying germline mutations and/or somatic mutations. Germline mutations refer to mutations existing in germline DNA of a subject. Somatic mutations refer to mutations originating in somatic cells of a subject, e.g., cancer cells. A sample can comprise DNA carrying cancer-associated mutations (e.g., cancer-associated somatic mutations). A sample can comprise an epigenetic variant (i.e., a chemical or protein modification), wherein the epigenetic variant associated with the presence of a genetic variant such as a cancer-associated mutation. In some embodiments, the sample comprises an epigenetic variant associated with the presence of a genetic variant, wherein the sample does not comprise the genetic variant.

Exemplary amounts of cell-free nucleic acids in a sample before amplification range from about 1 fg to about 1 µg, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng of cell-free nucleic acid molecules.

Cell-free DNA refers to DNA not contained within a cell at the time of its isolation from a subject. For example, cfDNA can be isolated from a sample as the DNA remaining in the sample after removing intact cells, without lysing the cells or otherwise extracting intracellular DNA. Cell- free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells. In some embodiments, cfDNA is cell-free fetal DNA (cffDNA) In some embodiments, cell free nucleic acids are produced by tumor cells. In some embodiments, cell free nucleic acids are produced by a mixture of tumor cells and non-tumor cells.

Cell-free nucleic acids have an exemplary size distribution of about 100-500 nucleotides, with molecules of 110 to about 230 nucleotides representing about 90% of molecules, with a mode of about 168 nucleotides and a second minor peak in a range between 240 to 440 nucleotides.

Cell-free nucleic acids can be isolated from bodily fluids through a fractionation or partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica based columns to remove contaminants or salts. Non-specific bulk carrier nucleic acids, such as herring sperm DNA, may be added throughout the reaction to optimize certain aspects of the procedure such as yield.

After such processing, samples can include various forms of nucleic acid including double stranded DNA, single stranded DNA and single stranded RNA. In some embodiments, single stranded DNA and RNA can be converted to double stranded forms so they are included in subsequent processing and analysis steps.

Double-stranded DNA molecules in a sample and single stranded nucleic acid molecules converted to double stranded DNA molecules can be linked to adapters at either one end or both ends. Typically, double stranded molecules are blunt ended by treatment with a polymerase with a 5'-3' polymerase and a 3 '-5' exonuclease (or proof reading function), in the presence of all four standard nucleotides. Klenow large fragment and T4 polymerase are examples of suitable polymerase. The blunt ended DNA molecules can be ligated with at least partially double stranded adapter (e.g., a Y shaped or bell-shaped adapter). Alternatively, complementary nucleotides can be added to blunt ends of sample nucleic acids and adapters to facilitate ligation. Contemplated herein are both blunt end ligation and sticky end ligation. In blunt end ligation, both the nucleic acid molecules and the adapter tags have blunt ends. In sticky-end ligation, typically, the nucleic acid molecules bear an "A" overhang and the adapters bear a "T" overhang.

### Amplification of DNA Comprising Adapters

Sample nucleic acids flanked by adapters can be amplified by PCR and other amplification methods. Such amplification can be used to increase the amount of DNA available for subsequent steps, such as sequencing. For example, this kind of amplification can be performed as part of library preparation and/or after preparation of a targeted library. The amplification may be performed after a conversion procedure. Amplification can be primed by primers binding to primer binding sites in adapters flanking a DNA molecule to be amplified. Amplification methods can involve cycles of denaturation, annealing and extension, resulting from thermocycling or can be isothermal as in transcription-mediated amplification. Other amplification methods include the ligase chain reaction, strand displacement amplification, nucleic acid sequence based amplification, and self-sustained sequence based replication.

In some embodiments, the present methods perform dsDNA ligations with T-tailed and C-tailed adapters, which result in amplification of at least 50, 60, 70 or 80% of double stranded nucleic acids before linking to adapters. Preferably the present methods increase the amount or number of amplified molecules relative to control methods performed with T-tailed adapters alone by at least 10, 15 or 20%.

### Applications

One important exemplary application of the methods of the disclosure is using the modified nucleoside profile in diagnosing and prognosing cancer or other genetic diseases or conditions.

Hence, in some embodiments, a method described herein comprises identifying or predicting the presence or absence of DNA produced by a tumor (or neoplastic cells, or cancer cells), determining the probability that a test subject has a tumor or cancer, and/or characterizing a tumor, neoplastic cells or cancer as described herein.

The present methods can be used to diagnose presence or absence of conditions, particularly cancer, in a subject, to characterize conditions (e.g., staging cancer or determining heterogeneity of a cancer), monitor response to treatment of a condition, effect prognosis risk of developing a condition or subsequent course of a condition. The present disclosure can also be useful in determining the efficacy of a particular treatment option. Successful treatment options may increase the amount of copy number variation or rare mutations detected in subject's blood if the treatment is successful as more cancers may die and shed DNA. In other examples, this may not occur. In another example, perhaps certain treatment options may be correlated with genetic profiles of cancers over time. This correlation may be useful in selecting a therapy. In some embodiments, hypermethylation variable epigenetic target regions are analyzed to determine whether they show hypermethylation characteristic of tumor cells or cells that do not ordinarily contribute significantly to cfDNA and/or hypomethylation variable epigenetic target regions are analyzed to determine whether they show hypomethylation characteristic of tumor cells or cells that do not ordinarily contribute significantly to cfDNA.

In some embodiments, the present methods are used for screening for a cancer, or in a method for screening cancer. For example, the sample can be from a subject who has not been previously diagnosed with cancer. In some embodiments, the subject may or may not have cancer. In some embodiments, the subject may or may not have an early-stage cancer. In some embodiments, the subject has one or more risk factors for cancer, such as tobacco use (e.g., smoking), being overweight or obese, having a high body mass index (BMI), being of advanced age, poor nutrition, high alcohol consumption, or a family history of cancer.

In some embodiments, the subject has used tobacco, e.g., for at least 1, 5, 10, or 15 years. In some embodiments, the subject has a high BMI, e.g., a BMI of 25 or greater, 26 or greater, 27 or greater, 28 or greater, 29 or greater, or 30 or greater. In some embodiments, the subject is at least 40, 45, 50, 55, 60, 65, 70, 75, or 80 years old. In some embodiments, the subject has poor nutrition, e.g., high consumption of one or more of red meat and/or processed meat, trans fat, saturated fat, and refined sugars, and/or low consumption of fruits and vegetables, complex carbohydrates, and/or unsaturated fats. High and low consumption can be defined, e.g., as exceeding or falling below, respectively, recommendations in Dietary Guidelines for Americans 2020-2025, available at www.dietaryguidelines.gov/sites/default/files/2021-03/Dietary_Guidelines_for_Americans-2020-2025.pdf . In some embodiments, the subject has high alcohol consumption, e.g., at least three, four, or five drinks per day on average (where a drink is about one ounce or 30 mL of 80-proof hard liquor or the equivalent). In some embodiments, the subject has a family history of cancer, e.g., at least one, two, or three blood relatives were previously diagnosed with cancer. In some embodiments, the relatives are at least third-degree relatives (e.g., great-grandparent, great aunt or uncle, first cousin), at least second-degree relatives (e.g., grandparent, aunt or uncle, or half-sibling), or first-degree relatives (e.g., parent or full sibling).

Additionally, if a cancer is observed to be in remission after treatment, the present methods can be used to monitor residual disease or recurrence of disease.

Typically, the disease under consideration is a type of cancer, such as any referred to herein. The types and number of cancers that may be detected may include blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like. Specific examples of such cancers include biliary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, Wilms tumor, leukemia, acute lymphocytic leukemia (ALL), acute myeloid leukemia (AML), chronic lymphocytic leukemia (CLL), chronic myeloid leukemia (CML), chronic myelomonocytic leukemia (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphoma/leukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas. Prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, or uterine sarcoma.

Type and/or stage of cancer can be detected from genetic variations including mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, and abnormal changes in nucleic acid 5-methylcytosine. Hence, the present methods can in some cases be used in combination with methods used to detect other genetic/epigenetic variations, e.g. in a method of detecting or characterizing a cancer or other methods described herein.

In some embodiments, a method described herein comprises identifying the presence of target regions and/or DNA produced by a tumor (or neoplastic cells, or cancer cells) or by precancer cells. In some embodiments, a method described herein comprises determining the level of target regions and/or identifying the presence of DNA produced by a tumor (or neoplastic cells, or cancer cells) or by precancer cells. In some embodiments, determining the level of target regions comprises determining either an increased level or decreased level of target regions, wherein the increased or decreased level of target regions is determined by comparing the level of target regions with a threshold level/value.

Genetic and/or epigenetic data can also be used for characterizing a specific form of cancer. Cancers are often heterogeneous in both composition and staging. Genetic and/or epigenetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers can progress to become more aggressive and genetically unstable. Other cancers may remain benign, inactive or dormant. The system and methods of this disclosure may be useful in determining disease progression.

Further, the methods of the disclosure may be used to characterize the heterogeneity of an abnormal condition in a subject. Such methods can include, e.g., generating a genetic and/or epigenetic profile of extracellular polynucleotides derived from the subject, wherein the genetic and/or epigenetic profile comprises a plurality of data resulting from copy number variation and rare mutation analyses. In some embodiments, an abnormal condition is cancer, e.g. as described herein. In some embodiments, the abnormal condition may be one resulting in a heterogeneous genomic population. In the example of cancer, some tumors are known to comprise tumor cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic and/or epigenetic information derived from different cells in a heterogeneous disease. This set of data may comprise copy number variation, epigenetic variation, and mutation analyses alone or in combination.

The present methods can be used to diagnose, prognose, monitor or observe cancers, or other diseases. In some embodiments, the methods herein do not involve the diagnosing, prognosing or monitoring a fetus and as such are not directed to non-invasive prenatal testing. In other embodiments, these methodologies may be employed in a pregnant subject to diagnose, prognose, monitor or observe cancers or other diseases in an unborn subject whose DNA and other polynucleotides may co-circulate with maternal molecules.

Non-limiting examples of other genetic-based diseases, disorders, or conditions that are optionally evaluated using the methods and systems disclosed herein include achondroplasia, alpha-1 antitrypsin deficiency, antiphospholipid syndrome, autism, autosomal dominant polycystic kidney disease, Charcot-Marie-Tooth (CMT), cri du chat, Crohn's disease, cystic fibrosis, Dercum disease, down syndrome, Duane syndrome, Duchenne muscular dystrophy, Factor V Leiden thrombophilia, familial hypercholesterolemia, familial Mediterranean fever, fragile X syndrome, Gaucher disease, hemochromatosis, hemophilia, holoprosencephaly, Huntington's disease, Klinefelter syndrome, Marfan syndrome, myotonic dystrophy, neurofibromatosis, Noonan syndrome, osteogenesis imperfecta, Parkinson's disease, phenylketonuria, Poland anomaly, porphyria, progeria, retinitis pigmentosa, severe combined immunodeficiency (SCID), sickle cell disease, spinal muscular atrophy, Tay-Sachs, thalassemia, trimethylaminuria, Turner syndrome, velocardiofacial syndrome, WAGR syndrome, Wilson disease, or the like.

In some embodiments, a method provided herein is a method of determining a risk of cancer recurrence in a subject. In some embodiments, a method provided herein is a method of classifying a subject as being a candidate for a subsequent cancer treatment.

Any of such methods may comprise collecting a sample from the subject diagnosed with the cancer at one or more preselected timepoints following one or more previous cancer treatments to the subject. The subject may be any of the subjects described herein. The sample may comprise DNA, e.g., cfDNA. The DNA may be obtained from a tissue sample or a liquid sample.

Any of such methods may comprise contacting the sample or a subsample thereof with a plurality of primers, generating capture probes, capturing and detecting the presence or level of at least one structural variation according to any of the embodiments as described herein. In some embodiments, the methods may comprise contacting the sample or a subsample thereof with a plurality of capture probes specific for members of an epigenetic target region set according to any of the embodiments as described herein. In some embodiments, the capture probes comprise capture probes generated using a sample obtained from the same subject at an earlier timepoint. The methods may further comprise capturing a plurality of sets of target regions from DNA from the subject, wherein the plurality of target region sets comprise a sequence-variable target region set, and/or an epigenetic target region set, whereby a captured set of DNA molecules is produced. The capturing step or steps may be performed according to any of the embodiments described elsewhere herein. Any of such methods may comprise sequencing the captured DNA molecules, whereby a set of sequence information is produced. The captured DNA molecules of a sequence-variable target region set may be sequenced to a greater depth of sequencing than the captured DNA molecules of the epigenetic target region set. Any of such methods may comprise detecting a presence or absence of DNA originating or derived from a tumor cell at a preselected timepoint using the set of sequence information. The detection of the presence or absence of DNA originating or derived from a tumor cell may be performed according to any of the embodiments thereof described elsewhere herein.

In any of such methods, the previous cancer treatment may comprise surgery, administration of a therapeutic composition, and/or chemotherapy.

Methods of determining a risk of cancer recurrence in a subject may comprise determining a cancer recurrence score that is indicative of the presence or absence, or amount, of at least one rearranged DNA sequence, type-specific target regions, and/or the DNA originating or derived from the tumor cell for the subject. The cancer recurrence score may further be used to determine a cancer recurrence status. The cancer recurrence status may be at risk for cancer recurrence, e.g., when the cancer recurrence score is above a predetermined threshold. The cancer recurrence status may be at low or lower risk for cancer recurrence, e.g., when the cancer recurrence score is above a predetermined threshold. In particular embodiments, a cancer recurrence score equal to the predetermined threshold may result in a cancer recurrence status of either at risk for cancer recurrence or at low or lower risk for cancer recurrence.

Methods of classifying a subject as being a candidate for a subsequent cancer treatment may comprise comparing the cancer recurrence score of the subject with a predetermined cancer recurrence threshold, thereby classifying the subject as a candidate for the subsequent cancer treatment when the cancer recurrence score is above the cancer recurrence threshold or not a candidate for therapy when the cancer recurrence score is below the cancer recurrence threshold. In particular embodiments, a cancer recurrence score equal to the cancer recurrence threshold may result in classification as either a candidate for a subsequent cancer treatment or not a candidate for therapy. In some embodiments, the subsequent cancer treatment comprises chemotherapy or administration of a therapeutic composition.

Any of such methods may comprise determining a disease-free survival (DFS) period for the subject based on the cancer recurrence score; for example, the DFS period may be 1 year, 2 years, 3, years, 4 years, 5 years, or 10 years.

In some embodiments, the set of sequence information comprises sequence-variable target region sequences and determining the cancer recurrence score may comprise determining at least a first subscore indicative of the levels of particular immune cell types, SNVs, insertions/deletions, CNVs and/or fusions present in sequence-variable target region sequences.

In some embodiments, a number of mutations in the sequence-variable target regions chosen from 1, 2, 3, 4, or 5 is sufficient for the first subscore to result in a cancer recurrence score classified as positive for cancer recurrence. In some embodiments, the number of mutations is chosen from 1, 2, or 3.

In some embodiments, the set of sequence information comprises epigenetic target region sequences, and determining the cancer recurrence score comprises determining a second subscore indicative of the amount of molecules (obtained from the epigenetic target region sequences) that represent an epigenetic state different from DNA found in a corresponding sample from a healthy subject (e.g., cfDNA found in a blood sample from a healthy subject, or DNA found in a tissue sample from a healthy subject where the tissue sample is of the same type of tissue as was obtained from the test subject). These abnormal molecules (i.e., molecules with an epigenetic state different from DNA found in a corresponding sample from a healthy subject) may be consistent with epigenetic changes associated with cancer, e.g., methylation of hypermethylation variable target regions and/or perturbed fragmentation of fragmentation variable target regions, where "perturbed" means different from DNA found in a corresponding sample from a healthy subject.

In some embodiments, a proportion of molecules corresponding to the hypermethylation variable target region set and/or fragmentation variable target region set that indicate hypermethylation in the hypermethylation variable target region set and/or abnormal fragmentation in the fragmentation variable target region set greater than or equal to a value in the range of 0.001%-10% is sufficient for the second subscore to be classified as positive for cancer recurrence. The range may be 0.001%-1%, 0.005%-1%, 0.01%-5%, 0.01%-2%, or 0.01%-1%.

In some embodiments, any of such methods may comprise determining a fraction of tumor DNA from the fraction of molecules in the set of sequence information that indicate one or more features indicative of origination from a tumor cell. This may be done for molecules corresponding to some or all of the epigenetic target regions, e.g., including one or both of hypermethylation variable target regions and fragmentation variable target regions (hypermethylation of a hypermethylation variable target region and/or abnormal fragmentation of a fragmentation variable target region may be considered indicative of origination from a tumor cell). This may be done for molecules corresponding to sequence variable target regions, e.g., molecules comprising alterations consistent with cancer, such as SNVs, indels, CNVs, and/or fusions. The fraction of tumor DNA may be determined based on a combination of molecules corresponding to epigenetic target regions and molecules corresponding to sequence variable target regions.

Determination of a cancer recurrence score may be based at least in part on the fraction of tumor DNA, wherein a fraction of tumor DNA greater than a threshold in the range of 10⁻¹¹ to 1 or 10⁻¹⁰ to 1 is sufficient for the cancer recurrence score to be classified as positive for cancer recurrence. In some embodiments, a fraction of tumor DNA greater than or equal to a threshold in the range of 10⁻¹⁰ to 10⁻⁹, 10⁻⁹ to 10⁻⁸, 10⁻⁸ to 10⁻⁷, 10⁻⁷ to 10⁻⁶, 10⁻⁶ to 10⁻⁵, 10⁻⁵ to 10⁻⁴, 10⁻⁴ to 10⁻³, 10⁻³ to 10⁻², or 10⁻² to 10⁻¹ is sufficient for the cancer recurrence score to be classified as positive for cancer recurrence. In some embodiments, the fraction of tumor DNA greater than a threshold of at least 10⁻⁷ is sufficient for the cancer recurrence score to be classified as positive for cancer recurrence. A determination that a fraction of tumor DNA is greater than a threshold, such as a threshold corresponding to any of the foregoing embodiments, may be made based on a cumulative probability. For example, the sample was considered positive if the cumulative probability that the tumor fraction was greater than a threshold in any of the foregoing ranges exceeds a probability threshold of at least 0.5, 0.75, 0.9, 0.95, 0.98, 0.99, 0.995, or 0.999. In some embodiments, the probability threshold is at least 0.95, such as 0.99.

In some embodiments, the set of sequence information comprises sequence-variable target region sequences and epigenetic target region sequences, and determining the cancer recurrence score comprises determining a first subscore indicative of the amount of SNVs, insertions/deletions, CNVs and/or fusions present in sequence-variable target region sequences and a second subscore indicative of the amount of abnormal molecules in epigenetic target region sequences, and combining the first and second subscores to provide the cancer recurrence score. Where the first and second subscores are combined, they may be combined by applying a threshold to each subscore independently (e.g., greater than a predetermined number of mutations (e.g., > 1) in sequence-variable target regions, and greater than a predetermined fraction of abnormal molecules (i.e., molecules with an epigenetic state different from the DNA found in a corresponding sample from a healthy subject; e.g., tumor) in epigenetic target regions), or training a machine learning classifier to determine status based on a plurality of positive and negative training samples.

In some embodiments, a value for the combined score in the range of -4 to 2 or -3 to 1 is sufficient for the cancer recurrence score to be classified as positive for cancer recurrence.

In any embodiment where a cancer recurrence score is classified as positive for cancer recurrence, the cancer recurrence status of the subject may be at risk for cancer recurrence and/or the subject may be classified as a candidate for a subsequent cancer treatment.

In some embodiments, the cancer is any one of the types of cancer described elsewhere herein, e.g., colorectal cancer.

In certain embodiments, the methods disclosed herein relate to identifying and administering customized therapies to patients. In some embodiments, determination of the levels of particular nucleic acids facilitates selection of appropriate treatment. In some embodiments, the patient or subject has a given disease, disorder, or condition. Essentially any cancer therapy (e.g., surgical therapy, radiation therapy, chemotherapy, and/or the like) may be included as part of these methods. In certain embodiments, the therapy administered to a subject comprises at least one chemotherapy drug. In some embodiments, the chemotherapy drug may comprise alkylating agents (for example, but not limited to, Chlorambucil, Cyclophosphamide, Cisplatin and Carboplatin), nitrosoureas (for example, but not limited to, Carmustine and Lomustine), anti-metabolites (for example, but not limited to, Fluorauracil, Methotrexate and Fludarabine), plant alkaloids and natural products (for example, but not limited to, Vincristine, Paclitaxel and Topotecan), anti- tumor antibiotics (for example, but not limited to, Bleomycin, Doxorubicin and Mitoxantrone), hormonal agents (for example, but not limited to, Prednisone, Dexamethasone, Tamoxifen and Leuprolide) and biological response modifiers (for example, but not limited to, Herceptin and Avastin, Erbitux and Rituxan). In some embodiments, the chemotherapy administered to a subject may comprise FOLFOX or FOLFIRI. In certain embodiments, a therapy may be administered to a subject that comprises at least one PARP inhibitor. In certain embodiments, the PARP inhibitor may include OLAPARIB, TALAZOPARIB, RUCAPARIB, NIRAPARIB (trade name ZEJULA), among others. Typically, therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In some embodiments, therapy is customized based on the status of a nucleic acid variant as being of somatic or germline origin. In some embodiments, essentially any cancer therapy (e.g., surgical therapy, radiation therapy, chemotherapy, and/or the like) may be included as part of these methods.

In some embodiments, the immunotherapy or immunotherapeutic agents targets an immune checkpoint molecule. Certain tumors are able to evade the immune system by co-opting an immune checkpoint pathway. Thus, targeting immune checkpoints has emerged as an effective approach for countering a tumor's ability to evade the immune system and activating anti-tumor immunity against certain cancers. Pardoll, Nature Reviews Cancer, 2012, 12:252-264.

In certain embodiments, the immune checkpoint molecule is an inhibitory molecule that reduces a signal involved in the T cell response to antigen. For example, CTLA4 is expressed on T cells and plays a role in downregulating T cell activation by binding to CD80 (aka B7.1) or CD86 (aka B7.2) on antigen presenting cells. PD-1 is another inhibitory checkpoint molecule that is expressed on T cells. PD-1 limits the activity of T cells in peripheral tissues during an inflammatory response. In addition, the ligand for PD-1 (PD-L1 or PD-L2) is commonly upregulated on the surface of many different tumors, resulting in the downregulation of anti-tumor immune responses in the tumor microenvironment. In certain embodiments, the inhibitory immune checkpoint molecule is CTLA4 or PD-1. In other embodiments, the inhibitory immune checkpoint molecule is a ligand for PD-1, such as PD-L1 or PD-L2. In other embodiments, the inhibitory immune checkpoint molecule is a ligand for CTLA4, such as CD80 or CD86. In other embodiments, the inhibitory immune checkpoint molecule is lymphocyte activation gene 3 (LAG3), killer cell immunoglobulin like receptor (KIR), T cell membrane protein 3 (TIM3), galectin 9 (GAL9), or adenosine A2a receptor (A2aR).

Antagonists that target these immune checkpoint molecules can be used to enhance antigen-specific T cell responses against certain cancers. Accordingly, in certain embodiments, the immunotherapy or immunotherapeutic agent is an antagonist of an inhibitory immune checkpoint molecule. In certain embodiments, the inhibitory immune checkpoint molecule is PD-1. In certain embodiments, the inhibitory immune checkpoint molecule is PD-L1. In certain embodiments, the antagonist of the inhibitory immune checkpoint molecule is an antibody (e.g., a monoclonal antibody). In certain embodiments, the antibody or monoclonal antibody is an anti-CTLA4, anti-PD-1, anti-PD-L1, or anti-PD-L2 antibody. In certain embodiments, the antibody is a monoclonal anti-PD-1 antibody. In some embodiments, the antibody is a monoclonal anti-PD-L1 antibody. In certain embodiments, the monoclonal antibody is a combination of an anti-CTLA4 antibody and an anti-PD-1 antibody, an anti-CTLA4 antibody and an anti-PD-L1 antibody, or an anti-PD-L1 antibody and an anti-PD-1 antibody. In certain embodiments, the anti-PD-1 antibody is one or more of pembrolizumab (Keytruda^{®}) or nivolumab (Opdivo^{®}). In certain embodiments, the anti-CTLA4 antibody is ipilimumab (Yervoy^{®}). In certain embodiments, the anti-PD-L1 antibody is one or more of atezolizumab (Tecentriq^{®}), avelumab (Bavencio^{®}), or durvalumab (Imfinzi^{®}).

In certain embodiments, the immunotherapy or immunotherapeutic agent is an antagonist (e.g. antibody) against CD80, CD86, LAG3, KIR, TIM3, GAL9, or A2aR. In other embodiments, the antagonist is a soluble version of the inhibitory immune checkpoint molecule, such as a soluble fusion protein comprising the extracellular domain of the inhibitory immune checkpoint molecule and an Fc domain of an antibody. In certain embodiments, the soluble fusion protein comprises the extracellular domain of CTLA4, PD-1, PD-L1, or PD-L2. In some embodiments, the soluble fusion protein comprises the extracellular domain of CD80, CD86, LAG3, KIR, TIM3, GAL9, or A2aR. In one embodiment, the soluble fusion protein comprises the extracellular domain of PD-L2 or LAG3.

In certain embodiments, the immune checkpoint molecule is a co-stimulatory molecule that amplifies a signal involved in a T cell response to an antigen. For example, CD28 is a co-stimulatory receptor expressed on T cells. When a T cell binds to antigen through its T cell receptor, CD28 binds to CD80 (aka B7.1) or CD86 (aka B7.2) on antigen-presenting cells to amplify T cell receptor signaling and promote T cell activation. Because CD28 binds to the same ligands (CD80 and CD86) as CTLA4, CTLA4 is able to counteract or regulate the co-stimulatory signaling mediated by CD28. In certain embodiments, the immune checkpoint molecule is a co-stimulatory molecule selected from CD28, inducible T cell co-stimulator (ICOS), CD137, OX40, or CD27. In other embodiments, the immune checkpoint molecule is a ligand of a co-stimulatory molecule, including, for example, CD80, CD86, B7RP1, B7-H3, B7-H4, CD137L, OX40L, or CD70.

Agonists that target these co-stimulatory checkpoint molecules can be used to enhance antigen-specific T cell responses against certain cancers. Accordingly, in certain embodiments, the immunotherapy or immunotherapeutic agent is an agonist of a co-stimulatory checkpoint molecule. In certain embodiments, the agonist of the co-stimulatory checkpoint molecule is an agonist antibody and preferably is a monoclonal antibody. In certain embodiments, the agonist antibody or monoclonal antibody is an anti-CD28 antibody. In other embodiments, the agonist antibody or monoclonal antibody is an anti-ICOS, anti-CD137, anti-OX40, or anti-CD27 antibody. In other embodiments, the agonist antibody or monoclonal antibody is an anti-CD80, anti-CD86, anti-B7RP1, anti-B7-H3, anti-B7-H4, anti-CD137L, anti-OX40L, or anti-CD70 antibody.

In certain embodiments, the status of a nucleic acid variant from a sample from a subject as being of somatic or germline origin may be compared with a database of comparator results from a reference population to identify customized or targeted therapies for that subject. Typically, the reference population includes patients with the same cancer or disease type as the subject and/or patients who are receiving, or who have received, the same therapy as the subject. A customized or targeted therapy (or therapies) may be identified when the nucleic variant and the comparator results satisfy certain classification criteria (e.g., are a substantial or an approximate match).

In certain embodiments, the customized therapies described herein are typically administered parenterally (e.g., intravenously or subcutaneously). Pharmaceutical compositions containing an immunotherapeutic agent are typically administered intravenously. Certain therapeutic agents are administered orally. However, customized therapies (e.g., immunotherapeutic agents, etc.) may also be administered by any method known in the art, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

Therapeutic options for treating specific genetic-based diseases, disorders, or conditions, other than cancer, are generally well-known to those of ordinary skill in the art and will be apparent given the particular disease, disorder, or condition under consideration.

In some embodiments, e.g., where genetic variants are detected, therapy is customized based on the status of a nucleic acid variant as being of somatic or germline origin. In some embodiments, essentially any cancer therapy (e.g., surgical therapy, radiation therapy, chemotherapy, and/or the like) may be included as part of these methods. Typically, customized therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In certain embodiments, the status of a nucleic acid variant from a sample from a subject as being of somatic or germline origin may be compared with a database of comparator results from a reference population to identify customized or targeted therapies for that subject. Typically, the reference population includes patients with the same cancer or disease type as the subject and/or patients who are receiving, or who have received, the same therapy as the subject. A customized or targeted therapy (or therapies) may be identified when the nucleic variant and the comparator results satisfy certain classification criteria (e.g., are a substantial or an approximate match).

In certain embodiments, the customized therapies described herein are typically administered parenterally (e.g., intravenously or subcutaneously). Pharmaceutical compositions containing an immunotherapeutic agent are typically administered intravenously. Certain therapeutic agents are administered orally. However, customized therapies (e.g., immunotherapeutic agents, etc.) may also be administered by methods such as, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

### Kits

Also provided are kits comprising the adapters as described herein. The adapters comprise quality control nucleosides. The kits can be for use in performing the methods as described herein. In some cases, a kit comprises a plurality of adapters.

In some cases, the kit comprises one or more conversion reagents. In some cases, a conversion reagent changes the base pairing specificity of the quality control nucleosides. In some cases, a conversion reagent does not change the base pairing specificity of the quality control nucleosides. In some cases, the kit includes conversion reagents used in bisulfite conversion, oxidative bisulfite conversion, Tet-assisted (TAB) conversion, Tet-assisted conversion with a substituted borane reducing agent, APOBEC-coupled epigenetic (ACE) conversion, or enzymatic conversion of a nucleobase. A reagent for protecting hmC (e.g., βGT) can be combined with a conversion reagent, such as Tet enzyme and/or a substituted borane reducing agent. In some cases, the one or more conversion reagents comprise Tet enzyme and/or a substituted borane reducing agent, such as any of those described elsewhere herein.

In some cases, a kit comprises a plurality of primers. In some cases, a kit comprises a plurality of capture probes. In some cases, the kit comprises a DNA polymerase. In some cases, the kit comprises deoxynucleoside triphosphates. In some cases, the kit comprises PCR primers that anneal to an adapter included in the kit. In some cases, the kit comprises additional elements described elsewhere herein. In some cases, the kit comprises instructions for performing a method described herein.

In some cases, a kit further comprises an agent that recognizes methyl cytosine in DNA. In some such cases, the agent is an antibody or a methyl binding protein or methyl binding domain. In some cases, the kit comprises capture probes that specifically bind to epigenetic and/or sequence-variable target region sets. In some such cases, the capture probes comprise a capture moiety. In some cases, the kit comprises a solid support linked to a binding partner of the capture moiety.

Kits may further comprise a plurality of oligonucleotide probes that selectively hybridize to least 5, 6, 7, 8, 9, 10, 20, 30, 40 or all genes selected from the group consisting of ALK, APC, BRAF, CDKN2A, EGFR, ERBB2, FBXW7, KRAS, MYC, NOTCH1, NRAS, PIK3CA, PTEN, RBI, TP53, MET, AR, ABLl, AKTl, ATM, CDHl, CSFIR, CTNNBI, ERBB4, EZH2, FGFRl, FGFR2, FGFR3, FLT3, GNA11, GNAQ, GNAS, HNF1A, HRAS, IDH1, IDH2, JAK2, JAK3, KDR, KIT, MLH1, MPL, NPM1, PDGFRA, PROC, PTPN11, RET,SMAD4, SMARCB1, SMO, SRC, STK11, VHL, TERT, CCND1, CDK4, CDKN2B, RAF1, BRCA1, CCND2, CDK6, NF1, TP53, ARID 1 A, BRCA2, CCNE1, ESR1, RIT1, GATA3, MAP2K1, RHEB, ROS1, ARAF, MAP2K2, NFE2L2, RHOA, and NTRKl . The number genes to which the oligonucleotide probes can selectively hybridize can vary. For example, the number of genes can comprise 1 , 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, or 54. The kit can include a container that includes the plurality of oligonucleotide probes and instructions for performing any of the methods described herein.

The kit can comprise at least 4, 5, 6, 7, or 8 different library adapters having distinct molecular barcodes and identical sample barcodes. The sample barcode may include the modified nucleotide, such as 5mC. The library adapters may not be sequencing adapters. For example, the library adapters do not include flow cell sequences or sequences that permit the formation of hairpin loops for sequencing. The different variations and combinations of molecular barcodes and sample barcodes are described throughout, and are applicable to the kit. Further, in some cases, the adapters are not sequencing adapters. Additionally, the adapters provided with the kit can also comprise sequencing adapters. A sequencing adapter can comprise a sequence hybridizing to one or more sequencing primers. A sequencing adapter can further comprise a sequence hybridizing to a solid support, e.g., a flow cell sequence. For example, a sequencing adapter can be a flow cell adapter. The sequencing adapters can be attached to one or both ends of a polynucleotide fragment. In some cases, the kit can comprise at least 8 different library adapters having distinct molecular barcodes and identical sample barcodes. The library adapters may not be sequencing adapters. The kit can further include a sequencing adapter having a first sequence that selectively hybridizes to the library adapters and a second sequence that selectively hybridizes to a flow cell sequence. In another example, a sequencing adapter can be hairpin shaped. For example, the hairpin shaped adapter can comprise a complementary double stranded portion and a loop portion, where the double stranded portion can be attached (e.g. , ligated) to a double-stranded polynucleotide. Hairpin shaped sequencing adapters can be attached to both ends of a polynucleotide fragment to generate a circular molecule, which can be sequenced multiple times. A sequencing adapter can comprise one or more barcodes. For example, a sequencing adapter can comprise a sample barcode. The sample barcode can comprise a pre-determined sequence. The sample barcodes can be used to identify the source of the polynucleotides. The sample barcode can be at least 1, 2, 3, 4, 5, 6, 7, 8, 9, 10, 11, 12, 13, 14, 15, 16, 17, 18, 19, 20, 21, 22, 23, 24, 25, or more (or any length as described throughout) nucleic acid bases, e.g., at least 8 bases. The barcode can be contiguous or non-contiguous sequences, as described above.

The library adapters can be blunt ended and Y-shaped and can be less than or equal to 40 nucleic acid bases in length. Other variations of the library adapters can be found throughout and are applicable to the kit.

### Computer Systems

Methods of the present disclosure can be implemented using, or with the aid of, computer systems. FIG. 2 shows a computer system 201 that is programmed or otherwise configured to implement the methods of the present disclosure. The computer system 201 can regulate various aspects sample preparation, sequencing, and/or analysis. In some examples, the computer system 201 is configured to perform sample preparation and sample analysis, including (where applicable) nucleic acid sequencing, e.g., according to any of the methods disclosed herein.

The computer system 201 includes a central processing unit (CPU, also "processor" and "computer processor" herein) 205, which can be a single core or multi core processor, or a plurality of processors for parallel processing. The computer system 201 also includes memory or memory location 210 (e.g., random-access memory, read-only memory, flash memory), electronic storage unit 215 (e.g., hard disk), communication interface 220 (e.g., network adapter) for communicating with one or more other systems, and peripheral devices 225, such as cache, other memory, data storage, and/or electronic display adapters. The memory 210, storage unit 215, interface 220, and peripheral devices 225 are in communication with the CPU 205 through a communication network or bus (solid lines), such as a motherboard. The storage unit 215 can be a data storage unit (or data repository) for storing data. The computer system 201 can be operatively coupled to a computer network 230 with the aid of the communication interface 220. The computer network 230 can be the Internet, an internet and/or extranet, or an intranet and/or extranet that is in communication with the Internet. The computer network 230 in some cases is a telecommunication and/or data network. The computer network 230 can include one or more computer servers, which can enable distributed computing, such as cloud computing. The computer network 230, in some cases with the aid of the computer system 201, can implement a peer-to-peer network, which may enable devices coupled to the computer system 201 to behave as a client or a server.

The CPU 205 can execute a sequence of machine-readable instructions, which can be embodied in a program or software. The instructions may be stored in a memory location, such as the memory 210. Examples of operations performed by the CPU 205 can include fetch, decode, execute, and writeback.

The storage unit 215 can store files, such as drivers, libraries, and saved programs. The storage unit 215 can store programs generated by users and recorded sessions, as well as output(s) associated with the programs. The storage unit 215 can store user data, e.g., user preferences and user programs. The computer system 201 in some cases can include one or more additional data storage units that are external to the computer system 201, such as located on a remote server that is in communication with the computer system 201 through an intranet or the Internet. Data may be transferred from one location to another using, for example, a communication network or physical data transfer (e.g., using a hard drive, thumb drive, or other data storage mechanism).

The computer system 201 can communicate with one or more remote computer systems through the network 230. For embodiment, the computer system 201 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 201 via the network 230.

Methods as described herein can be implemented by way of machine (e.g., computer processor) executable code stored on an electronic storage location of the computer system 201, such as, for example, on the memory 210 or electronic storage unit 215. The machine executable or machine-readable code can be provided in the form of software. During use, the code can be executed by the processor 205. In some cases, the code can be retrieved from the storage unit 215 and stored on the memory 210 for ready access by the processor 205. In some situations, the electronic storage unit 215 can be precluded, and machine-executable instructions are stored on memory 210.

In an aspect, the present disclosure provides a non-transitory computer-readable medium comprising computer-executable instructions which, when executed by at least one electronic processor, perform at least a portion of a method described herein. For example, the method may comprise: ligating the DNA to oligonucleotide adapters, wherein the adapters comprise quality control nucleosides that include modified nucleosides, wherein the quality control nucleosides have the same nucleoside identity and the same or a different modification status to modified nucleosides to be detected in the DNA, and wherein the modification status of the quality control nucleosides is known; subjecting the adapted DNA, or a subsample thereof, to a conversion procedure; sequencing the adapted DNA after conversion step; using the sequence data to determine base pairing specificity conversion of the quality control nucleosides in the adapters; and using the base pairing specificity conversion of the quality control nucleosides in the adapters as a quality control measure for conversion step.

The code can be pre-compiled and configured for use with a machine have a processor adapted to execute the code or can be compiled during runtime. The code can be supplied in a programming language that can be selected to enable the code to execute in a pre-compiled or as-compiled fashion.

Aspects of the systems and methods provided herein, such as the computer system 201, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as those used across physical interfaces between local devices, through wired and optical landline networks, and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards, paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 201 can include or be in communication with an electronic display that comprises a user interface (UI) for providing, for example, one or more results of sample analysis. Examples of UIs include, without limitation, a graphical user interface (GUI) and web-based user interface.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense.

For example, all the methods, systems, computer readable media, and/or component features, steps, elements, or other aspects thereof can be used in various combinations.

If different versions of a sequence are associated with an accession number at different times, the version associated with the accession number at the effective filing date of this application is meant. The effective filing date means the earlier of the actual filing date or filing date of a priority application referring to the accession number, if applicable. Likewise, if different versions of a publication, website or the like are published at different times, the version most recently published at the effective filing date of the application is meant, unless otherwise indicated.

### EXAMPLES

Exemplary workflows for analyzing the modified nucleoside profile of nucleic acid in a sample and library preparation are provided herein. In some embodiments, some or all features of the partitioning and library preparation workflows may be used in combination.

An exemplary method is as follows:
1. Optional physical partitioning of an extracted DNA sample (e.g., extracted blood plasma DNA from a human sample, which has optionally been subjected to target capture as described herein) using, for example, a methyl-binding domain protein-bead purification kit, saving all elutions from process for downstream processing.
2. (Parallel) application of differential molecular tags and NGS-enabling adapter sequences to the sample DNA or to each partition. For example, a hypermethylated, residual methylation ('wash'), and a hypomethylated partition are ligated with NGS- adapters with molecular tags. Adapters for the sample, or at least one partition of the sample, e.g. a hypermethylated partition, comprise one or more quality control nucleosides, as described herein having known nucleoside identity and known modification status;
3. Subject the sample, or at least one partition of the sample, e.g. a hypermethylated partition, to a conversion procedure that changes the base pairing specificity of the known modified nucleosides in the adapters;
4. Re-combining all molecular tagged partitions, if applicable, and optional subsequent amplification using adapter-specific DNA primer sequences.
5. Capture/hybridization of (re-combined and/or amplified) total library, targeting genomic regions of interest (e.g., cancer-specific genetic variants and/or differentially methylated regions).
6. Optional re-amplification of the captured DNA library, appending a sample tag. Different samples are pooled, and assayed in multiplex on an NGS instrument.
7. Bioinformatics analysis of NGS data, with the molecular tags optionally being used to identify unique molecules, as well deconvolution of the sample into molecules that were differentially partitioned. Genetic sequencing/variant detection identifies quality control nucleosides in the adapter regions that have or haven't been correctly converted. Determining sample or molecular level conversion rates and using as a quality control measure for the analysis.
8. Optionally applying a score or weighting to each identified unique molecule depending on the conversion rate of the known modified nucleosides in the adapter regions. Applying the score or weighting in downstream analysis or inference from the sample data, for example when used in diagnostics or prognosis.

After optionally partitioning and/or concentrating the DNA, the DNA may be made ligatable, e.g., by extending the end overhangs of the DNA molecules, and adding adenosine residues to the 3' ends of fragments and phosphorylating the 5' end of each DNA fragment. DNA ligase and adapters are added to ligate each partitioned DNA molecule with an adapter on one or both ends. These adapters may contain partition tags (e.g., non-random, non-unique barcodes) that are distinguishable from the partition tags in the adapters used in the other partitions. After making the DNA ligatable and performing the ligation, the sample, or a partition thereof (e.g. a hypermethylated partition) is subjected to a conversion procedure that changes the base pairing specificity of nucleosides in the adapters dependent on modification status, as described herein, for example using the TAPS method. If the sample has been partitioned, then the partitions may be pooled together prior to amplification. Amplification may be by PCR, with primers specific for the adapters.

Following amplification, DNA may be cleaned and concentrated prior to enrichment. The amplified DNA may be contacted with a collection of probes, for example as described herein (which may be, e.g., biotinylated RNA probes) that target specific regions of interest. The mixture may be incubated, e.g., overnight, e.g., in a salt buffer. The probes may be captured (e.g., using streptavidin magnetic beads) and separated from the amplified DNA that was not captured, such as by a series of salt washes, thereby enriching the sample. After the enrichment, the enriched sample may be (further) amplified, for example by PCR. In some embodiments, the PCR primers contain a sample tag, thereby incorporating the sample tag into the DNA molecules. In some embodiments, DNA from different samples is pooled together and then multiplex sequenced, e.g., using an Illumina NovaSeq sequencer.

## Claims

1. A quality control method for monitoring false negative and/or false positive detection of modified nucleosides in DNA in a sample, the method comprising:
(a) ligating the DNA to oligonucleotide adapters, wherein the adapters comprise quality control nucleosides, wherein the adapter quality control nucleosides include a first quality control nucleoside which is modified and a second quality control nucleoside which is unmodified, wherein the quality control nucleosides have the same nucleoside identity and the same or a different modification status to modified nucleosides to be detected in the DNA, and wherein the modification status of the quality control nucleosides is known;
(b) subjecting the adapted DNA, or a subsample thereof, to a conversion procedure that changes the base pairing specificity of the quality control nucleosides or does not change the base pairing specificity of the quality control nucleosides, depending on the modification status of the nucleosides, wherein the conversion procedure is selected to
(i) change the base pairing specificity of adapted DNA nucleosides having the same nucleoside identity and modification status as quality control nucleosides in the adapters, and not change the base pairing specificity of adapted DNA nucleosides having the same nucleosides identity as quality control nucleosides in the adapters but a different modification status; and/or
(ii) not change the base pairing specificity of adapted DNA nucleosides having the same nucleoside identity and modification status as quality control nucleosides in the adapters, and change the base pairing specificity of adapted DNA nucleosides having the same pairing identity as quality control nucleosides in the adapters but a different modification status;
(c) sequencing the adapted DNA after conversion step (b);
(d) using the sequence data obtained in step (c) to determine base pairing specificity conversion of the quality control nucleosides in the adapters; and
(e) using the base pairing specificity conversion of the quality control nucleosides in the adapters as a quality control measure for conversion step (b), wherein sub-optimal conversion of adapter quality control nucleosides following a conversion procedure of step (b)(i) and/or erroneous conversion of adapter quality control nucleosides following a conversion procedure of step (b)(ii) predicts false negative and/or false positive detection of modified nucleosides in the DNA sample.

2. The method of claim 1, wherein the conversion procedure is selected to change the base pairing specificity of quality control nucleosides in the adapters, but not the base pairing specificity of DNA sample nucleosides having the same nucleoside identity and a different modification status; and wherein suboptimal conversion of the quality control nucleosides predicts false positive detection of DNA sample nucleosides having the different modification status; for example wherein the quality control nucleosides in the adapters include cytosine and/or wherein conversion procedure comprises bisulfite conversion.

3. The method of claim 1 or claim 2, wherein the first quality control nucleoside is a modified cytosine and the second quality control nucleoside is an unmodified cytosine, for example wherein the first quality control nucleoside is 5-methylcytosine (5mC) or 5-hydroxymethylcytosine (5hmC).

4. The method of any one of the preceding claims, wherein the conversion procedure is selected to
(i) change the base pairing specificity of the first quality control nucleoside but not the second quality control nucleoside;
(ii) change the base pairing specificity of the second quality control nucleoside but not the first quality control nucleoside; or
(iii) change the base pairing specificity of modified quality control nucleosides in the adapters, but not the base pairing specificity of DNA sample nucleosides having the same nucleoside identity but a different modification status and/or no modification; and wherein suboptimal conversion of the modified quality control nucleosides predicts false negative detection of DNA sample nucleosides having the same nucleoside identity and modification status as the quality control nucleosides or a different modification status and the same change in base pairing specificity on exposure to the conversion procedure.

5. The method of any one of the preceding claims, wherein the method further comprises using the sequence data obtained in step (c) to
(i) identify adapted DNA molecules with sub-optimal or erroneous conversion of quality control nucleosides in the adapter sequence; and
(ii) infer sub-optimal or erroneous conversion of nucleosides having the same nucleoside identity and modification status in the full length molecules identified in step (i).

6. The method of any one of the preceding claims, wherein the method further comprises determining the conversion rate for quality control nucleosides in the adapted DNA or in individual adapted DNA molecules and
(i) applying a weighting to analysis of the modified nucleoside detection in
(A) the DNA sample; or
(B) individual adapted DNA molecules,
wherein the weighting is dependent on the conversion rate;
(ii) excluding DNA samples having
(A) suboptimal conversion of adapter quality control nucleosides or a conversion rate for adapter quality control nucleosides that is below a pre-determined quality control threshold; and/or
(B) erroneous conversion of adapter quality control nucleosides, or a conversion rate for adapter quality control nucleosides that is above a pre-determined quality control threshold,
from further analysis for detecting modified nucleosides; and/or
(iii) excluding adapted DNA molecules having
(A) suboptimal conversion of adapter quality control nucleosides or a conversion rate for adapter quality control nucleosides that is below a pre-determined quality control threshold; and/or
(B) erroneous conversion of adapter quality control nucleosides, or a conversion rate for adapter quality control nucleosides that is above a pre-determined quality control threshold,
from further analysis for detecting modified nucleosides.

7. The method of any one of the preceding claims, wherein the method further comprises enriching the DNA by capturing a target region set from the sample, wherein the capture step is before, after or in between the ligating step (a) and the conversion step (b).

8. The method of any one of the preceding claims, further comprising
(i) comparing the sequence data obtained in step (c) with
(A) a pre-determined reference sequence; and/or
(B) sequence data obtained by sequencing a sub-sample of the DNA that was not subjected to the conversion procedure; and
(ii) identifying point differences between the converted DNA sequences and the reference sequence (A) or non-converted DNA sequence data (B) as nucleosides having a modification status that permits a change in base pairing specificity on exposure to the conversion procedure.

9. The method of any one of the preceding claims,
(i) wherein the DNA comprises cell-free DNA (cfDNA) obtained from a test subject, optionally wherein the test subject is a patient having or suspected of having cancer;
(ii) further comprising using the detection of modified nucleosides in the DNA sample to determine or predict the presence of DNA produced by a cancer cell or tumor, to determine the probability that a test subject has a tumor or cancer, or to characterize a cancer or tumor of the subject;
(iii) wherein a sub-sample of the DNA is not subjected to the conversion procedure before sequencing, wherein the converted subsample and the non-converted subsample have different adapter sequences, and wherein the converted subsample and the non-converted subsample are recombined for sequencing step (c);
(iv) further comprising analyzing the DNA to detect copy number variation, single nucleotide variants, insertions, deletions, methylation, and/or fusions;
(v) further comprising capturing epigenetic target regions from the adapter-ligated DNA and amplifying and sequencing the epigenetic target regions, wherein the captured epigenetic target regions form an epigenetic target region set; for example wherein the epigenetic target region set comprises a plurality of type-specific epigenetic target regions, and wherein the type-specific epigenetic target regions are type-specific differentially methylated regions and/or type specific fragments, further optionally wherein the plurality of type-specific epigenetic target regions comprises type-specific hypomethylated regions or type-specific hypermethylated regions; and/or
(vi) wherein the sample is a blood sample.

10. The method of claim 9, wherein (i) the type-specific epigenetic target regions comprise cell-type specific, tissue-type specific, and/or cancer-type specific epigenetic target regions; and/or (ii) the blood sample is fractionated prior to capturing at least an epigenetic target region set of DNA.

11. The method of any one of the preceding claims, further comprising:
partitioning the sample or an aliquot thereof into a plurality of partitioned subsamples, including a first partitioned subsample and a second partitioned subsample, wherein the first subsample comprises DNA with a cytosine modification in a greater proportion than the second subsample;
contacting the second partitioned subsample with a methylation-dependent nuclease, thereby degrading nonspecifically partitioned DNA in the second subsample to produce a treated second subsample and optionally contacting the first partitioned subsample with a methylation-sensitive endonuclease, thereby degrading nonspecifically partitioned DNA in the first partitioned subsample to produce a treated first subsample;
wherein epigenetic target regions are captured from at least a portion of the first subsample or the treated first subsample,
optionally wherein the DNA from the subject that is contacted with the one or more capture probes comprises DNA from the first partitioned subsample, the treated first subsample, the second partitioned subsample, and/or the treated second subsample.

12. The method of claim 11, wherein
(i) the cytosine modification is methylation, optionally wherein the cytosine modification is methylation at the 5 position of cytosine;
(ii) the first subsample is contacted with a methylation-sensitive endonuclease, for example wherein the methylation-sensitive endonuclease cleaves an unmethylated CpG sequence; and/or
(iii) the methylation-sensitive endonuclease is one or more of AatII, AccII, AciI, Aor13HI, Aor15HI, BspT104I, BssHII, BstUI, Cfr10I, ClaI, CpoI, Eco52I, HaeII, HapII, HhaI, Hin6I, HpaII, HpyCH4IV, MluI, NaeI, NotI, NruI, NsbI, PmaCI, Psp1406I, PvuI, SacII, SalI, SmaI, and SnaBI.

13. The method of any one of the preceding claims, wherein the conversion procedure comprises bisulfite conversion; protection of 5hmC; Tet-assisted bisulfite conversion; Tet-assisted conversion with a substituted borane reducing agent, optionally wherein the substituted borane reducing agent is 2-picoline borane, borane pyridine, tert-butylamine borane, or ammonia borane; protection of hmC followed by Tet-assisted conversion with a substituted borane reducing agent, optionally wherein the substituted borane reducing agent is 2-picoline borane, borane pyridine, tert-butylamine borane, or ammonia borane; protection of hmC followed by deamination of mC and/or C; chemical-assisted conversion with a substituted borane reducing agent, optionally wherein the substituted borane reducing agent is 2-picoline borane, borane pyridine, tert-butylamine borane, or ammonia borane; or enzymatic protection of modified cytosines followed by deamination of unprotected cytosines to uracil.

14. The method of claim 13, wherein the deamination of mC and/or C comprises treatment with an AID/APOBEC family DNA deaminase enzyme or wherein protection of hmC comprises glucosylation of hmC.

15. The method of any one of the preceding claims, wherein
(i) the oligonucleotide adapters comprise sequencing primer binding sites and the quality control nucleosides are located downstream of the sequencing primer binding sites;
(ii) the method further comprises amplifying the DNA using primers targeting the adapters, wherein the amplifying step is in between the conversion step (b) and the sequencing step (c); and/or
(iii) the results of the method are used as an input to generate a report, for example wherein (a) the report is in a paper or electronic format, (b) the detection of false positives and/or false negatives, or information derived therefrom, is displayed directly in the report, and/or (c) diagnostic information or therapeutic recommendations which are at least in part based on the method are included in the report.

## Patentansprüche

1. Qualitätskontrollverfahren zur Überwachung von falsch negativem und/oder falsch positivem Nachweis modifizierter Nukleoside in DNA in einer Probe, wobei das Verfahren Folgendes umfasst:
(a) Ligieren der DNA an Oligonukleotid-Adapter, wobei die Adapter Qualitätskontrollnukleoside umfassen, wobei die Adapter-Qualitätskontrollnukleoside ein erstes Qualitätskontrollnukleosid, das modifiziert ist, und ein zweites Qualitätskontrollnukleosid, das nicht modifiziert ist, umfassen, wobei die Qualitätskontrollnukleoside die gleiche Nukleosididentität und den gleichen oder einen unterschiedlichen Modifikationsstatus gegenüber in der DNA nachzuweisenden modifizierten Nukleosiden aufweisen und wobei der Modifikationsstatus der Qualitätskontrollnukleoside bekannt ist;
(b) die adaptierte DNA oder eine Teilprobe davon einem Umwandlungsvorgang unterzogen wird, durch den die Basenpaarungsspezifität der Qualitätskontrollnukleoside je nach dem Modifikationsstatus der Nukleoside geändert wird oder nicht geändert wird, wobei der Umwandlungsvorgang so ausgewählt wird, dass
(i) die Basenpaarungsspezifität von Adaptierte-DNA-Nukleosiden, die die gleiche Nukleosididentität und den gleichen Modifikationsstatus wie Qualitätskontrollnukleoside in den Adaptern aufweisen, geändert wird und die Basenpaarungsspezifität von Adaptierte-DNA-Nukleosiden, die die gleiche Nukleosididentität wie Qualitätskontrollnukleoside in den Adaptern, aber einen unterschiedlichen Modifikationsstatus aufweisen, nicht geändert wird; und/oder
(ii) die Basenpaarungsspezifität von Adaptierte-DNA-Nukleosiden, die die gleiche Nukleosididentität und den gleichen Modifikationsstatus wie Qualitätskontrollnukleoside in den Adaptern aufweisen, nicht geändert wird und die Basenpaarungsspezifität von Adaptierte-DNA-Nukleosiden, die die gleiche Nukleosididentität wie Qualitätskontrollnukleoside in den Adaptern, aber einen unterschiedlichen Modifikationsstatus aufweisen, geändert wird;
(c) Sequenzieren der adaptierten DNA nach Umwandlungsschritt (b);
(d) Verwenden der in Schritt (c) erhaltenen Sequenzdaten zur Bestimmung von Basenpaarungsspezifitätsumwandlung der Qualitätskontrollnukleoside in den Adaptern; und
(e) Verwenden der Basenpaarungsspezifitätsumwandlung der Qualitätskontrollnukleoside in den Adaptern als ein Qualitätskontrollmaß für Umwandlungsschritt (b), wobei eine suboptimale Umwandlung von Adapter-Qualitätskontrollnukleosiden im Anschluss an einen Umwandlungsvorgang unter Schritt (b)(i) und/oder eine fehlerhafte Umwandlung von Adapter-Qualitätskontrollnukleosiden im Anschluss an einen Umwandlungsvorgang unter Schritt (b)(ii) einen falsch negativen und/oder falsch positive Nachweis modifizierter Nukleoside in der DNA-Probe vorhersagt.

2. Verfahren nach Anspruch 1, wobei der Umwandlungsvorgang so ausgewählt wird, dass die Basenpaarungsspezifität von Qualitätskontrollnukleosiden in den Adaptern, aber nicht die Basenpaarungsspezifität von DNA-Probe-Nukleosiden, die die gleiche Nukleosididentität und einen unterschiedlichen Modifikationsstatus aufweisen, geändert wird; und wobei eine suboptimale Umwandlung der Qualitätskontrollnukleoside einen falsch positiven Nachweis von DNA-Probe-Nukleosiden mit dem unterschiedlichen Modifikationsstatus vorhersagt; beispielsweise wobei die Qualitätskontrollnukleoside in den Adaptern Cytosin umfassen und/oder wobei der Umwandlungsvorgang Hydrogensulfitumwandlung umfasst.

3. Verfahren nach Anspruch 1 oder Anspruch 2, wobei es sich bei dem ersten Qualitätskontrollnukleosid um ein modifiziertes Cytosin und bei dem zweiten Qualitätskontrollnukleosid um ein nicht modifiziertes Cytosin handelt, beispielsweise wobei es sich bei dem ersten Qualitätskontrollnukleosid um 5-Methylcytosin (5mC) oder 5-Hydroxymethylcytosin (5hmC) handelt.

4. Verfahren nach einem der vorhergehenden Ansprüche, wobei der Umwandlungsvorgang so ausgewählt wird, dass
(i) die Basenpaarungsspezifität des ersten Qualitätskontrollnukleosids, aber nicht die des zweiten Qualitätskontrollnukleosids geändert wird;
(ii) die Basenpaarungsspezifität des zweiten Qualitätskontrollnukleosids, aber nicht die des ersten Qualitätskontrollnukleosids geändert wird; oder
(iii) die Basenpaarungsspezifität von modifizierten Qualitätskontrollnukleosiden in den Adaptern, aber nicht die Basenpaarungsspezifität von DNA-Probe-Nukleosiden, die die gleiche Nukleosididentität, aber einen unterschiedlichen Modifikationsstatus und/oder keine Modifikation aufweisen, geändert wird; und wobei eine suboptimale Umwandlung der modifizierten Qualitätskontrollnukleoside einen falsch negativen Nachweis von DNA-Probe-Nukleosiden vorhersagt, die die gleiche Nukleosididentität und den gleichen Modifikationsstatus wie die Qualitätskontrollnukleoside oder einen unterschiedlichen Modifikationsstatus und die gleiche Änderung der Basenpaarungsspezifität nach durchlaufenem Umwandlungsvorgang aufweisen.

5. Verfahren nach einem der vorhergehenden Ansprüche, wobei das Verfahren ferner Verwenden der in Schritt (c) erhaltenen Sequenzdaten zum
(i) Identifizieren adaptierter DNA-Moleküle mit suboptimaler oder fehlerhafter Umwandlung von Qualitätskontrollnukleosiden in der Adaptersequenz umfasst; und
(ii) Ableiten suboptimaler oder fehlerhafter Umwandlung von Nukleosiden mit der gleichen Nukleosididentität und dem gleichen Modifikationsstatus in den in Schritt (i) Volllängenmolekülen umfasst.

6. Verfahren nach einem der vorhergehenden Ansprüche, wobei das Verfahren ferner Bestimmen der Umwandlungsrate für Qualitätskontrollnukleoside in der adaptierten DNA oder in einzelnen adaptierten DNA-Molekülen und
(i) Anwenden einer Wichtung auf die Analyse des Nachweises modifizierter Nukleoside in
(A) der DNA-Probe oder
(B) einzelnen adaptierten DNA-Molekülen umfasst, wobei die Wichtung von der Umwandlungsrate abhängt;
(ii) Ausschließen von DNA-Proben mit
(A) suboptimaler Umwandlung von Adapter-Qualitätskontrollnukleosiden oder einer Umwandlungsrate für Adapter-Qualitätskontrollnukleoside, die unter einem vorbestimmten Qualitätskontrollschwellenwert liegt, und/oder
(B) fehlerhafter Umwandlung von Adapter-Qualitätskontrollnukleosiden oder einer Umwandlungsrate für Adapter-Qualitätskontrollnukleoside, die über einem vorbestimmten Qualitätskontrollschwellenwert liegt,
von weiterer Analyse zum Nachweisen modifizierter Nukleoside umfasst; und/oder
(iii) Ausschließen adaptierter DNA-Moleküle mit
(A) suboptimaler Umwandlung von Adapter-Qualitätskontrollnukleosiden oder einer Umwandlungsrate für Adapter-Qualitätskontrollnukleoside, die unter einem vorbestimmten Qualitätskontrollschwellenwert liegt, und/oder
(B) fehlerhafter Umwandlung von Adapter-Qualitätskontrollnukleosiden oder einer Umwandlungsrate für Adapter-Qualitätskontrollnukleoside, die über einem vorbestimmten Qualitätskontrollschwellenwert liegt,
von weiterer Analyse zum Nachweisen modifizierter Nukleoside umfasst.

7. Verfahren nach einem der vorhergehenden Ansprüche, wobei das Verfahren ferner Anreichern der DNA durch Einfangen eines Zielregionsatzes aus der Probe umfasst, wobei der Einfangschritt vor, nach oder zwischen dem Ligationsschritt (a) und dem Umwandlungsschritt (b) liegt.

8. Verfahren nach einem der vorhergehenden Ansprüche ferner umfassend
(i) Vergleichen der in Schritt (c) erhaltenen Sequenzdaten mit
(A) einer vorbestimmten Referenzsequenz und/oder
(B) Sequenzdaten, die durch Sequenzieren einer Teilprobe der DNA, die nicht dem Umwandlungsvorgang unterzogen wurde, erhalten wurden; und
(ii) Identifizieren von Punktunterschieden zwischen den umgewandelten DNA-Sequenzen und der Referenzsequenz (A) oder Sequenzdaten nicht umgewandelter DNA (B) als Nukleoside mit einem Modifikationsstatus, der eine Änderung der Basenpaarungsspezifität bei Durchlaufen des Umwandlungsvorgangs gestattet.

9. Verfahren nach einem der vorhergehenden Ansprüche,
(i) wobei die DNA von einem Testindividuum erhaltene zellfreie DNA (cfDNA) umfasst, gegebenenfalls wobei es sich bei dem Testindividuum um einen Patienten handelt, der an Krebs erkrankt ist oder bei dem eine Krebserkrankung vermutet wird;
(ii) ferner umfassend Verwenden des Nachweises modifizierter Nukleoside in der DNA-Probe zur Bestimmung oder Vorhersage des Vorliegens von von einer Krebszelle oder einem Tumor produzierter DNA zur Bestimmung der Wahrscheinlichkeit, dass bei einem Testindividuum ein Tumor bzw. eine Krebserkrankung vorliegt, oder zur Charakterisierung einer Krebserkrankung bzw. eines Tumors des Testindividuums;
(iii) wobei eine Teilprobe der DNA vor dem Sequenzieren nicht dem Umwandlungsvorgang unterzogen wird, wobei die umgewandelte Teilprobe und die nicht umgewandelte Teilprobe unterschiedliche Adaptersequenzen aufweisen und wobei die umgewandelte Teilprobe und die nicht umgewandelte Teilprobe für Sequenzierschritt (c) rekombiniert werden;
(iv) ferner umfassend Analysieren der DNA zum Nachweisen von Kopienzahlvariation, Einzelnukleotidvarianten, Insertionen, Deletionen, Methylierung und/oder Fusionen;
(v) ferner umfassend Einfangen epigenetischer Zielregionen aus der Adapter-ligierten DNA und Amplifizieren und Sequenzieren der epigenetischen Zielregionen, wobei die eingefangenen epigenetischen Zielregionen einen Satz epigenetischer Zielregionen bilden; beispielsweise wobei der Satz epigenetischer Zielregionen mehrere typspezifische epigenetische Zielregionen umfasst und wobei es sich bei den typspezifischen epigenetischen Zielregionen um typspezifische unterschiedlich methylierte Regionen und/oder typspezifische Fragmente handelt, weiter gegebenenfalls wobei die mehreren typspezifischen epigenetischen Zielregionen typspezifische hypomethylierte Regionen oder typspezifische hypermethylierte Regionen umfassen; und/oder
(vi) wobei es sich bei der Probe um eine Blutprobe handelt.

10. Verfahren nach Anspruch 9, wobei (i) die typspezifischen epigenetischen Zielregionen zelltypspezifische, gewebetypspezifische und/oder krebstypspezifische epigenetische Zielregionen umfassen und/oder (ii) die Blutprobe vor dem Einfangen wenigstens eines Satzes epigenetischer Zielregionen von DNA fraktioniert wird.

11. Verfahren nach einem der vorhergehenden Ansprüche, ferner umfassend:
Unterteilen der Probe oder eines Aliquots davon in mehrere unterteilte Teilproben, einschließlich einer ersten unterteilten Teilprobe und einer zweiten unterteilten Teilprobe, wobei die erste Teilprobe einen größeren Anteil von DNA mit einer Cytosinmodifikation als die zweite Teilprobe umfasst;
Inkontaktbringen der zweiten unterteilten Teilprobe mit einer methylierungsabhängigen Nuklease, wodurch unspezifisch unterteilte DNA in der zweiten Teilprobe unter Erhalt einer behandelten zweiten Teilprobe abgebaut wird, und gegebenenfalls Inkontaktbringen der zweiten unterteilten Teilprobe mit einer methylierungssensitiven Endonuklease, wodurch unspezifisch unterteilte DNA in der ersten Teilprobe unter Erhalt einer behandelten ersten Teilprobe abgebaut wird;
wobei epigenetische Zielregionen aus wenigstens einem Teil der ersten Teilprobe oder der behandelten ersten Teilprobe eingefangen werden,
gegebenenfalls wobei die DNA von dem Individuum, das mit den eine oder mehreren Einfangsonden in Kontakt gebracht wird, DNA aus der ersten unterteilten Teilprobe, der behandelten ersten Teilprobe, der zweiten unterteilten Teilprobe und/oder der behandelten zweiten Teilprobe umfasst.

12. Verfahren nach Anspruch 11, wobei
(i) es sich bei der Cytosinmodifikation um Methylierung handelt, gegebenenfalls wobei es sich bei der Cytosinmodifikation um Methylierung an der 5-Position von Cytosin handelt;
(ii) die erste Teilprobe mit einer methylierungssensitiven Endonuklease in Kontakt gebracht wird, beispielsweise wobei die methylierungssensitive Endonuklease eine nicht methylierte CpG-Sequenz spaltet; und/oder
(iii) es sich bei der methylierungssensitiven Endonuklease um eine oder mehrere von AatII, AccII, AciI, Aor13HI, Aor15HI, BspT104I, BssHII, BstUI, Cfr10I, ClaI, CpoI, Eco52I, HaeII, HapII, HhaI, Hin6I, HpaII, HpyCH4IV, MluI, NaeI, NotI, NruI, NsbI, PmaCI, Psp1406I, PvuI, SacII, SalI, SmaI und SnaBI handelt.

13. Verfahren nach einem der vorhergehenden Ansprüche, wobei der Umwandlungsvorgang Hydrogensulfitumwandlung, Schutz von 5hmC, Tet-gestützte Hydrogensulfitumwandlung, Tet-gestützte Umwandlung mit einem substituierten Boran-Reduktionsmittel, gegebenenfalls wobei es sich bei dem substituierten Boran-Reduktionsmittel um 2-Picolinboran, Boranpyridin, tert-Butylaminboran oder Ammoniakboran handelt, Schutz von hmC gefolgt von Tet-gestützter Umwandlung mit einem substituierten Boran-Reduktionsmittel, gegebenenfalls wobei es sich bei dem substituierten Boran-Reduktionsmittel um 2-Picolinboran, Boranpyridin, tert-Butylaminboran oder Ammoniakboran handelt, Schutz von hmC gefolgt von Deaminierung von mC und/oder C, chemikaliengestützte Umwandlung mit einem substituierten Boran-Reduktionsmittel, gegebenenfalls wobei es sich bei dem substituierten Boran-Reduktionsmittel um 2-Picolinboran, Boranpyridin, *tert-*Butylaminboran oder Ammoniakboran handelt, oder enzymatischen Schutz modifizierter Cytosinen gefolgt von Deaminierung ungeschützter Cytosine zu Uracil umfasst.

14. Verfahren nach Anspruch 13, wobei die Deaminierung von mC und/oder C Behandlung mit einem DNA-Deaminase-Enzym der AID/APOBEC-Familie umfasst oder wobei Schutz von hmC Glucosylierung von hmC umfasst.

15. Verfahren nach einem der vorhergehenden Ansprüche, wobei
(i) die Oligonukleotid-Adapter Sequenzierprimer-Bindungsstellen umfassen und die Qualitätskontrollnukleoside stromabwärts von den Sequenzierprimer-Bindungsstellen liegen;
(ii) das Verfahren ferner Amplifizieren der DNA unter Verwendung von auf die Adapter zielenden Primern umfasst, wobei der Amplifizierschritt zwischen dem Umwandlungsschritt (b) und dem Sequenzierschritt (c) liegt; und/oder
(iii) die Ergebnisse des Verfahren als Eingabe zur Erstellung eines Berichts verwendet werden, beispielsweise wobei (a) der Bericht in einem Papier- oder elektronischen Format vorliegt, (b) der Nachweis falsch Positiver und/oder falsch Negativer oder daraus abgeleitete Information direkt im Bericht präsentiert wird und/oder (c) Angaben zur Diagnose oder Therapieempfehlungen, die wenigstens teilweise auf dem Verfahren beruhen, in den Bericht aufgenommen werden.

## Revendications

1. Procédé de contrôle de qualité pour le suivi de la détection de faux négatifs et/ou de faux positifs de nucléosides modifiés dans de l'ADN présent dans un échantillon, le procédé comprenant :
(a) la ligature de l'ADN à des adaptateurs oligonucléotidiques, les adaptateurs comprenant des nucléosides de contrôle de qualité, les nucléosides de contrôle de qualité des adaptateurs incluant un premier nucléoside de contrôle de qualité qui est modifié et un second nucléoside de contrôle de qualité qui est non modifié, les nucléosides de contrôle de qualité ayant la même identité de nucléoside et le même état de modification ou un état de modification différent par rapport à des nucléosides modifiés devant être détectés dans l'ADN et l'état de modification des nucléosides de contrôle de qualité étant connu ;
(b) le fait de soumettre l'ADN adapté, ou un sous-échantillon de celui-ci, à une procédure de conversion qui change la spécificité de l'appariement de bases des nucléosides de contrôle de qualité ou qui ne change pas la spécificité de l'appariement de bases des nucléosides de contrôle de qualité, en fonction de l'état de modification des nucléosides, la procédure de conversion étant choisie pour
(i) changer la spécificité de l'appariement de bases de nucléosides de l'ADN adapté ayant la même identité de nucléoside et le même état de modification que les nucléosides de contrôle de qualité présents dans les adaptateurs et ne pas changer la spécificité de l'appariement de bases de nucléosides de l'ADN adapté ayant la même identité de nucléoside que les nucléosides de contrôle de qualité présents dans les adaptateurs mais un état de modification différent ; et/ou
(ii) ne pas changer la spécificité de l'appariement de bases de nucléosides de l'ADN adapté ayant la même identité de nucléoside et le même état de modification que les nucléosides de contrôle de qualité présents dans les adaptateurs et changer la spécificité de l'appariement de bases de nucléosides de l'ADN adapté ayant la même identité d'appariement que les nucléosides de contrôle de qualité présents dans les adaptateurs mais un état de modification différent ;
(c) le séquençage de l'ADN adapté après l'étape de conversion (b) ;
(d) l'utilisation des données de séquence obtenues à l'étape (c) pour déterminer la conversion de la spécificité de l'appariement de bases des nucléosides de contrôle de qualité présents dans les adaptateurs ; et
(e) l'utilisation de la conversion de la spécificité de l'appariement de bases des nucléosides de contrôle de qualité présents dans les adaptateurs en tant que mesure de contrôle de qualité pour l'étape de conversion (b), une conversion sous-optimale de nucléosides de contrôle de qualité des adaptateurs suite à une procédure de conversion de l'étape (b) (i) et/ou une conversion erronée de nucléosides de contrôle de qualité des adaptateurs suite à une procédure de conversion de l'étape (b) (ii) prédisant la détection de faux négatifs et/ou de faux positifs de nucléosides modifiés dans l'échantillon d'ADN.

2. Procédé selon la revendication 1, dans lequel la procédure de conversion est choisie pour changer la spécificité de l'appariement de bases de nucléosides de contrôle de qualité présents dans les adaptateurs, mais pas la spécificité de l'appariement de bases de nucléosides de l'échantillon d'ADN ayant la même identité de nucléoside et un état de modification différent ; et dans lequel une conversion sous-optimale des nucléosides de contrôle de qualité prédit la détection de faux positifs de nucléosides de l'échantillon d'ADN ayant l'état de modification différent ; par exemple dans lequel les nucléosides de contrôle de qualité présents dans les adaptateurs incluent une cytosine et/ou dans lequel la procédure de conversion comprend une conversion au bisulfite.

3. Procédé selon la revendication 1 ou la revendication 2, dans lequel le premier nucléoside de contrôle de qualité est une cytosines modifiée et le second nucléoside de contrôle de qualité est une cytosine non modifiée, par exemple dans lequel le premier nucléoside de contrôle de qualité est la 5-méthylcytosine (5mC) ou la 5-hydroxyméthylcytosine (5hmC).

4. Procédé selon l'une quelconque des revendications précédentes, dans lequel la procédure de conversion est choisie pour
(i) changer la spécificité de l'appariement de bases du premier nucléoside de contrôle de qualité mais pas du second nucléoside de contrôle de qualité ;
(ii) changer la spécificité de l'appariement de bases du second nucléoside de contrôle de qualité mais pas du premier nucléoside de contrôle de qualité ; ou
(iii) changer la spécificité de l'appariement de bases de nucléosides de contrôle de qualité modifiés présents dans les adaptateurs, mais pas la spécificité de l'appariement de bases de nucléosides de l'échantillon d'ADN ayant la même identité de nucléoside mais un état de modification différent et/ou pas de modification ; et dans lequel une conversion sous-optimale des nucléosides de contrôle de qualité modifiés prédit la détection de faux négatifs de nucléosides de l'échantillon d'ADN ayant la même identité de nucléoside et le même état de modification que les nucléosides de contrôle de qualité ou un état de modification différent et le même changement de spécificité de l'appariement de bases lors de l'exposition à la procédure de conversion.

5. Procédé selon l'une quelconque des revendications précédentes, le procédé comprenant en outre l'utilisation des données de séquence obtenues à l'étape (c) pour
(i) identifier des molécules d'ADN adapté présentant une conversion sous-optimale ou erronée de nucléosides de contrôle de qualité présents dans la séquence des adaptateurs ; et
(ii) déduire une conversion sous-optimale ou erronée de nucléosides ayant la même identité de nucléoside et le même état de modification dans les molécules de pleine longueur identifiées à l'étape (i).

6. Procédé selon l'une quelconque des revendications précédentes, le procédé comprenant en outre la détermination du taux de conversion pour les nucléosides de contrôle de qualité présents dans l'ADN adapté ou dans des molécules d'ADN adapté individuelles et
(i) l'application d'une pondération à l'analyse de la détection de nucléosides modifiés présents dans
(A) l'échantillon d'ADN ; ou
(B) des molécules d'ADN adapté individuelles,
la pondération étant fonction du taux de conversion ;
(ii) l'exclusion d'échantillons d'ADN ayant
(A) une conversion sous-optimale de nucléosides de contrôle de qualité des adaptateurs ou un taux de conversion pour les nucléosides de contrôle de qualité des adaptateurs qui est au-dessous d'un seuil de contrôle de qualité prédéfini ; et/ou
(B) une conversion erronée de nucléosides de contrôle de qualité des adaptateurs ou un taux de conversion pour les nucléosides de contrôle de qualité des adaptateurs qui est au-dessus d'un seuil de contrôle de qualité prédéfini,
de l'analyse ultérieure pour la détection de nucléosides modifiés ; et/ou
(iii) l'exclusion de molécules d'ADN adapté ayant
(A) une conversion sous-optimale de nucléosides de contrôle de qualité des adaptateurs ou un taux de conversion pour les nucléosides de contrôle de qualité des adaptateurs qui est au-dessous d'un seuil de contrôle de qualité prédéfini ; et/ou
(B) une conversion erronée de nucléosides de contrôle de qualité des adaptateurs ou un taux de conversion pour les nucléosides de contrôle de qualité des adaptateurs qui est au-dessus d'un seuil de contrôle de qualité prédéfini,
de l'analyse ultérieure pour la détection de nucléosides modifiés.

7. Procédé selon l'une quelconque des revendications précédentes, le procédé comprenant en outre l'enrichissement de l'ADN par la capture d'un ensemble de régions cibles à partir de l'échantillon, dans lequel l'étape de capture se trouve avant, après ou entre l'étape de ligature (a) et l'étape de conversion (b).

8. Procédé selon l'une quelconque des revendications précédentes, comprenant en outre
(i) la comparaison des données de séquence obtenues à l'étape (c) avec
(A) une séquence de références prédéfinie ; et/ou
(B) des données de séquence obtenues par séquençage d'un sous-échantillon de l'ADN qui n'a pas été soumis à la procédure de conversion ; et
(ii) l'identification de différences ponctuelles entre les séquences d'ADN converti et la séquence de référence (A) ou les données de séquence d'ADN non converti (B) comme étant des nucléosides ayant un état de modification qui permet un changement de la spécificité de l'appariement de bases lors de l'exposition à la procédure de conversion.

9. Procédé selon l'une quelconque des revendications précédentes,
(i) dans lequel l'ADN comprend de l'ADN acellulaire (ADNac) obtenu à partir d'un sujet testé, éventuellement le sujet testé étant un patient ayant un cancer ou pour lequel un cancer est suspecté ;
(ii) comprenant en outre l'utilisation de la détection de nucléosides modifiés dans l'échantillon d'ADN pour déterminer ou prédire la présence d'ADN produit par une cellule cancéreuse ou une tumeur, pour déterminer la probabilité qu'un sujet testé ait une tumeur ou un cancer ou pour caractériser un cancer ou une tumeur du sujet ;
(iii) dans lequel un sous-échantillon de l'ADN n'est pas soumis à la procédure de conversion avant séquençage, le sous-échantillon converti et le sous-échantillon non converti ayant des séquences d'adaptateurs différentes et le sous-échantillon converti et le sous-échantillon non converti étant recombinés pour l'étape de séquençage (c) ;
(iv) comprenant en outre l'analyse de l'ADN pour détecter la variation du nombre de copies, des variants mononucléotidiques, des insertions, des délétions, la méthylation et/ou des fusions ;
(v) comprenant en outre la capture de régions cibles épigénétiques à partir de l'ADN ligaturé aux adaptateurs et l'amplification et le séquençage des régions cibles épigénétiques, les régions cibles épigénétiques capturées formant un ensemble de régions cibles épigénétiques ; par exemple l'ensemble de régions cibles épigénétiques comprenant une pluralité de régions cibles épigénétiques spécifiques d'un type et les régions cibles épigénétiques spécifiques d'un type étant des régions méthylées de façon différente spécifiques d'un type et/ou des fragments spécifiques d'un type, éventuellement en outre la pluralité de régions cibles épigénétiques spécifiques d'un type comprenant des régions hypométhylées spécifiques d'un type ou des régions hyperméthylées spécifiques d'un type ; et/ou
(vi) dans lequel l'échantillon est un échantillon de sang.

10. Procédé selon la revendication 9, dans lequel (i) les régions cibles épigénétiques spécifiques d'un type comprennent des régions cibles épigénétiques spécifiques d'un type de cellule, spécifiques d'un type de tissu et/ou spécifiques d'un type de cancer ; et/ou (ii) l'échantillon de sang est fractionné avant la capture d'au moins un ensemble de régions cibles épigénétiques de l'ADN.

11. Procédé selon l'une quelconque des revendications précédentes, comprenant en outre :
la séparation de l'échantillon ou d'une aliquote de celui-ci en une pluralité de sous-échantillons séparés, incluant un premier sous-échantillon séparé et un second sous-échantillon séparé, le premier sous-échantillon séparé comprenant de l'ADN présentant une modification de cytosines en une plus grande proportion que le second sous-échantillon ;
la mise en contact du second sous-échantillon séparé avec une nucléase dépendante de la méthylation, ce qui dégrade ainsi de façon non spécifique de l'ADN séparé présent dans le second sous-échantillon pour produire un second sous-échantillon traité, et éventuellement la mise en contact du premier sous-échantillon séparé avec une endonucléase sensible à la méthylation, ce qui dégrade ainsi de façon non spécifique de l'ADN séparé présent dans le premier sous-échantillon séparé pour produire un premier sous-échantillon traité ;
dans lequel des régions cibles épigénétiques sont capturées à partir d'au moins une partie du premier sous-échantillon ou du premier sous-échantillon traité ;
éventuellement l'ADN provenant du sujet qui est mis en contact avec la ou les sondes de capture comprenant de l'ADN provenant du premier sous-échantillon séparé, du premier sous-échantillon traité, du second sous-échantillon séparé et/ou du second sous-échantillon traité.

12. Procédé selon revendication 11, dans lequel
(i) la modification de cytosines est une méthylation, éventuellement la modification de cytosines étant une méthylation au niveau de la position 5 de la cytosine ;
(ii) le premier sous-échantillon est mis en contact avec une endonucléase sensible à la méthylation, par exemple l'endonucléase sensible à la méthylation clivant une séquence CpG non méthylée ; et/ou
(iii) l'endonucléase sensible à la méthylation est une ou plusieurs de AatII, AccII, AciI, Aor13HI, Aor15HI, BspT104I, BssHII, BssUI, Cfr10I, ClaI, CpoI, Eco52I, HaeII, HapII, HhaI, Hin6I, HpaII, HpyCH4IV, MluI, NaeI, NotI, NruI, NsbI, PmaCI, Psp1406I, PvuI, SacII, SaII, SmaI et SnaBI.

13. Procédé selon l'une quelconque des revendications précédentes, dans lequel la procédure de conversion comprend une conversion au bisulfite ; la protection de 5hmC ; une conversion au bisulfite assistée par Tet ; une conversion au bisulfite assistée par Tet avec un agent réducteur borane substitué, éventuellement l'agent réducteur borane substitué étant le complexe de 2-picoline et de borane, le complexe de borane et de pyridine, le complexe de tert-butylamine et de borane ou le complexe d'ammoniac et de borane ; la protection de hmC suivie d'une conversion au bisulfite assistée par Tet avec un agent réducteur borane substitué, éventuellement l'agent réducteur borane substitué étant le complexe de 2-picoline et de borane, le complexe de borane et de pyridine, le complexe de tert-butylamine et de borane ou le complexe d'ammoniac et de borane ; la protection de hmC suivie d'une désamination de mC et/ou de C ; une conversion assistée par un agent chimique avec un agent réducteur borane substitué, éventuellement l'agent réducteur borane substitué étant le complexe de 2-picoline et de borane, le complexe de borane et de pyridine, le complexe de tert-butylamine et de borane ou le complexe d'ammoniac et de borane ; ou la protection enzymatique de cytosines modifiées suivie de la désamination de cytosines non protégées en uracile.

14. Procédé selon la revendication 13, dans lequel la désamination de mC et/ou de C comprend le traitement avec une enzyme ADN désaminase de la famille de AID/APOBEC ou dans lequel la protection de hmC comprend la glucosylation de hmC.

15. Procédé selon l'une quelconque des revendications précédentes, dans lequel
(i) les adaptateurs oligonucléotidiques comprennent des sites de liaison à des amorces de séquençage et les nucléosides de contrôle de qualité sont situés en aval des sites de liaison aux amorces de séquençage ;
(ii) le procédé comprend en outre l'amplification de l'ADN à l'aide d'amorces ciblant les adaptateurs, l'étape d'amplification se trouvant entre l'étape de conversion (b) et l'étape de séquençage (c) ; et/ou
(iii) les résultats du procédé sont utilisés en tant qu'entrée pour générer un rapport, par exemple (a) le rapport étant au format papier ou électronique, (b) la détection de faux positifs et/ou de faux négatifs, ou des informations dérivées de celle-ci, étant affichées directement dans le rapport et/ou (c) des informations de diagnostic ou des recommandations thérapeutiques qui sont au moins en partie basées sur le procédé étant incluses dans le rapport.
